# EUROPEAN PATENT APPLICATION

(11) **EP 4 485 463 A1**
(43) Date of publication of application: **01.01.2025**
(21) Application number: 24185838.0
(22) Date of filing: 01.07.2024
(51) Int. Cl.: G16B 20/30, G16B 40/20

(54) **MODELING ENHANCERS USING MACHINE-LEARNING**

(30) Priority: 29.06.2023 US 202363511138 P; 25.09.2023 US 202363585119 P; 03.10.2023 US 202363587665 P
(71) Applicant: Shape Therapeutics Inc., Seattle, WA 98109 (US)
(72) Inventor: JIANG, Yue, Los Angeles, CA (US); HAUSE Jr, Ronald James, Seattle, WA (US); JOHNSTONE, Timothy George, Seattle, WA (US); TOME, Jacob Michael, Seattle, WA (US)
(74) Representative: Korenberg, Alexander Tal

(57) **Abstract**

A method of modeling enhancer activity obtains a training dataset in electronic form. The dataset comprises a plurality of training enhancers and, for each such enhancer, a corresponding measured amount of activity of the enhancer in each of one or more states. Each enhancer is a tandem repeat. A model comprising a plurality of parameters is trained by inputting each training enhancer into the model. Upon input, the model applies the parameters to the training enhancer to generate, as model output, a corresponding predicted activity of the training enhancer for a first state of the one or more states. The plurality of parameters is refined based differentials between corresponding measured and predicted amounts of activity in the first state for each of the training enhancer. A plurality of test enhancers is generated using the trained model and their activity tested thereby modeling enhancer activity.

## Description

### CROSS REFERENCE TO RELATED APPLICATIONS

This application claims priority to United States Provisional Patent Application No. 63/511,138 entitled "GENERATIVE SEQUENCE SCREENING WITH CONDITIONAL GANS, DIFFUSION MODELS, AND DENOISING DIFFUSION CONDITIONAL GANS," filed June 29, 2023, which is hereby incorporated by reference.

This application also claims priority to United States Provisional Patent Application No. 63/585,119 entitled "GENERATIVE SEQUENCE SCREENING WITH CONDITIONAL GANS, DIFFUSION MODELS, AND DENOISING DIFFUSION CONDITIONAL GANS," filed September 25, 2023, which is hereby incorporated by reference.

This application also claims priority to United States Provisional Patent Application No. 63/587,665 entitled "REGULATING ENHANCER ACTIVITY USING MACHINE-LEARNING," filed October 3, 2023, which is hereby incorporated by reference.

### TECHNICAL FIELD

This specification describes technologies generally relating to modeling enhancers, and in particular enhancer selectivity, using machine learning.

### SEQUENCE LISTING

The instant application contains a Sequence Listing that has been submitted electronically in XML format and is hereby incorporated by reference in its entirety.

### BACKGROUND

Enhancers are DNA regulatory elements that stimulate transcription, somewhat independent of their position and orientation with respect to the transcriptional initiation site. *See,* Banerji et al., "Expression of a beta-globin gene is enhanced by remove SV40 DNA sequences, " Cell 2, pp. 299-308. Typical enhancers span 200-1,000 base pairs and bind to dozens of sequence-specific proteins including transcription factors. *See,* Carroll et al., 2001, "From DNA to diversity: Molecular genetics and the evolution of animal design," Malden: Blackwell Science, 192 pages; and Davidson, 2001, Genomic regulatory systems: Development and evolution, San Diego; Academic Press, 261 pages.

To measure enhancer activity, assays have been developed to study the activity of yeast, *Drosophila* and human gene regulatory elements on a genome-wide scale. However, discovery of sequence determinants of enhancer mediated gene expression, including tissue specific enhancer mediated gene expression, using only genomic sequences is made difficult by the fact that the genome is repetitive and has evolved to perform multiple functions. Furthermore, the human genome is too short to even encode all combinations, orientations and spacings of approximately 1,639 human transcription factors in multiple independent sequence contexts. Thus, despite the information generated by genome-scale experiments, most sequence determinants that drive the activity of enhancers, including tissue specific activity, remain unknown. Thus, learning the rules by which enhancers help to determine where and when genes are expressed has proven difficult, despite the availability of full genome sequences of several mammals, extensive maps of genomic features and genome-scale data about transcription factor protein expression levels and transcription factor DNA binding *in vitro. See* Sahu et al., 2022, "Sequence determinants of human gene regulatory elements," Nature Genetics 54, pp. 283-294.

Discovery of sequence determinants of enhancer mediated gene expression, including tissue specific enhancer mediated gene expression, is further complicated by the intricacy of binding site (*e.g*., transcription factor binding sites) grammar of individual enhancers. Enhancers typically have clusters of such binding sites, the presence and arrangement of which is defined by a grammar that affects the overall ability of a given enhancer to promote gene expression and, in some instances, the tissue specificity of such gene expression. Models for this grammar range from a simple linear "billboard" model in which each binding site within an enhancer is flexibly disposed, because the proteins bound to the enhancer do not operate as a single unit, with a strictly defined overall architecture, but rather as an ensemble of separately acting factors or small groups of factors that independently interact with their targets, to an "enhanceosome model" in which there is a high degree of cooperativity between enhancer-bound proteins, such that alterations in individual binding sites can have drastic effects on enhancer output. The enhanceosome and billboard models represent two extremes of a continuum that probably describes most cellular enhancers. Sub-elements of a particular regulatory region might exhibit critical cooperative interactions between some of the factors, while other portions of the regulatory region are loosely structured. *See,* Arnosti and Kulkarni, 2005, "Transcriptional Enhancers: Intelligent Enhanceosomes or Flexible Billboards?," Journal of Cellular Biochemistry 94, p. 890-898, which is hereby incorporated by reference. Thus, to improve tissue specific gene expression, it is necessary to learn the grammar of enhancers, including the complex grammar that arises when such enhancers include or rely on binding components that are best described by the enhanceosome model.

Given the above background, systems and methods for modeling enhancer selectivity are needed in the art.

### SUMMARY

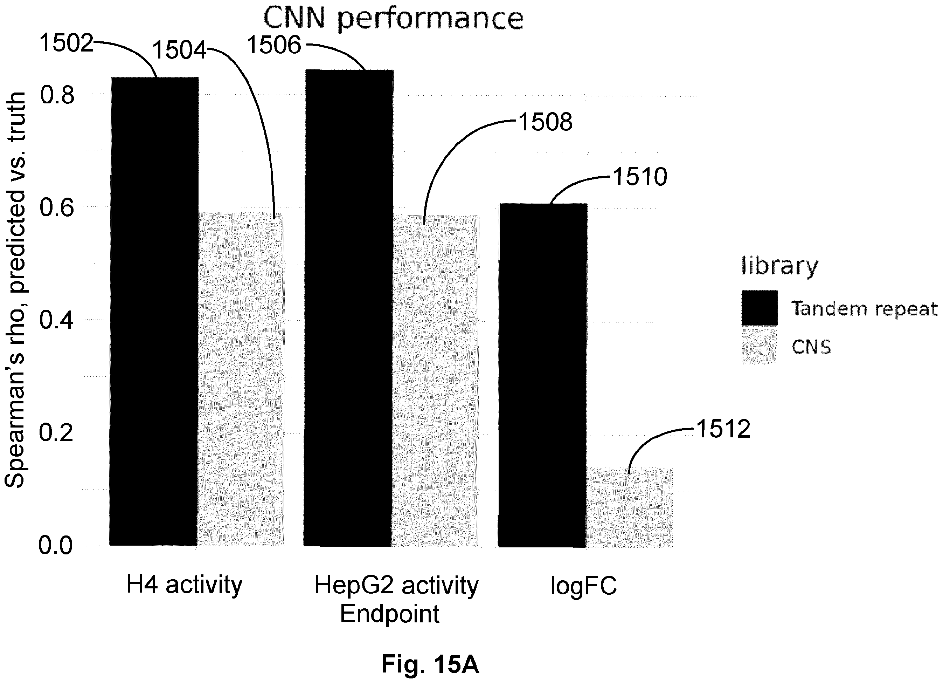

The present disclosure addresses the above-identified shortcomings by providing systems and methods for modeling enhancer activity using a training dataset comprising training enhancer sequences. For each respective training enhancer sequence, a corresponding measured activity of the respective sequence in each of one or more *in vivo* states is obtained. In instances where activity in more than one *in vivo* state is acquired, measured selectivity can be determined between any combination of measured states. For instance, if activity is measured for two *in vivo* states, selectivity between the two *in vivo* states can be determined by comparing the measured activity for the two *in vivo* states. In some embodiments, the activity for a sequence is a log of RNA counts to DNA counts for the given sequence. Advantageously, each training enhancer sequence is a tandem n-mer repeat. By using tandem n-mer repeats, a very large library of training enhancer sequences can be studied in order to identify the binding components within enhancers and their grammar. This library is studied in the present disclosure using models that comprise a plurality of parameters. Such a model is trained by inputting each training enhancer sequence into the model thereby obtaining as output from the model, for each sequence, a predicted amount of activity (*e.g.*, transcriptional activity) of the respective sequence in each of the one or more *in vivo* states. The parameters of the model are trained (refined) based on differentials between the corresponding measured amounts of activity and the corresponding predicted amounts of activity (*e.g.*, in one of the one or more *in vivo* states) for the training enhancer sequences. As discussed below in conjunction with Figure 15A, training a model on tandem n-mer repeat sequences rather than naturally occurring enhancers allows the trained model to produce predictions across the training set that are in better agreement with the training set than a model trained on naturally occurring enhancer sequences.

In accordance with the present disclosure, test enhancer sequences are generated using the trained model and their activity is assayed in an *in vivo* assay thereby screening for improved enhancer activity. By optimizing the model for activity in a particular *in vivo* state, such as in a particular tissue, it is possible to produce enhancers that are specific to particular *in vivo* states such that they exhibit high transcriptional activity in one *in vivo* state (*e.g.*, a first cell line, a first tissue) and low transcriptional activity in another *in vivo* state (*e.g.*, a second cell line, a second tissue).

In further detail, an aspect of the present disclosure provides a method of screening for improved enhancer activity. A first training dataset is obtained in electronic form. The first training dataset comprises a first plurality of training enhancer sequences and, for each respective training enhancer sequence in the first plurality of training enhancer sequences, a corresponding measured amount of activity of the respective training enhancer sequence in each of one or more *in vivo* states (*e.g*., 1, 2, 3, 4, 5, 6, 7, 8 or more *in vivo* states). Each training enhancer sequence in the first plurality of training enhancer sequences is a tandem n-mer repeat sequence.

In some embodiments, the first plurality of training enhancer sequences comprises 10,000 or more training enhancer sequences, 20,000 or more training enhancer sequences, 30,000 or more training enhancer sequences, 40,000 or more training enhancer sequences, 50,000 or more training enhancer sequences, 60,000 or more training enhancer sequences, 70,000 or more training enhancer sequences, 80,000 or more training enhancer sequences, 90,000 or more training enhancer sequences, or 100,000 or more training enhancer sequences.

In some embodiments, each training enhancer sequence in the first plurality of training enhancer sequences comprises 5 to 20 contiguous repeats of a different n-mer, where n is a positive integer between 4 and 20.

In some embodiments, each training enhancer sequence in the first plurality of training enhancer sequences comprises 5 to 20 contiguous repeats of a different 10-mer.

In some embodiments, each training enhancer sequence in the first plurality of training enhancer sequences consists of between 15 residues and 200 residues.

In some embodiments, each respective training enhancer sequence in the first plurality of training enhancer sequences is operably connected to a common promoter and a common payload. Each respective *in vivo* state in the plurality of *in vivo* states is an amount of abundance of the common payload (*e.g*., transcriptional expression, translation abundance, *etc.*) in a corresponding tissue. In some such embodiments, the tissue is brain tissue. In some embodiments the tissue is liver tissue. In some such embodiments, the tissue is blood, brain, colon, diaphragm, gastrocnemius muscle, heart, inguinal adipose, kidney, lung, lymph node, skin, small intestine, spinal cord, spleen, trachea, liver, stomach, large intestine, pancreas, gallbladder, bladder, eyes, thymus, or adrenal gland.

In some embodiments, each respective training enhancer sequence in the first plurality of training enhancer sequences is operably connected to a common promoter and a common payload under a particular transcription factor. Each respective *in vivo* state in the one or more *in vivo* states is an amount of expression of the common payload in a corresponding cell line originating from a particular tissue in conjunction with the particular transcription factors. In some embodiments the cell line is mouse primary neurons, HepG2, H4, or K-562. In some embodiments the particular transcription factor is FOS, FOSB::JUNB, FOSL2::JUNB, FOSL1::JUNB, FOS::JUN; FOSL1::JUN; BATF3, BNC2; ZBTB12; HSF4; HSF1; HSF2; REL; RELA; TEAD3; TEAD1; TP53; TP63; TP73; SMAD5; SMAD2; ZNF416; GRHL2; TFCP2; MLX; ARNT::HIF1A; or HIF1A.

In some embodiments, the one or more *in vivo* states consists of a first *in vivo* state and a second *in vivo* state, and the corresponding measured amount of abundance of the respective training enhancer sequence in each of the one or more *in vivo* states comprises a log fold change in (i) an abundance of the respective training enhancer sequence measured in the first *in vivo* state and (ii) an abundance of the respective training enhancer sequence measured in the second *in vivo* state.

In some embodiments, the one or more *in vivo* states consists of a first *in vivo* state and a second *in vivo* state, and the corresponding measured amount of abundance of the respective training enhancer sequence in each of the one or more *in vivo* states comprises a binary indication of measured activity (e.g., transcriptional activity) between the first *in vivo* state and the second *in vivo* state. Thus, if more activity is observed in the first *in vivo* state the binary indication has a first value (*e.g*., "1") and if more activity is observed in the second *in vivo* state, the binary indication has a second value (*e.g*., "0")*.*

In some embodiments, the corresponding measured amount of abundance of the respective training enhancer sequence in each of the one or more *in vivo* states comprises a corresponding measured abundance of the respective training enhancer sequence in each of the one or more *in vivo* states.

In some embodiments, the corresponding measured abundance of the respective training enhancer sequence in an *in vivo* state in the one or more *in vivo* states is a corresponding averaged or summed count of a number of barcoded instances of the respective training enhancer sequence found in a corresponding plurality of sequence reads of RNA harvested from each replica in a plurality of replicas of a plurality of cells, representative of the *in vivo* state exposed to the respective training enhancer sequence. In some such embodiments, the corresponding averaged or summed count is log normalized by an amount of the respective training enhancer sequence exposed to each replica of the plurality of cells.

In some embodiments, each respective training enhancer sequence in the plurality of respective training enhancer sequences is uniquely assigned a corresponding plurality of barcodes comprising 5 or more, 10 or more, 20 or more, 50 or more, 75 or more, or 100 or more barcodes. Each replica of the plurality of cells representative of the *in vivo* state exposed to the respective training enhancer sequence is, for each respective barcode uniquely assigned to the respective training enhancer sequence, one or more copies of the respective training enhancer sequence tagged with the respective barcode. In some such embodiments, each barcode in the corresponding plurality of barcodes encodes a unique predetermined value selected from the set {1, ..., 1024}, {1, ..., 4096}, {1, ..., 16384}, {1, ..., 65536}, {1, ..., 262144}, {1, ..., 1048576}, {1, ..., 4194304}, {1, ..., 16777216}, {1, ...,67108864}, or {1, ..., 1 × 10¹²}.

In some embodiments, the corresponding plurality of sequence reads of RNA harvested from each replica in the plurality of replicas of a plurality of cells comprises at least 1000, at least 2000, at least 3000, at least 4000, at least 5000, at least 6000, at least 7000, at least 8000, at least 9000, at least 10,000, at least 50,000, at least 100,000, at least 500,000, at least 1 million, at least 2 million, at least 3 million, at least 4 million, at least 5 million, at least 6 million, at least 7 million, at least 8 million, at least 9 million, or more sequence reads.

In some embodiments, the corresponding plurality of replicas of the plurality of cells comprises 2, 3, 4, 5, 6, 7, 8, 9, or 10 replicas.

In some embodiments, the one or more *in vivo* states consists of a first *in vivo* state and a second *in vivo* state, and the corresponding measured amount of abundance of the respective training enhancer sequence in each of the one or more *in vivo* states comprises a scalar indication of measured activity (*e.g*., transcriptional activity) between the first *in vivo* state and the second *in vivo* state. Thus, if 2.5 times more activity is observed in the first *in vivo* state than in the second *in vivo* state, the scalar indication has a value of 2.5.

A model comprising a plurality of parameters is trained by a procedure comprising inputting a sequence of each respective training enhancer sequence in the first plurality of training enhancer sequences into the model. The model applies the plurality of parameters to the sequence of each respective training enhancer sequence to generate as output from the model, for each respective training enhancer sequence in the first plurality of training enhancer sequences, a corresponding predicted amount of activity of the respective training enhancer sequence in at least a first *in vivo* state in the one or more *in vivo states.* The procedure further refines the plurality of parameters based on a differential between the corresponding measured amount of abundance and the corresponding predicted amount of abundance (in at least the first *in vivo* state) for each respective training enhancer sequence in the first plurality of training enhancer sequences.

In some such embodiments, the model is a support vector machine. In some such embodiments, the support vector machine comprises a gapped k-mer kernel.

In some such embodiments, the model is a convolutional neural network.

In some embodiments, the plurality of parameters comprises at least 100 parameters, 1000 parameters, at least 5000 parameters, at least 10,000 parameters, at least 100,000 parameters, at least 250,000 parameters, at least 500,000 parameters, or at least 1,000,000 parameters.

In some embodiments, the method further comprises obtaining a second training dataset, in electronic form. The second training dataset comprises a second plurality of training enhancer sequences. For each respective training enhancer sequence in the second plurality of training enhancer sequences, a corresponding measured amount of activity (*e.g*., transcriptional activity, translation activity, *etc.*) of the respective training enhancer sequence in each of the one or more *in vivo* states. Each training enhancer sequence in the second plurality of training enhancer sequences is an endogenous enhancer (*e.g*., has at least 60 percent, at least 70 percent, at least 80 percent, at least 90 percent, or at least 99 percent sequence identity to the sequence of a naturally occurring enhancer). The procedure further comprises, after the inputting and refining described above, inputting a sequence of each respective training enhancer sequence in the second plurality of training enhancer sequences into the model thereby obtaining as output from the model, for each respective training enhancer sequence in the second plurality of training enhancer sequences, a corresponding predicted amount of abundance of the respective training enhancer in each of the one or more *in vivo* states. The plurality of parameters is then refined based on a differential between the corresponding measured amount of abundance and the corresponding predicted amount of abundance for each respective training enhancer sequence in the second plurality of training enhancer sequences (in at least one of the one or more *in vivo* states).

In some embodiments, the second plurality of training enhancer sequences comprises 10,000 or more training enhancer sequences, 20,000 or more training enhancer sequences, 30,000 or more training enhancer sequences, 40,000 or more training enhancer sequences, 50,000 or more training enhancer sequences, 60,000 or more training enhancer sequences, 70,000 or more training enhancer sequences, 80,000 or more training enhancer sequences, 90,000 or more training enhancer sequences, or 100,000 or more training enhancer sequences.

A plurality of test enhancer sequences is generated using the trained model. In some such embodiments, the plurality of test enhancer sequences comprises 10 or more, 100 or more, 1000 or more, or 10,000 or more test enhancer sequences.

In some such embodiments, a feature attribution method is applied to a test nucleic acid sequence (e.g., an endogenous enhancer) using the trained model to obtain a predictive weight matrix that provides a predictive contribution of every residue position in the test nucleic acid sequence to the plurality of *in vivo* states. In such embodiments, the generating the plurality of test enhancer sequences is in accordance with at least a portion of the predictive weight matrix. In some such embodiments, the model is a convolutional neural network and the feature attribution method is an integrated gradients feature attribution method.

In some embodiments, a test enhancer sequence in the plurality of test enhancer sequences comprises a plurality of residue positions. Each respective residue position in the plurality of residue positions is populated with one of four naturally occurring nucleotides based on a corresponding probability for the respective residue position given by the predictive weight matrix.

In some embodiments, the trained model is used to generate the plurality of test enhancer sequences using an input optimization algorithm.

In some embodiments, the trained model is used to generate the plurality of test enhancer sequences using a bit diffusion algorithm.

An activity (e.g., transcriptional activity, translation activity, *etc.)* of each respective test enhancer sequence in the plurality of test enhancer sequences in an *in vivo* assay is determined thereby screening for improved enhancer activity.

In some such embodiments, the determining the activity of each respective test enhancer sequence in the plurality of test enhancer sequences in an *in vivo* assay comprises exposing the respective test enhancer sequence operably connected to the common promoter and the common payload to each *in vivo* state in the one or more *in vivo* states and measuring an abundance of the common payload in each respective *in vivo* state in the one or more *in vivo* states.

Another aspect of the present disclosure provides a system comprising a processor and a memory storing instructions, which when executed by the processor, cause the processor to perform steps comprising any of the methods described in the present disclosure.

Still another aspect of the present disclosure provides a non-transitory computer-readable medium storing computer code comprising instructions, when executed by one or more processors, causing the processors to perform any of the methods described in the present disclosure.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "figure" and "FIG." herein), of which:

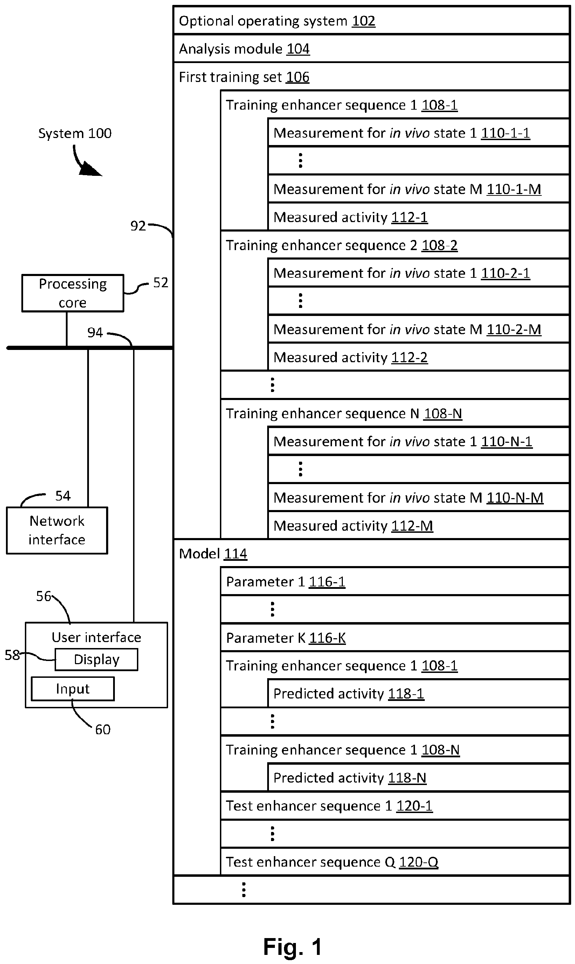

FIG. 1 illustrates an exemplary system for screening for improved enhancer activity, in accordance with some embodiments of the present disclosure.

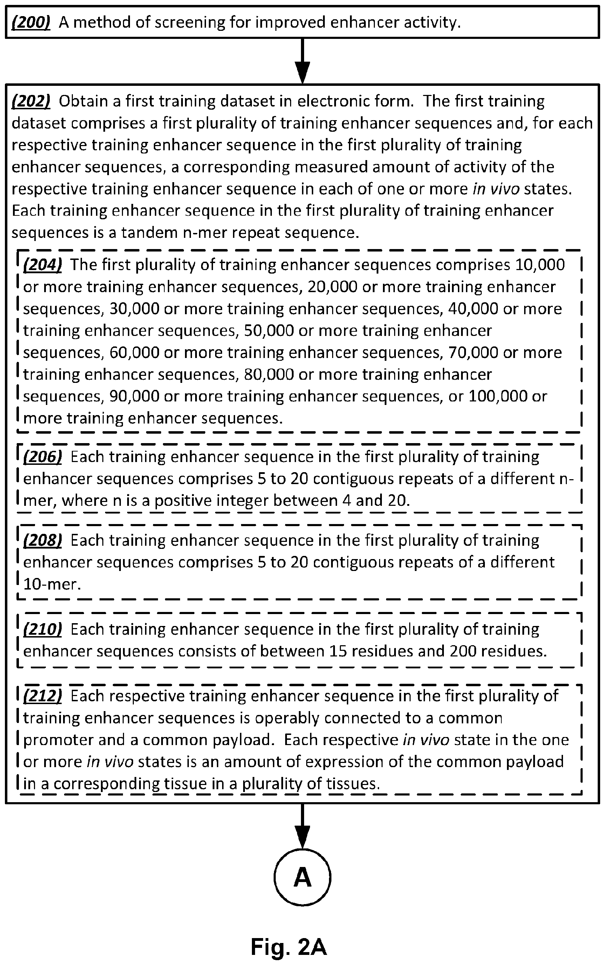

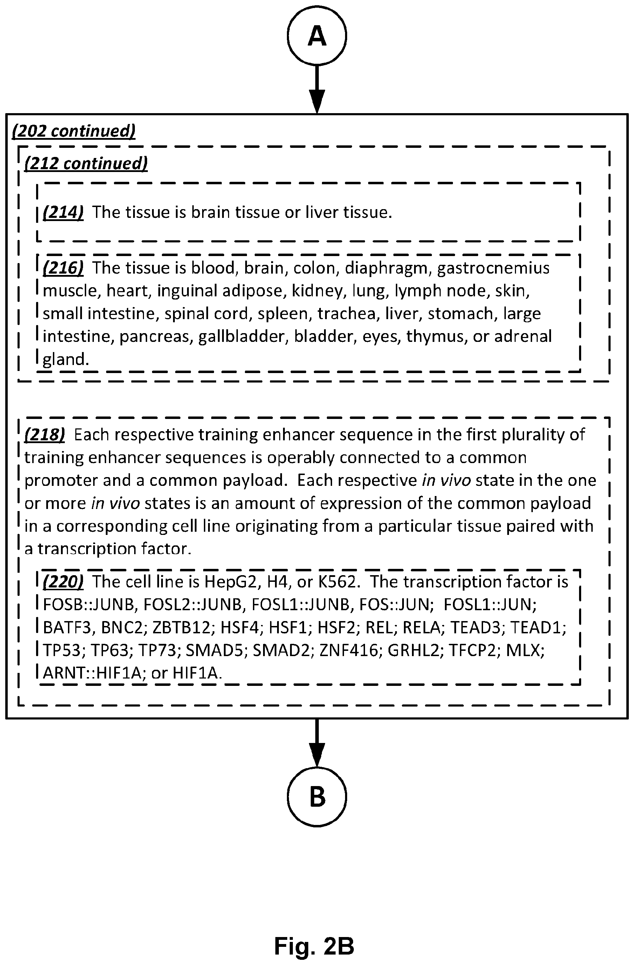

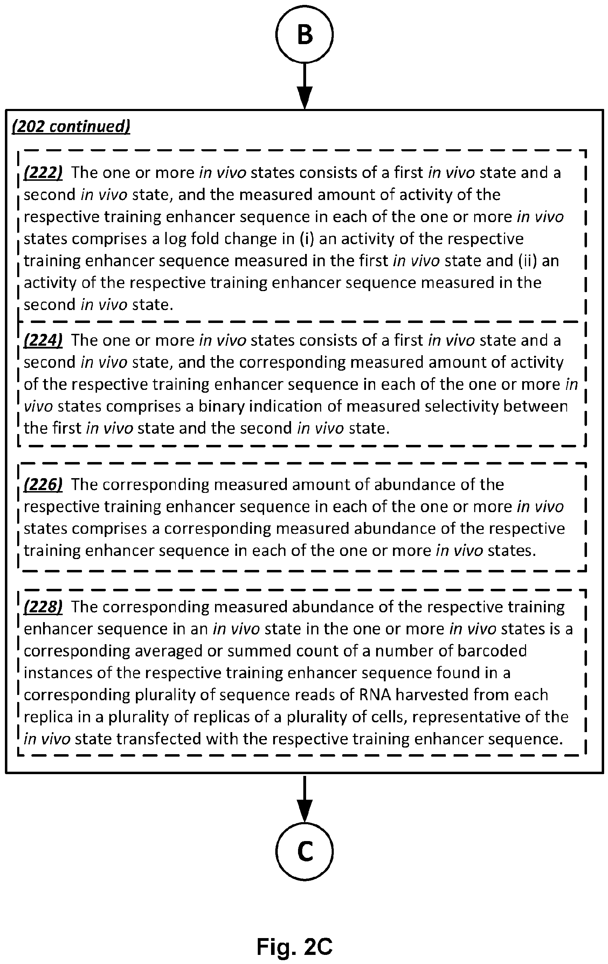

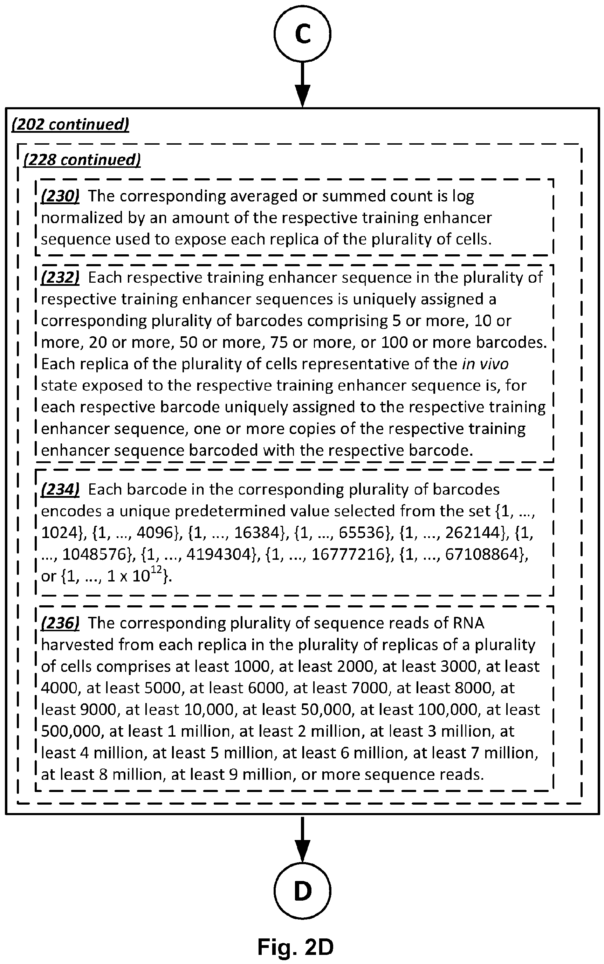

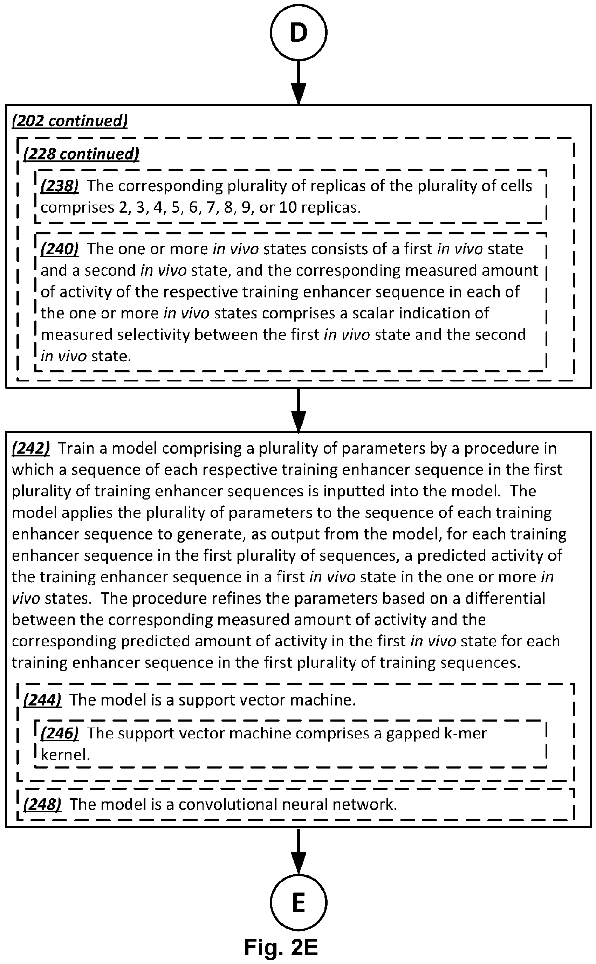

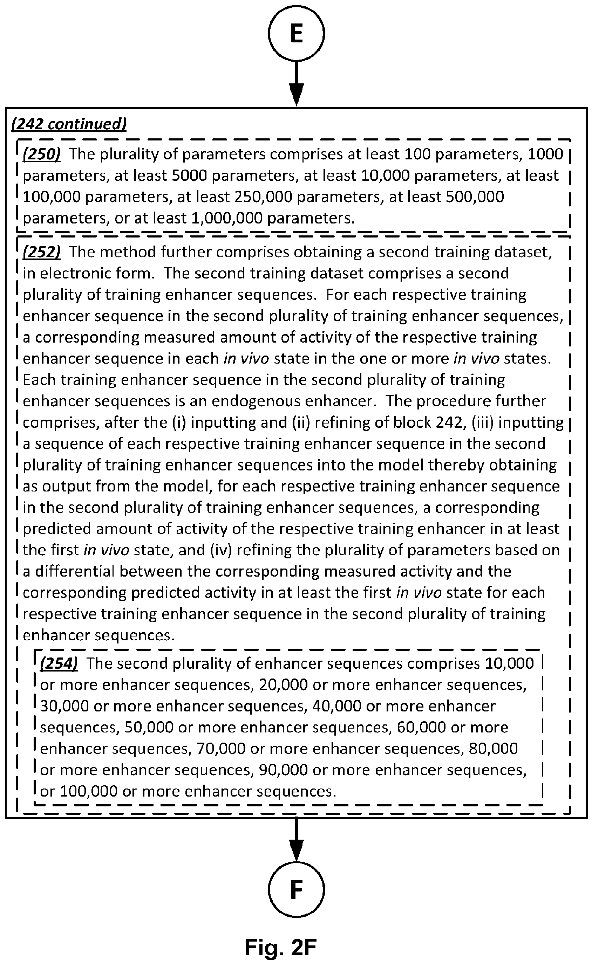

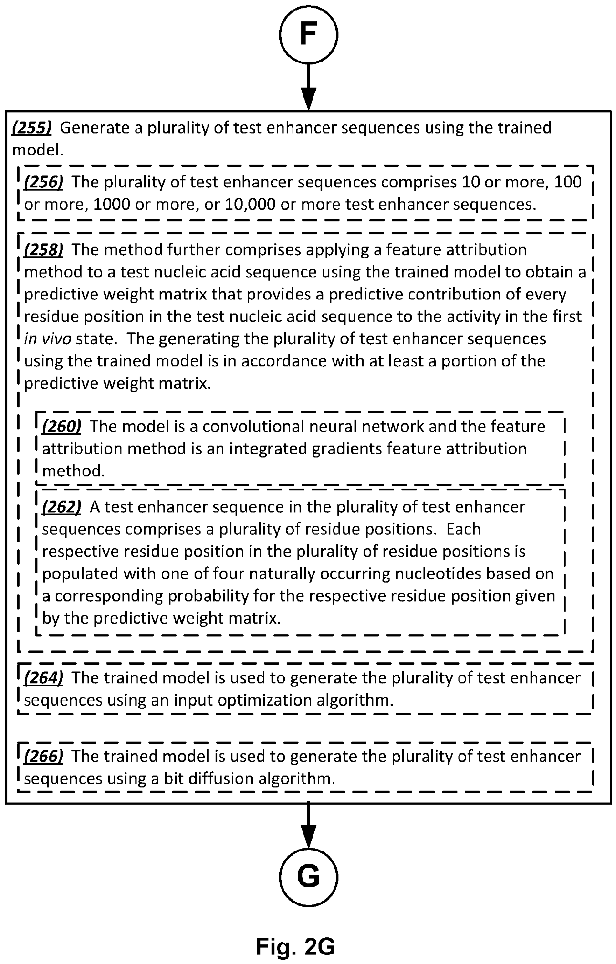

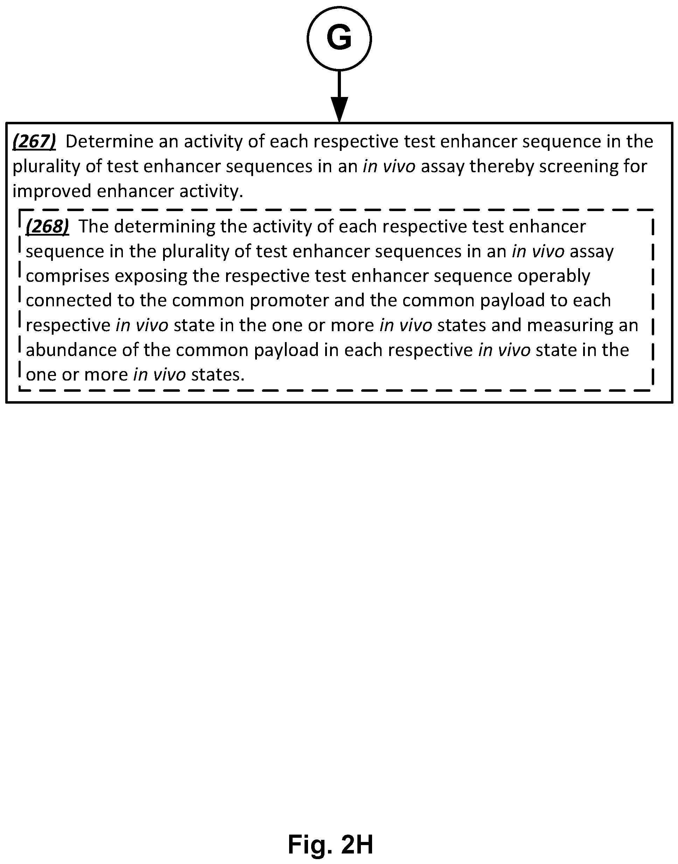

FIGs. 2A, 2B, 2C, 2D, 2E, 2F, 2G, and 2H provide an example flowchart depicting an example process for screening for improved enhancer activity, in which optional elements are denoted by dashed boxes, in accordance with some embodiments of the present disclosure.

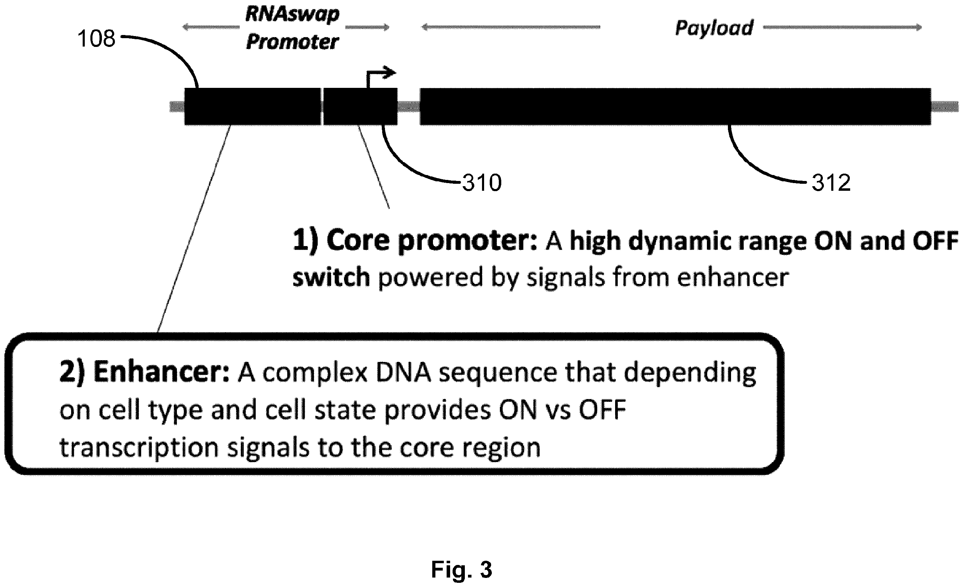

Fig. 3 illustrates a training enhancer sequence operably connected to a common promoter and a common payload in accordance with an embodiment of the present disclosure.

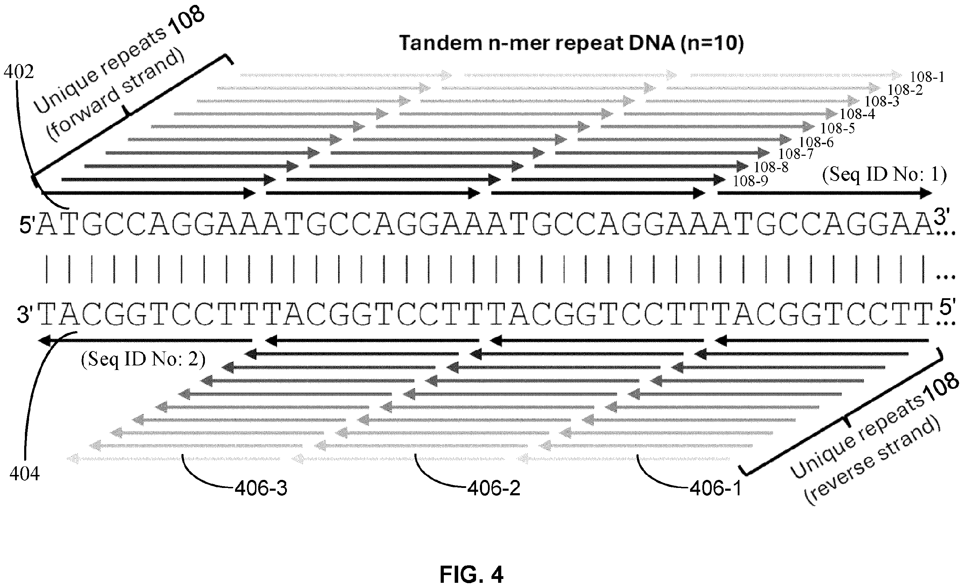

Fig. 4 illustrates the construction of a plurality of training enhancer sequences that each comprise a tandem 10-mer repeat sequence in accordance with an embodiment of the present disclosure.

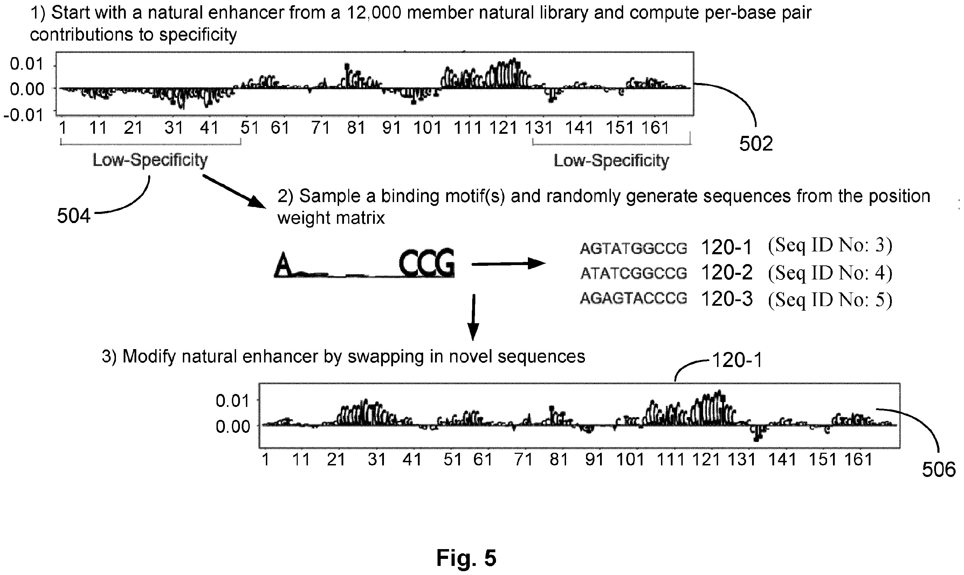

Fig. 5 illustrates applying a feature attribution method to a test nucleic acid sequence using a trained model to obtain a predictive weight matrix that provides a predictive contribution of every residue position in the test nucleic acid sequence to activity in an *in vivo* state, using the predictive weight matrix to identify regions within the test nucleic acid sequence that do not contribute to activity in the *in vivo* state, and using the predictive weight matrix to replace such regions in order to derive test enhancer sequences with improved activity based on the test nucleic acid sequence in accordance with some embodiments of the present disclosure.

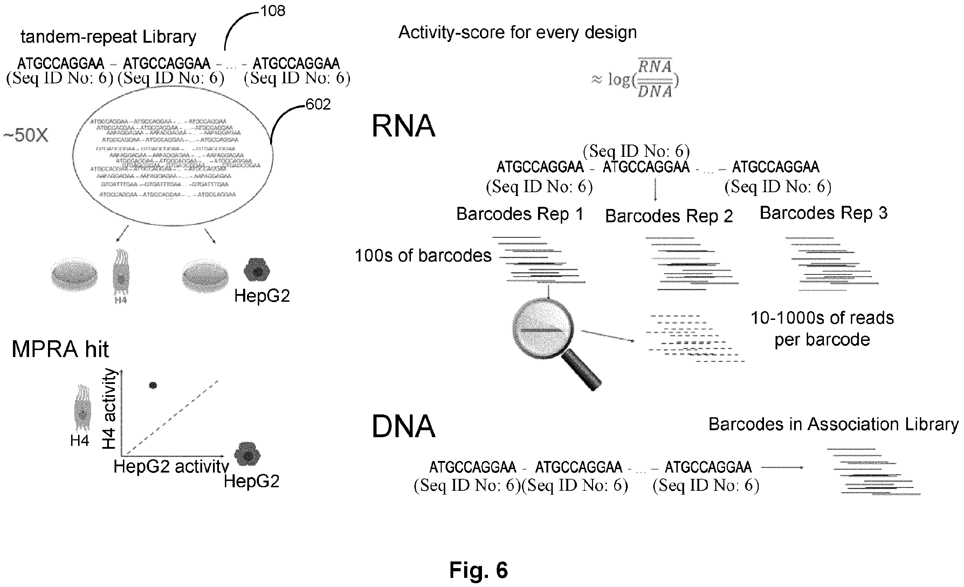

Fig. 6 illustrates how the corresponding measured abundance of a respective training enhancer sequence in an *in vivo* state is a corresponding averaged or summed count of a number of barcoded instances of the respective training enhancer sequence found in a corresponding plurality of sequence reads of RNA harvested from each replica in a plurality of replicas of a plurality of cells, representative of the *in vivo* state exposed to the respective training enhancer sequence, and how such counts can be normalized by an amount of the training enhancer sequence exposed to the *in vivo* state in accordance with an embodiment of the present disclosure. In Figure 6, the activity score for a given training enhancer sequence is a log of RNA counts to DNA counts for the given training enhancer sequence.

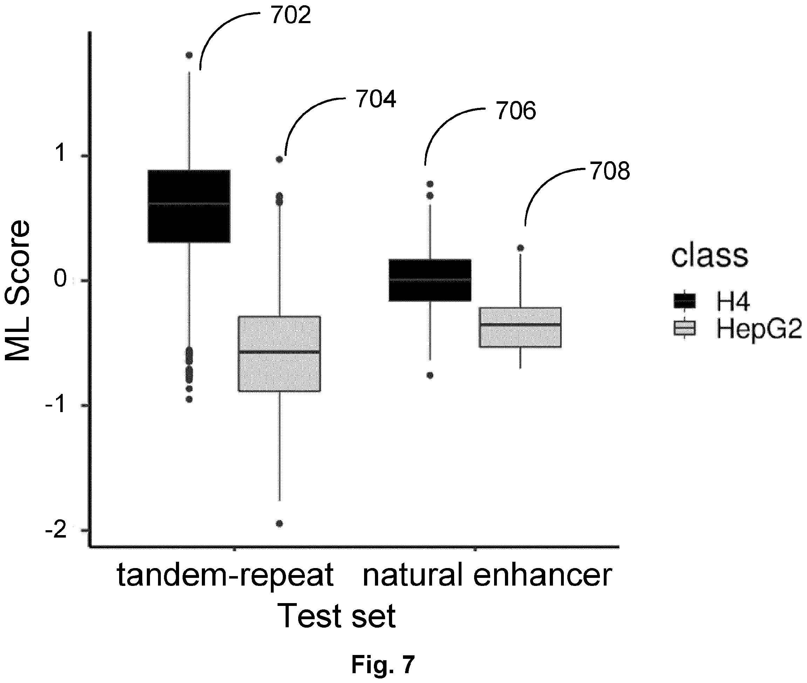

Fig. 7 illustrates training a model by inputting a sequence of each training enhancer sequence in a plurality of training enhancer sequences and generating, as output from the model, for each respective training enhancer sequence in the first plurality of training enhancer sequences, a corresponding predicted amount of activity of the respective training enhancer sequence in an *in vivo* state in accordance with an embodiment of the present invention.

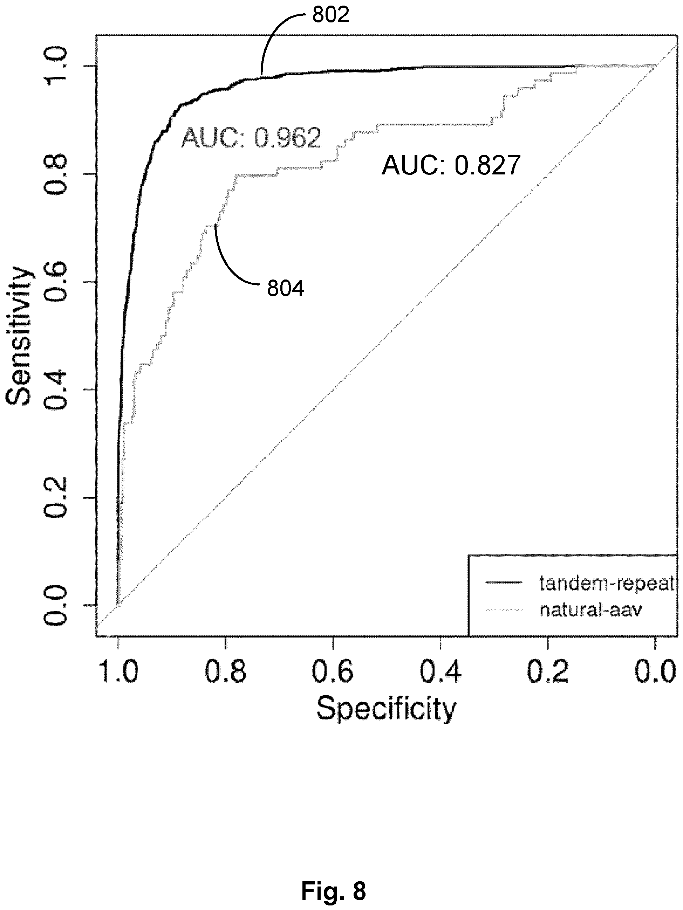

Fig. 8 illustrates the performance of the trained model of Fig. 7 both on enhancer sequences it was trained on for H4 or HepG2 activity (tandem-repeat) and on enhancer sequences for which it was not trained on for H4 or HepG2 activity (natural enhancer), in accordance with an embodiment of the present disclosure.

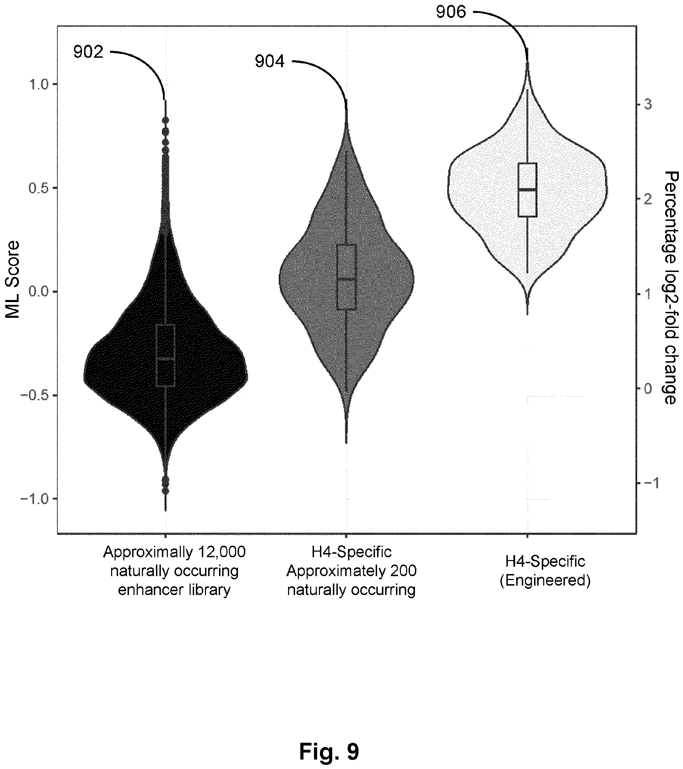

Fig. 9 illustrates generating test enhancer sequences using corresponding predictive weight matrices in accordance with an embodiment of the present disclosure. In Figure 9, is the predicted activity score for each of the sequences.

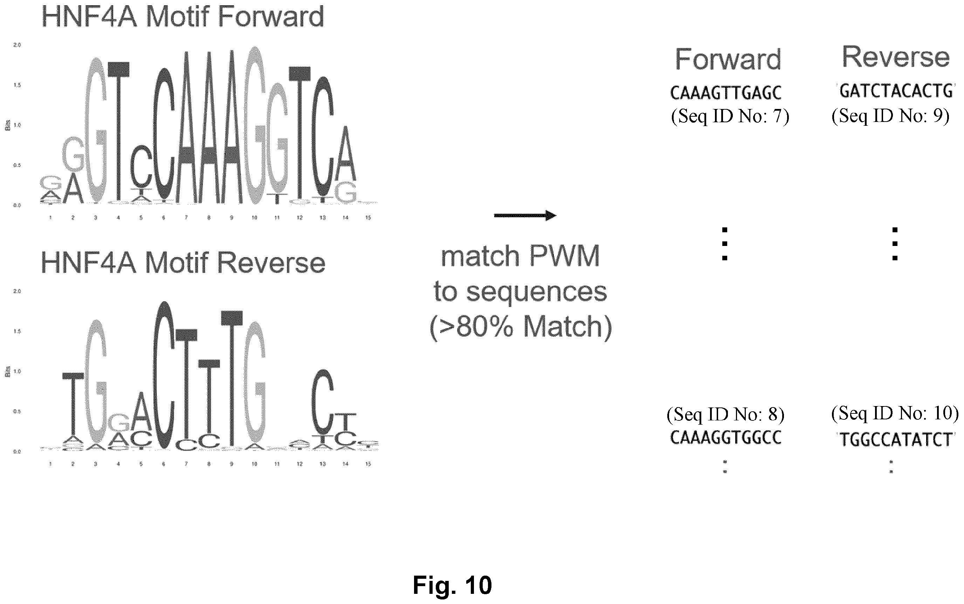

Fig. 10 illustrates the forward and reverse position weight matrices of a HNF4a transcription factor binding site and the selection of tandem 10-mer repeats, from a 50,000 10-mer tandem repeat training enhancer sequence library (Background), that have at least an 80 percent match (HNF4a match) to these matrices, in accordance with an embodiment of the present disclosure.

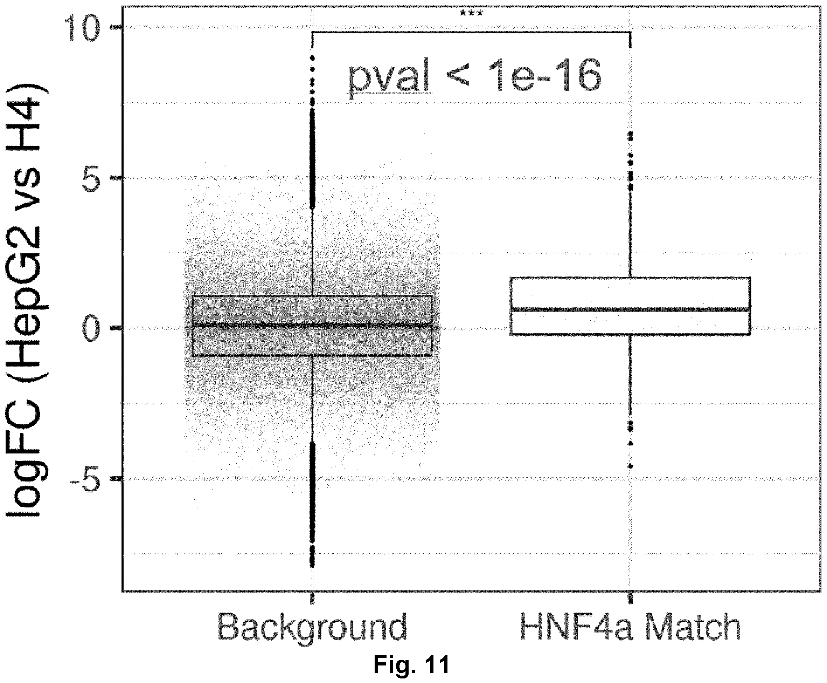

Fig. 11 illustrates how the HNF4a match sequences of Figure 10 have higher selectivity for HepG2 than the Background sequence of Figure 10 in accordance with an embodiment of the present disclosure.

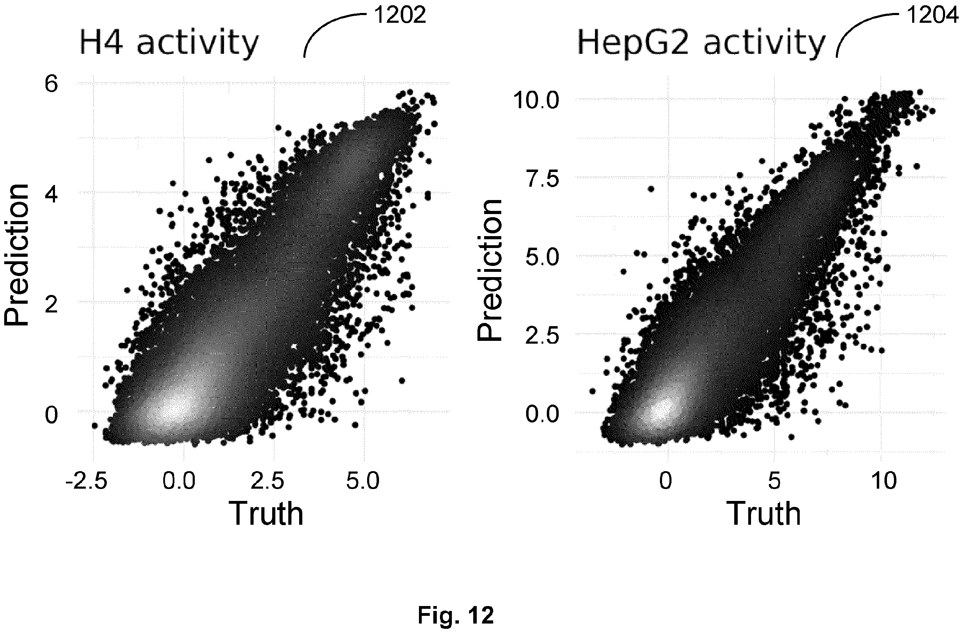

Fig. 12 illustrates the agreement between actual activity and convolutional neural network predicted activity for a 50,000 10-mer tandem repeat training enhancer sequence member dataset in H4 and HepG2 cells in accordance with an embodiment of the present disclosure.

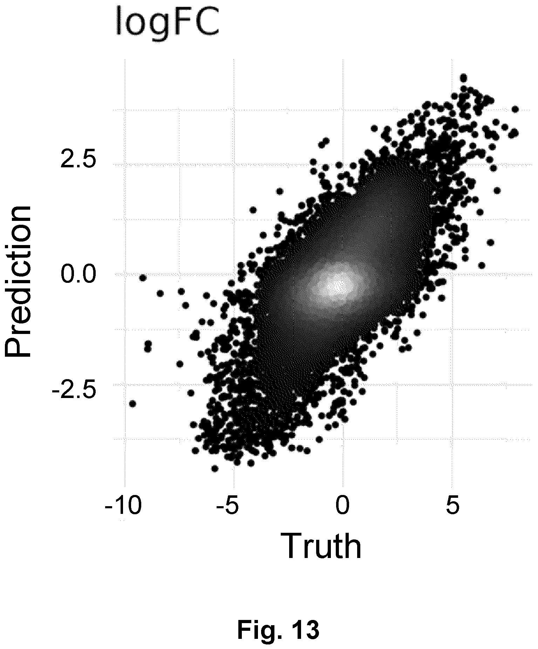

Fig. 13 illustrates the agreement between (i) the log fold change of predicted H2 activity to predicted HepG2 activity and (ii) actual log fold change of H2 activity to HepG2 activity of each of the tandem repeat training enhancer sequences in a 50,000 10-mer tandem repeat training enhancer sequence member dataset in accordance with an embodiment of the present disclosure.

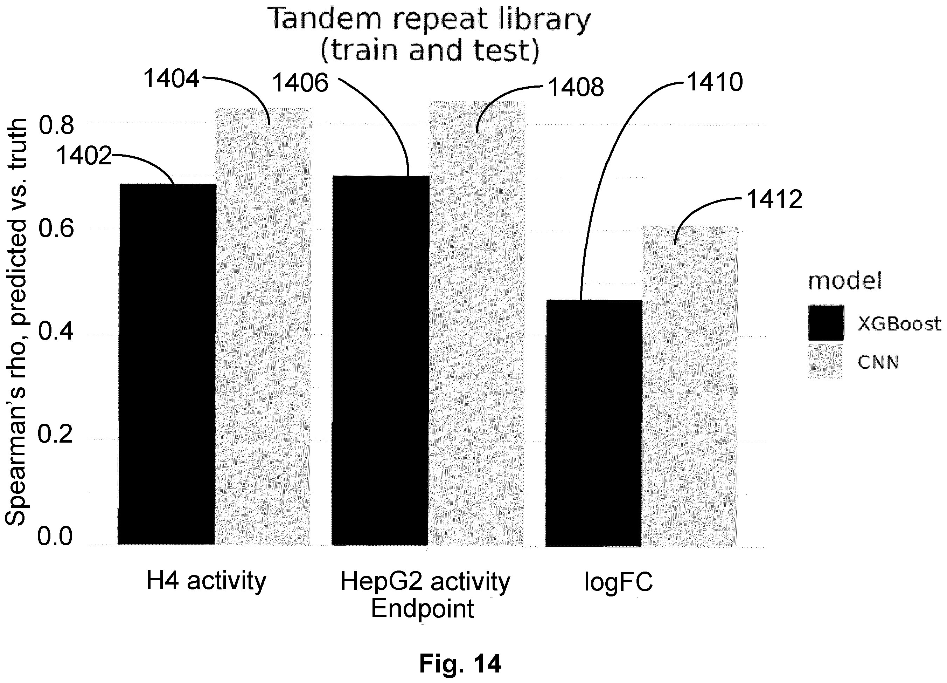

Fig. 14 illustrates the performance (Spearman's rho, predicted versus truth) for the agreement between (i) predicted H4 or HepG2 activity of each of the tandem repeat training enhancer sequences in a 50,000 10-mer tandem repeat training enhancer sequence member dataset from either a trained convolutional neural network or a trained XGBoost model and (ii) their actual H4 or HepG2 activity in replicas of H4 or HepG2 cells as well as the agreement between (i) the log fold change of predicted H2 to predicted HepG2 activity and (ii) actual log fold change of H2 activity to HepG2 activity of each of the tandem repeat training enhancer sequences in replicas of HepG2 cells for the CNN model or the XGBoost model in accordance with an embodiment of the present disclosure.
Fig. 15A compares the performance of (i) models trained using tandem repeat training enhancer sequences at identifying H4 cell activity and HepG2 cell activity and (ii) models trained using naturally occurring enhancer sequences in accordance with an embodiment of the present disclosure.

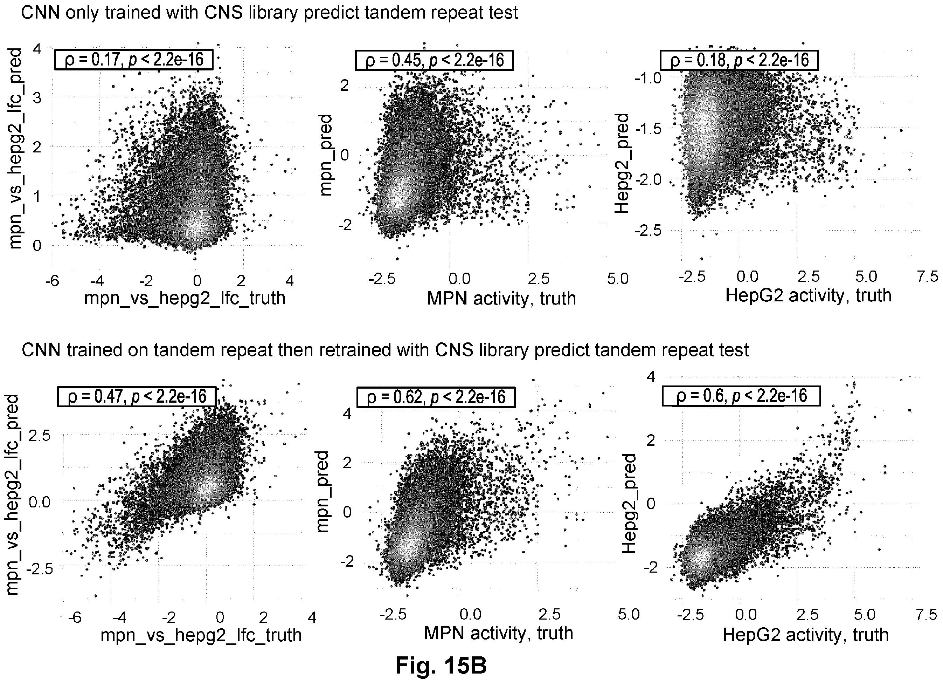

Fig. 15B compares the performance of (i) CNN models trained using only naturally occurring enhancer sequences versus (ii) CNN models first trained using tandem repeat training enhancer sequences and then retrained using naturally occurring enhancer sequences in determining MPN versus HEPG2 specificity, MPN activity, and HepG2 activity in accordance with an embodiment of the present disclosure.

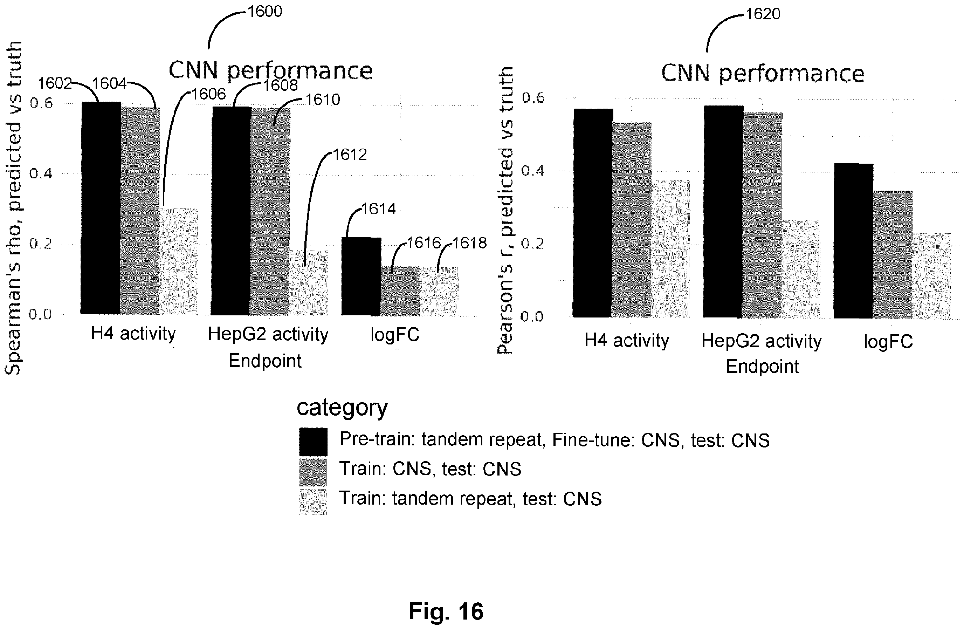

Fig. 16 illustrates how pre-training a model for H4 and HepG2 cell specific activity using tandem repeat training enhancer sequences followed by using transfer learning to train the model on endogenous enhancers lead to better model performance, in terms of Spearman's rho and Pearson's r, in predicting the H4 and HepG2 cell specific activity of the endogenous enhancers in accordance with an embodiment of the present disclosure.

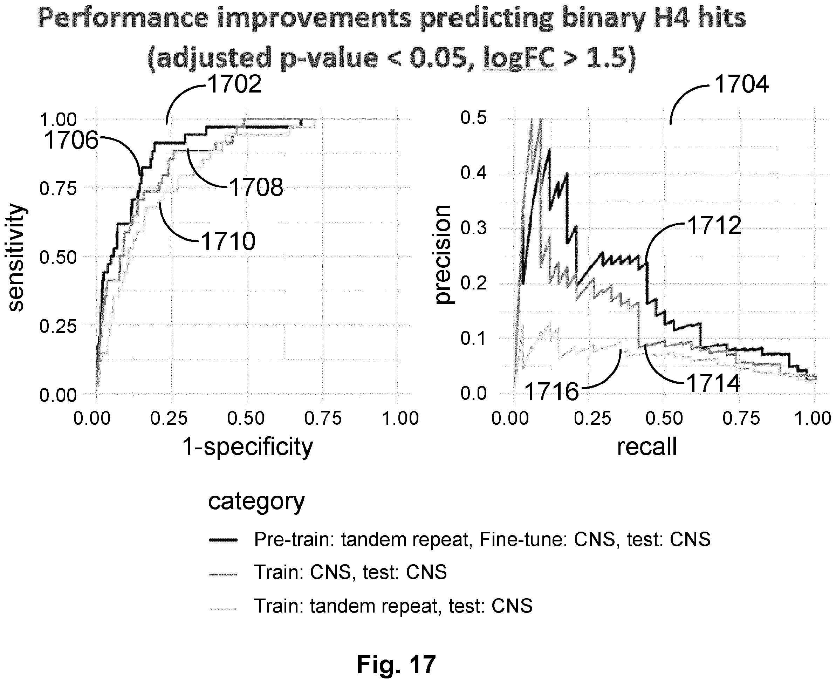

Fig. 17 illustrates how pre-training a model for H4 cell specific activity using tandem repeat training enhancer sequences followed by using transfer learning to train the model on endogenous enhancers lead to moderately enhanced model performance, in terms of Area Under the Curve values and precision / recall values, in predicting the H4 cell activity of the endogenous enhancers in accordance with an embodiment of the present disclosure.

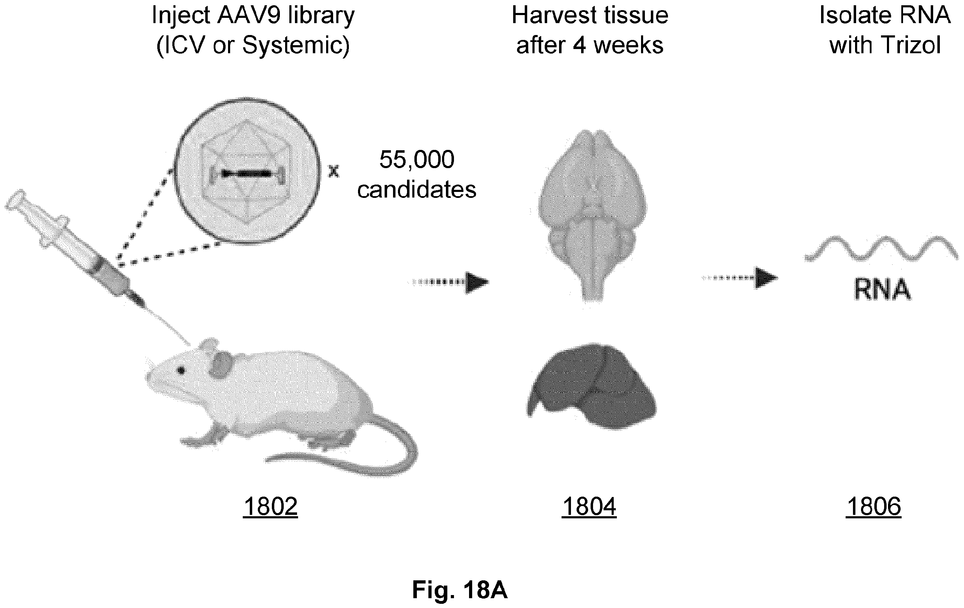

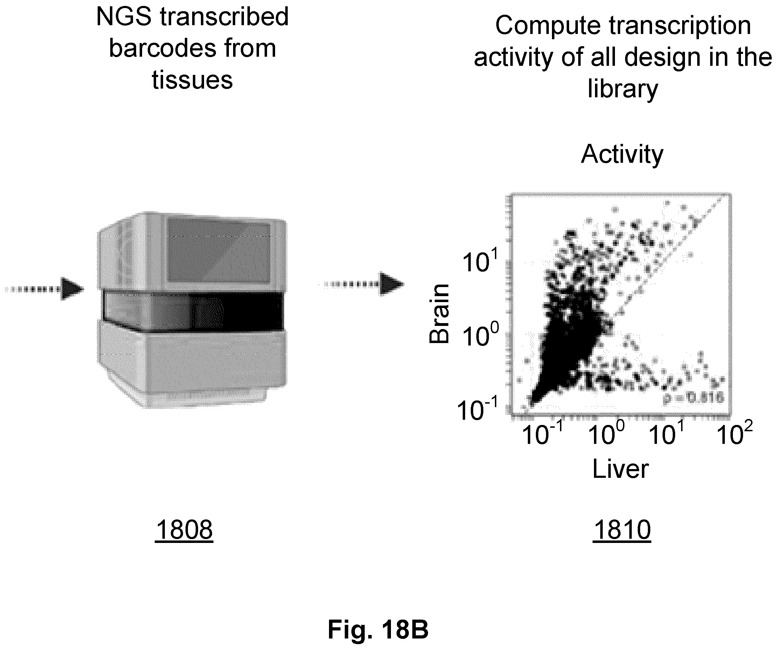

Figs. 18A and 18B illustrate an exemplary mechanism by which corresponding measured amounts of activity of respective training enhancer sequences in two different *in vivo* states, where the *in vivo* states are particular tissues, can be obtained in accordance with the present disclosure.

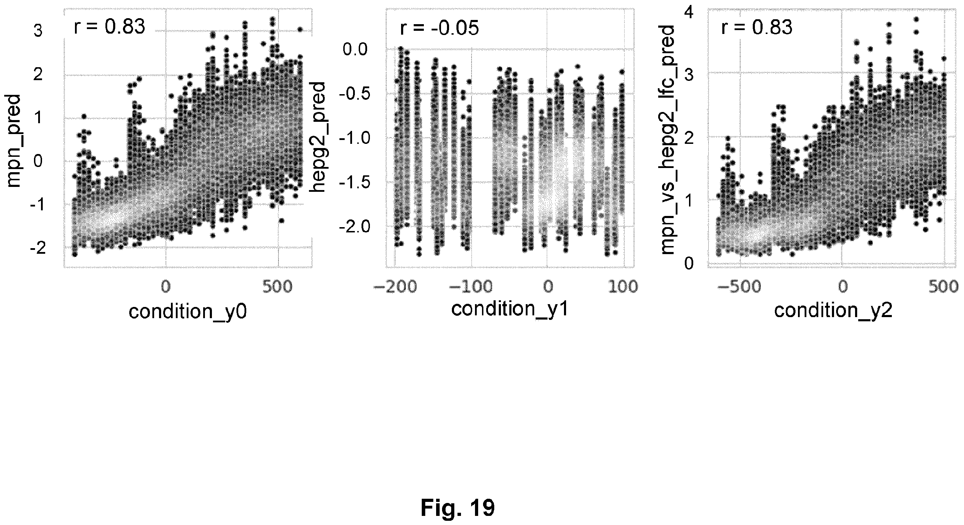

Fig. 19 illustrates example performance of a model for generating enhancer sequences from seeds, where performance is measured *in silico* using a correlation between the conditioning used for bit diffusion and a prediction using a separate model trained on the same dataset, in accordance with an embodiment of the present disclosure.

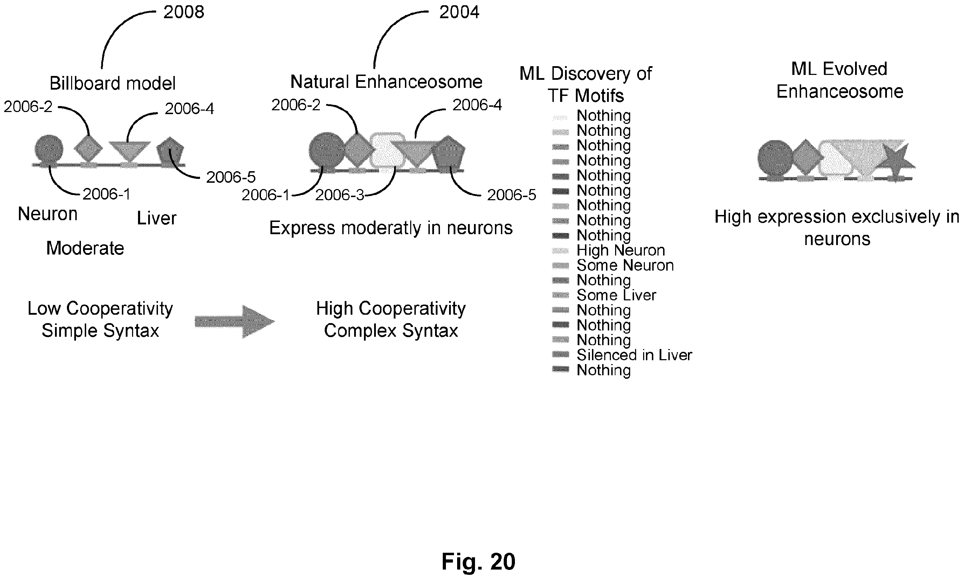

Fig. 20 illustrates an enhancer sequence adhering to a billboard model with low cooperatively simple syntax among the binding elements of the enhancer sequence, an enhancer adhering to a natural enhanceosome model with high cooperatively complex syntax among the binding elements of the enhancer sequence, observation of differential activity of the respective training enhancer sequence in each of a plurality of *in vivo* states, and the construction of an evolved enhancer with improved enhancer activity, which is also optionally tissue specific, in accordance with an embodiment of the present disclosure.

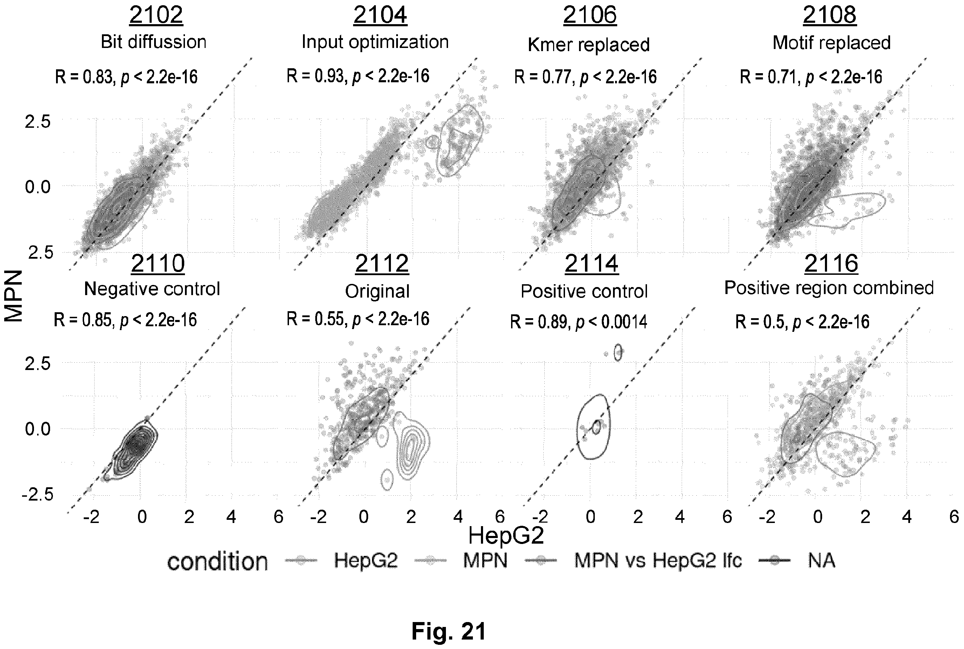

Fig. 21 illustrates the mouse primary neuron (MPN) cell line transcriptional activity versus HepG2 cell line transcriptional activity of test enhancer sequences that have been engineered using bit diffusion, input optimization, k-mer replacement, motif replacement, and positive region combined methods in accordance with an embodiment of the present disclosure.

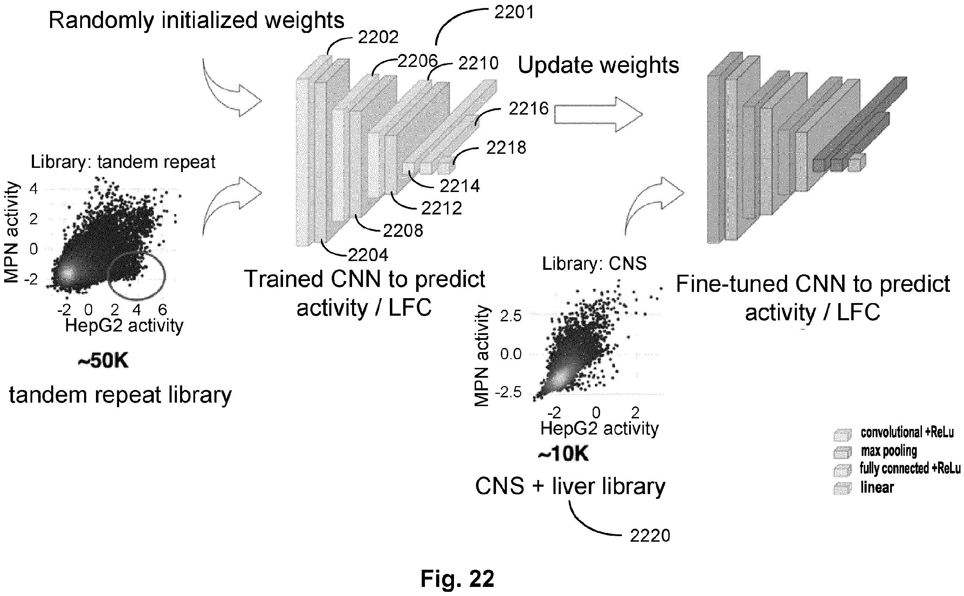

Fig. 22 illustrates the use of transfer learning to train a convolutional neural network, pre-trained using a training set comprising 50K tandem 10-mer training enhancer sequences, with a second training set comprising 10K biologically informed training enhancer sequences in accordance with an embodiment of the present disclosure.

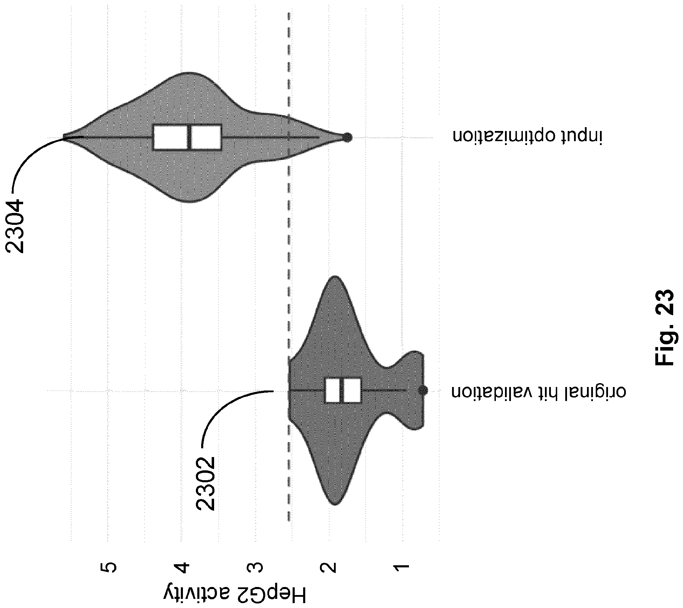

Fig. 23 illustrates how a convolutional neural network, which has been first trained on a 10-mer tandem repeat training enhancer library and then trained on a biologically informed enhancer library, is input optimized for HepG2 transcriptional activity to produce proposed enhancers that exhibit, as a group, higher average transcriptional activity in HepG2 cells than the original enhancers prior to input optimization, in accordance with an embodiment of the present disclosure.

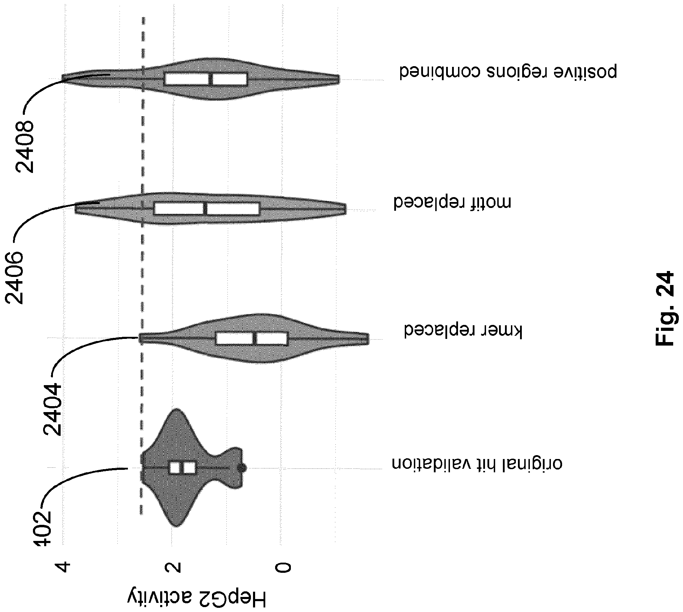

Fig. 24 provides a comparison of the HepG2 transcriptional activity results from original hit validation (panel 2402), subjecting these original hits to k-mer replacement (panel 2404), subjecting these original hits to motif replacement (panel 2406), and combining positive regions of enhancers in the original hit validation (panel 2408), in accordance with an embodiment of the present disclosure.

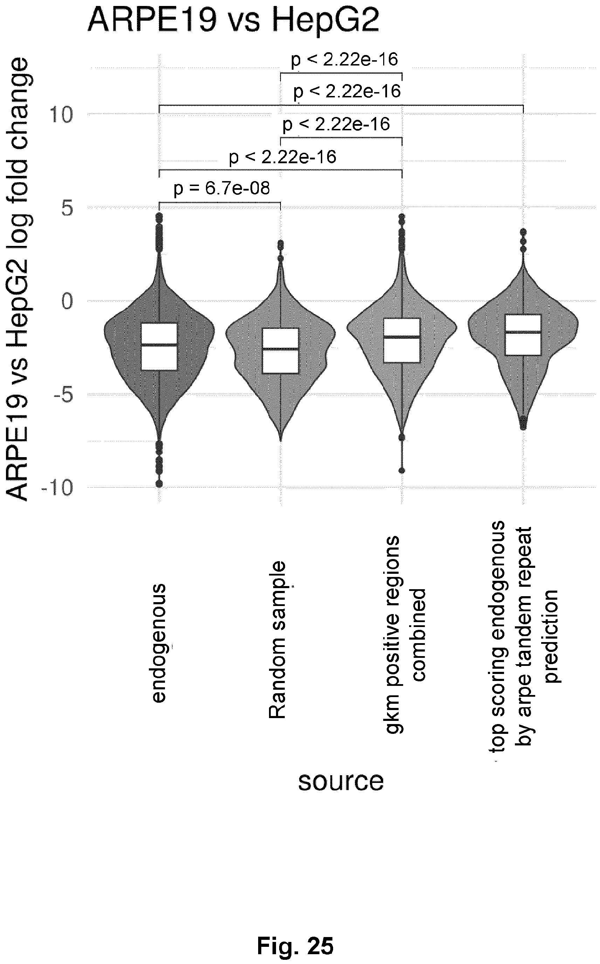

Fig. 25 compares the selectivity of enhancers, selected by four different methods, between ARPE19 and HepG2 cells, in accordance with an embodiment of the present disclosure.

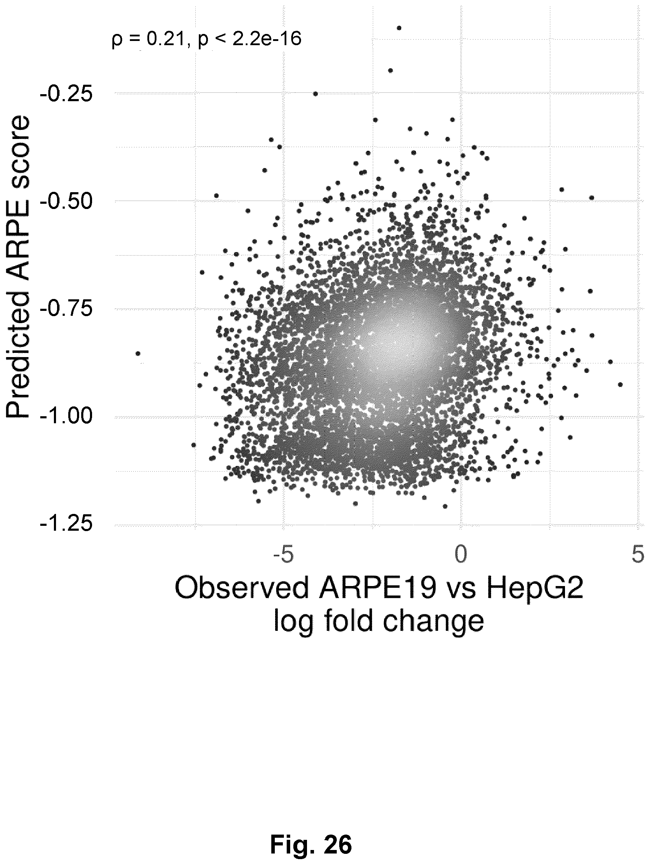

Fig. 26 is a plot of the predicted ARPE19 activity of enhancers made by a tandem repeat library trained model versus the measured ARPE19 versus HepG2 log fold change in activity for these enhancers, in accordance with an embodiment of the present disclosure.

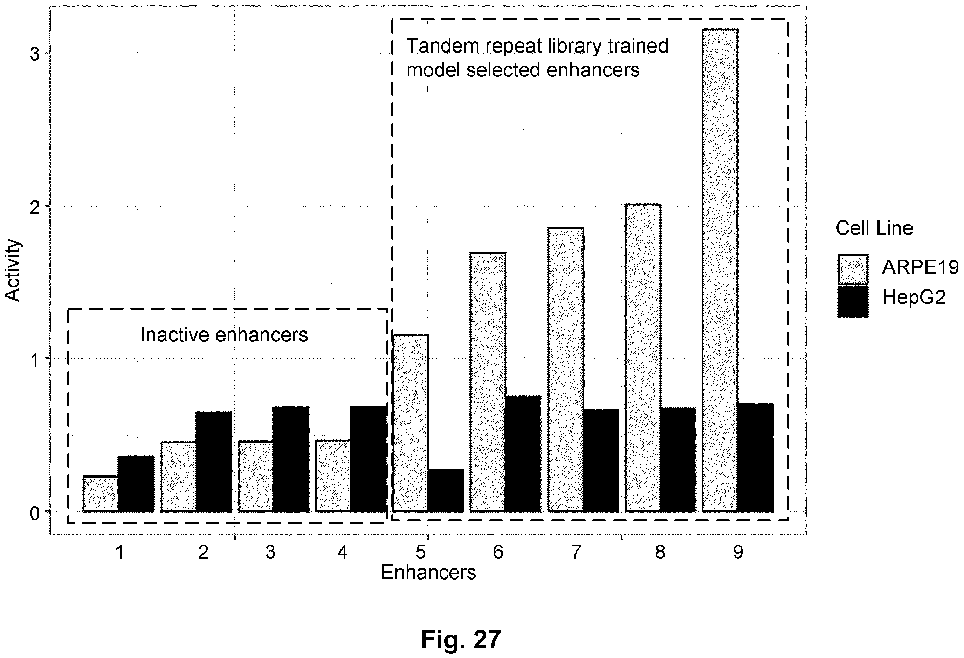

Fig. 27 is a plot of the actual ARPE19 activity and actual HepG2 activity of five enhancers identified using a tandem repeat library trained model compared to four random inactive enhancers, in accordance with an embodiment of the present disclosure.

### DETAILED DESCRIPTION

Given the above background, the present disclosure provides systems and methods for screening for enhancer activity using a training dataset comprising training enhancer sequences and, for each respective sequence, a corresponding measured activity of the respective sequence in each of one or more *in vivo* states. Each sequence is a tandem n-mer repeat. A model comprising a plurality of parameters is trained by inputting each sequence into the model thereby obtaining as output from the model, for each sequence, a predicted amount of activity of the respective sequence in each of the one or more *in vivo* states. The procedure refines the parameters based on differentials between the corresponding measured activity and the corresponding predicted activity for the training enhancer sequences. A plurality of test enhancer sequences is generated using the trained model. An activity of each respective test enhancer sequence in the plurality of test enhancer sequences is determined in an *in vivo* assay thereby screening for improved enhancer activity.

Reference will now be made in detail to embodiments, examples of which are illustrated in the accompanying drawings. In the following detailed description, numerous specific details are set forth in order to provide a thorough understanding of the present disclosure. However, it will be apparent to one of ordinary skill in the art that the present disclosure may be practiced without these specific details. In other instances, well-known methods, procedures, components, circuits, and networks have not been described in detail so as not to unnecessarily obscure aspects of the embodiments.

Plural instances may be provided for components, operations or structures described herein as a single instance. Finally, boundaries between various components, operations, and data stores are somewhat arbitrary, and particular operations are illustrated in the context of specific illustrative configurations. Other forms of functionality are envisioned and may fall within the scope of the implementation(s). In general, structures and functionality presented as separate components in the example configurations may be implemented as a combined structure or component. Similarly, structures and functionality presented as a single component may be implemented as separate components. These and other variations, modifications, additions, and improvements fall within the scope of the implementation(s).

It will also be understood that, although the terms "first," "second," *etc.* may be used herein to describe various elements, these elements should not be limited by these terms. These terms are only used to distinguish one element from another. For example, a first dataset could be termed a second dataset, and, similarly, a second dataset could be termed a first dataset, without departing from the scope of the present invention. The first dataset and the second dataset are both datasets, but they are not the same dataset.

The terminology used herein is for the purpose of describing particular implementations only and is not intended to be limiting of the claims. As used in the description of the implementations and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if' may be construed to mean "when" or "upon" or "in response to determining" or "in accordance with a determination" or "in response to detecting," that a stated condition precedent is true, depending on the context. Similarly, the phrase "if it is determined (that a stated condition precedent is true)" or "if (a stated condition precedent is true)" or "when (a stated condition precedent is true)" may be construed to mean "upon determining" or "in response to determining" or "in accordance with a determination" or "upon detecting" or "in response to detecting" that the stated condition precedent is true, depending on the context.

Furthermore, when a reference number is given an "*i*^{th}" denotation, the reference number refers to a generic component, set, or embodiment. For instance, a cellular-component termed "cellular-component *i"* refers to the *i*^{th} cellular-component in a plurality of cellular-components.

In the interest of clarity, not all of the routine features of the implementations described herein are shown and described. It will be appreciated that, in the development of any such actual implementation, numerous implementation-specific decisions are made in order to achieve the designer's specific goals, such as compliance with use case- and business-related constraints, and that these specific goals will vary from one implementation to another and from one designer to another. Moreover, it will be appreciated that such a design effort might be complex and time-consuming, but nevertheless be a routine undertaking of engineering for those of ordering skill in the art having the benefit of the present disclosure.

Some portions of this description describe the embodiments of the invention in terms of algorithms and symbolic representations of operations on information. These algorithmic descriptions and representations are commonly used by those skilled in the data processing arts to convey the substance of their work effectively to others skilled in the art. These operations, while described functionally, computationally, or logically, are understood to be implemented by computer programs or equivalent electrical circuits, microcode, or the like.

The language used in the specification has been principally selected for readability and instructional purposes, and it may not have been selected to delineate or circumscribe the inventive subject matter. It is therefore intended that the scope of the invention be limited not by this detailed description, but rather by any claims that issue on an application based hereon. Accordingly, the disclosure of the embodiments of the invention is intended to be illustrative, but not limiting, of the scope of the invention.

In general, terms used in the claims and the specification are intended to be construed as having the plain meaning understood by a person of ordinary skill in the art. Certain terms are defined below to provide additional clarity. In case of conflict between the plain meaning and the provided definitions, the provided definitions are to be used.

Any terms not directly defined herein shall be understood to have the meanings commonly associated with them as understood within the art of the invention. Certain terms are discussed herein to provide additional guidance to the practitioner in describing the compositions, devices, methods and the like of aspects of the invention, and how to make or use them. It will be appreciated that the same thing may be said in more than one way. Consequently, alternative language and synonyms may be used for any one or more of the terms discussed herein. No significance is to be placed upon whether a term is elaborated or discussed herein. Some synonyms or substitutable methods, materials and the like are provided. Recital of one or a few synonyms or equivalents does not exclude use of other synonyms or equivalents, unless it is explicitly stated. Use of examples, including examples of terms, is for illustrative purposes only and does not limit the scope and meaning of the aspects of the invention herein.

### Definitions

Unless defined otherwise, all technical and scientific terms used herein have the meaning commonly understood by one of ordinary skill in the art to which the invention pertains.

As used herein, the term "endogenous enhancer" refers to an enhancer that is either naturally occurring or that has at least 60 percent, at least 70 percent, at least 80 percent, at least 90 percent, or at least 99 percent sequence identity a naturally occurring enhancer.

As used herein, the terms "abundance," "abundance level," or "expression level" refers to an amount of a cellular constituent (*e.g*., a gene product such as an RNA species, *e.g*., mRNA or miRNA, or a protein molecule) present in one or more cells, or an average amount of a cellular constituent present across multiple cells. When referring to mRNA or protein expression, the term generally refers to the amount of any RNA or protein species corresponding to a particular genomic locus, *e.g*., a particular gene. However, in some embodiments, an abundance can refer to the amount of a particular isoform of an mRNA or protein corresponding to a particular gene that gives rise to multiple mRNA or protein isoforms. The genomic locus can be identified using a gene name, a chromosomal location, or any other genetic mapping metric.

As used interchangeably herein, a "cell state" or "biological state" refers to a state or phenotype of a cell or a population of cells. For example, a cell state can be healthy or diseased. A cell state can be one of a plurality of diseases. A cell state can be a response to a compound treatment and/or a differentiated cell lineage. A cell state can be characterized by a measure (*e.g*., an activation, expression, and/or measure of abundance) of one or more cellular constituents, including but not limited to one or more genes, one or more proteins, and/or one or more biological pathways.

As used herein, "messenger RNA" or "mRNA" are RNA molecules comprising a sequence that encodes a polypeptide or protein. In general, RNA can be transcribed from DNA. In some cases, precursor mRNA containing non-protein coding regions in the sequence can be transcribed from DNA and then processed to remove all or a portion of the non-coding regions (introns) to produce mature mRNA. As used herein, the term "pre-mRNA" can refer to the RNA molecule transcribed from DNA before undergoing processing to remove the non-protein coding regions.

As used herein, unless otherwise dictated by context "nucleotide" or "nt" refers to ribonucleotide.

As used herein, the terms "patient" and "subject" are used interchangeably, and may be taken to mean any living organism which may be treated with compounds of the present invention. As such, the terms "patient" and "subject" include, but are not limited to, any non-human mammal, primate and human.

The terms "sequence reads" or "reads," used interchangeably herein, refer to nucleotide sequences produced by any sequencing process described herein or known in the art. Reads can be generated from one end of nucleic acid fragments ("single-end reads"), and sometimes are generated from both ends of nucleic acids (*e.g*., paired-end reads, double-end reads). The length of the sequence read is often associated with the particular sequencing technology. High-throughput methods, for example, provide sequence reads that can vary in size from tens to hundreds of base pairs (bp). In some embodiments, the sequence reads are of a mean, median or average length of about 15 bp to 900 bp long (*e.g*.i*,* about 20 bp, about 25 bp, about 30 bp, about 35 bp, about 40 bp, about 45 bp, about 50 bp, about 55 bp, about 60 bp, about 65 bp, about 70 bp, about 75 bp, about 80 bp, about 85 bp, about 90 bp, about 95 bp, about 100 bp, about 110 bp, about 120 bp, about 130 bp, about 140 bp, about 150 bp, about 200 bp, about 250 bp, about 300 bp, about 350 bp, about 400 bp, about 450 bp, or about 500 bp. In some embodiments, the sequence reads are of a mean, median or average length of about 1000 bp or more. Nanopore sequencing, for example, can provide sequence reads that vary in size from tens to hundreds to thousands of base pairs. Illumina parallel sequencing can provide sequence reads vary to a lesser extent (*e.g*., where most sequence reads are of a length of about 200 bp or less). A sequence read (or sequencing read) can refer to sequence information corresponding to a nucleic acid molecule (*e.g*., a string of nucleotides). For example, a sequence read can correspond to a string of nucleotides (*e.g*., about 20 to about 150) from part of a nucleic acid fragment, can correspond to a string of nucleotides at one or both ends of a nucleic acid fragment, or can correspond to nucleotides of the entire nucleic acid fragment. A sequence read can be obtained in a variety of ways, *e.g.,* using sequencing techniques or using probes (*e.g.*, in hybridization arrays or capture probes) or amplification techniques, such as the polymerase chain reaction (PCR) or linear amplification using a single primer or isothermal amplification.

As disclosed herein, the terms "sequencing," "sequence determination," and the like refer generally to any and all biochemical processes that may be used to determine the order of biological macromolecules such as nucleic acids or proteins. For example, sequencing data can include all or a portion of the nucleotide bases in a nucleic acid molecule such as a DNA fragment.

As used herein, the term "tissue" corresponds to a group of cells that group together as a functional unit. More than one type of cell can be found in a single tissue. Different types of tissue may consist of different types of cells (*e.g*., hepatocytes, alveolar cells or blood cells), but also can correspond to tissue from different organisms (mother vs. fetus) or to healthy cells vs. tumor cells. The term "tissue" can generally refer to any group of cells found in the human body (*e.g*., heart tissue, lung tissue, kidney tissue, nasopharyngeal tissue, oropharyngeal tissue). In some aspects, the term "tissue" or "tissue type" can be used to refer to a tissue from which a cell-free nucleic acid originates. In one example, viral nucleic acid fragments can be derived from blood tissue. In another example, viral nucleic acid fragments can be derived from tumor tissue.

As used interchangeably herein, the terms "algorithm", "model", "regressor", and/or "classifier" refers to a machine learning model and/or artificial intelligence model. In some embodiments, a model makes use of supervised machine learning. Nonlimiting examples of supervised learning models include, but are not limited to, logistic regression, neural networks, support vector machines, Naive Bayes models, nearest neighbor models, random forest models, decision tree models, boosted trees models, multinomial logistic regression models, linear models, linear regression, Gradient Boosting, mixture models, Gaussian NB model, linear discriminant analysis, diffusion models, or any combinations thereof. In some embodiments, a model is a multinomial classifier. In some embodiments, a model is a 2-stage stochastic gradient descent (SGD) model. In some embodiments, a model is a deep neural network (*e.g*., a deep-and-wide sample-level model).

*Neural networks.* In some embodiments, the model is a neural network (e*e.g*, a convolutional neural network and/or a residual neural network). Neural networks, also known as artificial neural networks (ANNs or NNs), include convolutional and/or residual neural network networks (deep learning models). In some embodiments, neural networks are trained to map an input dataset to an output dataset, where the neural network includes an interconnected group of nodes organized into multiple layers of nodes. For example, in some embodiments, the neural network includes at least an input layer, one or more hidden layers, and an output layer. In some embodiments, the neural network includes any total number of layers, and any number of hidden layers, where the hidden layers function as trainable feature extractors that allow mapping of a set of input data to an output value or set of output values. In some embodiments, a deep learning model is a neural network including a plurality of hidden layers, *e.g*., two or more hidden layers. In some instances, each layer of the neural network includes a number of nodes (or "neurons"). In some embodiments, a node receives input that comes either directly from the input data or the output of nodes in previous layers, and performs a specific operation, *e.g*., a summation operation. In some embodiments, a connection from an input to a node is associated with a parameter (*e.g*., a weight and/or weighting factor). In some embodiments, the node sums up the products of all pairs of inputs, xᵢ, and their associated parameters. In some embodiments, the weighted sum is offset with a bias, b. In some embodiments, the output of a node or neuron is gated using a threshold or activation function, f, which, in some instances, is a linear or non-linear function. In some embodiments, the activation function is, for example, a rectified linear unit (ReLU) activation function, a Leaky ReLU activation function, or other function such as a saturating hyperbolic tangent, identity, binary step, logistic, arcTan, softsign, parametric rectified linear unit, exponential linear unit, softPlus, bent identity, softExponential, Sinusoid, Sine, Gaussian, or sigmoid function, or any combination thereof.

In some embodiments, the weighting factors, bias values, and threshold values, or other computational parameters of the neural network, are "taught" or "learned" in a training phase using one or more sets of training data. For example, in some implementations, the parameters are trained using the input data from a training dataset and a gradient descent or backward propagation method so that the output value(s) that the neural network computes are consistent with the examples included in the training dataset. In some embodiments, the parameters are obtained from a back propagation neural network training process.

Any of a variety of neural networks are suitable for use in accordance with the present disclosure. Examples include, but are not limited to, feedforward neural networks, radial basis function networks, recurrent neural networks, residual neural networks, convolutional neural networks, residual convolutional neural networks, and the like, or any combination thereof. In some embodiments, the machine learning makes use of a pre-trained and/or transfer-learned neural network or deep learning architecture. In some implementations, convolutional and/or residual neural networks are used, in accordance with the present disclosure.

For instance, a deep neural network model includes an input layer, a plurality of individually parameterized (*e.g*., weighted) convolutional layers, and an output scorer. The parameters (*e.g*., weights) of each of the convolutional layers as well as the input layer contribute to the plurality of parameters (*e.g*., weights) associated with the deep neural network model. In some embodiments, at least 50 parameters, at least 100 parameters, at least 1000 parameters, at least 2000 parameters or at least 5000 parameters are associated with the deep neural network model. As such, a deep neural network models requires a computer to be used because they cannot be mentally solved. In other words, given an input to the model, the model output needs to be determined using a computer rather than mentally in such embodiments. *See,* for example, Krizhevsky et al., 2012, "Imagenet classification with deep convolutional neural networks," in Advances in Neural Information Processing Systems 2, Pereira, Burges, Bottou, Weinberger, eds., pp. 1097-1105, Curran Associates, Inc.; Zeiler, 2012 "ADADELTA: an adaptive learning rate method," CoRR, vol. abs/1212.5701; and Rumelhart et al., 1988, "Neurocomputing: Foundations of research," ch. Learning Representations by Back-propagating Errors, pp. 696-699, Cambridge, MA, USA: MIT Press, each of which is hereby incorporated by reference.

Neural network models, including convolutional neural networks, suitable for use as models are disclosed in, for example, Vincent et al., 2010, "Stacked denoising autoencoders: Learning useful representations in a deep network with a local denoising criterion," J Mach Learn Res 11, pp. 3371-3408; Larochelle et al., 2009, "Exploring strategies for training deep neural networks," J Mach Learn Res 10, pp. 1-40; and Hassoun, 1995, Fundamentals of Artificial Neural Networks, Massachusetts Institute of Technology, each of which is hereby incorporated by reference. Additional example neural networks suitable for use as models are disclosed in Duda et al., 2001, Pattern Classification, Second Edition, John Wiley & Sons, Inc., New York; and Hastie et al., 2001, The Elements of Statistical Learning, Springer-Verlag, New York, each of which is hereby incorporated by reference in its entirety. Additional example neural networks suitable for use as models in the present disclosure are described in Draghici, 2003, Data Analysis Tools for DNA Microarrays, Chapman & Hall/CRC; and Mount, 2001, Bioinformatics: sequence and genome analysis, Cold Spring Harbor Laboratory Press, Cold Spring Harbor, New York, each of which is hereby incorporated by reference in its entirety.

*Support vector machines.* In some embodiments, the model is a support vector machine (SVM). SVMs suitable for use as models are described in, for example, Cristianini and Shawe-Taylor, 2000, "An Introduction to Support Vector Machines," Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914, each of which is hereby incorporated by reference in its entirety. When used for classification, SVMs separate a given set of binary labeled data with a hyper-plane that is maximally distant from the labeled data. For certain cases in which no linear separation is possible, SVMs work in combination with the technique of 'kernels', that automatically realizes a non-linear mapping to a feature space. The hyper-plane found by the SVM in feature space corresponds, in some instances, to a non-linear decision boundary in the input space. In some embodiments, the plurality of parameters (*e.g*., weights) associated with the SVM define the hyper-plane. In some embodiments, the hyper-plane is defined by at least 10, at least 20, at least 50, or at least 100 parameters and the SVM model requires a computer to calculate because it cannot be mentally solved.

*Naive Bayes models.* In some embodiments, the model is a Naive Bayes model. Naive Bayes models suitable for use as models are disclosed, for example, in Ng et al., 2002, "On discriminative vs. generative classifiers: A comparison of logistic regression and naive Bayes," Advances in Neural Information Processing Systems, 14, which is hereby incorporated by reference. A Naive Bayes model is any model in a family of "probabilistic models" based on applying Bayes' theorem with strong (naive) independence assumptions between the features. In some embodiments, they are coupled with Kernel density estimation. *See,* for example, Hastie et al., 2001, The elements of statistical learning: data mining, inference, and prediction, eds. Tibshirani and Friedman, Springer, New York, which is hereby incorporated by reference.

*Nearest neighbor models.* In some embodiments, a model is a nearest neighbor model. In some implementations, nearest neighbor models are memory-based and include no model to be fit. For nearest neighbors, given a query point x₀ (a test subject), the k training points x_{(*r*)}, r, ... , k (here the training subjects) closest in distance to x₀ are identified and then the point x₀ is classified using the k nearest neighbors. In some embodiments, Euclidean distance in feature space is used to determine distance as *d*_{(*i*)} = ∥*x*_{(*i*)} - *x*_{(*o*)}∥. Typically, when the nearest neighbor model is used, the abundance data used to compute the linear discriminant is standardized to have mean zero and variance 1. In some embodiments, the nearest neighbor rule is refined to address issues of unequal class priors, differential misclassification costs, and feature selection. Many of these refinements involve some form of weighted voting for the neighbors. For more information on nearest neighbor analysis, *see* Duda, Pattern Classification, Second Edition, 2001, John Wiley & Sons, Inc; and Hastie, 2001, The Elements of Statistical Learning, Springer, New York, each of which is hereby incorporated by reference.

A k-nearest neighbor model is a non-parametric machine learning method in which the input consists of the k closest training examples in feature space. The output is a class membership. An object is classified by a plurality vote of its neighbors, with the object being assigned to the class most common among its k nearest neighbors (k is a positive integer, typically small). If k = 1, then the object is simply assigned to the class of that single nearest neighbor. *See,* Duda et al., 2001, Pattern Classification, Second Edition, John Wiley & Sons, which is hereby incorporated by reference. In some embodiments, the number of distance calculations needed to solve the k-nearest neighbor model is such that a computer is used to solve the model for a given input because it cannot be mentally performed.

*Random forest, decision tree, and boosted tree models.* In some embodiments, the model is a decision tree. Decision trees suitable for use as models are described generally by Duda, 2001, Pattern Classification, John Wiley & Sons, Inc., New York, pp. 395-396, which is hereby incorporated by reference. Tree-based methods partition the feature space into a set of rectangles, and then fit a model (like a constant) in each one. In some embodiments, the decision tree is random forest regression. For example, one specific model is a classification and regression tree (CART). Other specific decision tree models include, but are not limited to, ID3, C4.5, MART, and Random Forests. CART, ID3, and C4.5 are described in Duda, 2001, Pattern Classification, John Wiley & Sons, Inc., New York, pp. 396-408 and pp. 411-412, which is hereby incorporated by reference. CART, MART, and C4.5 are described in Hastie et al., 2001, The Elements of Statistical Learning, Springer-Verlag, New York, Chapter 9, which is hereby incorporated by reference in its entirety. Random Forests are described in Breiman, 1999, "Random Forests--Random Features," Technical Report 567, Statistics Department, U.C. Berkeley, September 1999, which is hereby incorporated by reference in its entirety. In some embodiments, the decision tree model includes at least 10, at least 20, at least 50, or at least 100 parameters (*e.g*., weights and/or decisions) and requires a computer to calculate because it cannot be mentally solved.

*Regression.* In some embodiments, the model uses any type of regression. For example, in some embodiments, the regression is logistic regression. In some embodiments, the regression is logistic regression with lasso, L2 or elastic net regularization. In some embodiments, those extracted features that have a corresponding regression coefficient that fails to satisfy a threshold value are pruned (removed from) consideration. In some embodiments, a generalization of the logistic regression model that handles multi category responses is used as the model. Logistic regression is disclosed in Agresti, An Introduction to Categorical Data Analysis, 1996, Chapter 5, pp. 103-144, John Wiley & Son, New York, which is hereby incorporated by reference. In some embodiments, the model makes use of a regression model disclosed in Hastie et al., 2001, The Elements of Statistical Learning, Springer-Verlag, New York. In some embodiments, the logistic regression model includes at least 10, at least 20, at least 50, at least 100, or at least 1000 parameters (*e.g*., weights) and requires a computer to calculate because it cannot be mentally solved.

*Linear discriminant analysis.* In some embodiments, the model is linear discriminant analysis (LDA), normal discriminant analysis (NDA), or a discriminant function. These are a generalization of Fisher's linear discriminant, a method used in statistics, pattern recognition, and machine learning to find a linear combination of features that characterizes or separates two or more classes of objects or events. In some embodiments, the resulting combination is used as the model (linear model) in some embodiments of the present disclosure.

*Ensembles of models and boosting.* In some embodiments, an ensemble (two or more) of models is used. In some embodiments, a boosting technique such as AdaBoost is used in conjunction with many other types of models to improve the performance of the model. In this approach, the output of any of the models disclosed herein, or their equivalents, is combined into a weighted sum that represents the final output of the boosted model. In some embodiments, the plurality of outputs from the models is combined using any measure of central tendency known in the art, including but not limited to a mean, median, mode, a weighted mean, weighted median, weighted mode, *etc.* In some embodiments, the plurality of outputs is combined using a voting method. In some embodiments, a respective model in the ensemble of models is weighted or unweighted.

As used herein, the term "parameter" refers to any coefficient or, similarly, any value of an internal or external element (*e.g*., a weight and/or a hyperparameter) in an algorithm, model, regressor, and/or classifier that can affect (*e.g*., modify, tailor, and/or adjust) one or more inputs, outputs, and/or functions in the algorithm, model, regressor and/or classifier. For example, in some embodiments, a parameter refers to any coefficient, weight, and/or hyperparameter that can be used to control, modify, tailor, and/or adjust the behavior, learning, and/or performance of an algorithm, model, regressor, and/or classifier. In some instances, a parameter is used to increase or decrease the influence of an input (*e.g*., a feature) to an algorithm, model, regressor, and/or classifier. As a nonlimiting example, in some embodiments, a parameter is used to increase or decrease the influence of a node (*e.g*., of a neural network), where the node includes one or more activation functions. Assignment of parameters to specific inputs, outputs, and/or functions is not limited to any one paradigm for a given algorithm, model, regressor, and/or classifier but can be used in any suitable algorithm, model, regressor, and/or classifier architecture for a desired performance. In some embodiments, a parameter has a fixed value. In some embodiments, a value of a parameter is manually and/or automatically adjustable. In some embodiments, a value of a parameter is modified by a validation and/or training process for an algorithm, model, regressor, and/or classifier (*e.g*., by error minimization and/or backpropagation methods). In some embodiments, an algorithm, model, regressor, and/or classifier of the present disclosure includes a plurality of parameters. In some embodiments, the plurality of parameters is n parameters, where: n ≥ 2; n ≥ 5; n ≥ 10; n ≥ 25; n ≥ 40; n ≥ 50; n ≥ 75; n ≥ 100; n ≥ 125; n ≥ 150; n ≥ 200; n ≥ 225; n ≥ 250; n ≥ 350; n ≥ 500; n ≥ 600; n ≥ 750; n ≥ 1,000; n ≥ 2,000; n ≥ 4,000; n ≥ 5,000; n ≥ 7,500; n ≥ 10,000; n ≥ 20,000; n ≥ 40,000; n ≥ 75,000; n ≥ 100,000; n ≥ 200,000; n ≥ 500,000, n ≥ 1 x 10⁶, n ≥ 5 x 10⁶, or n ≥ 1 × 10⁷. As such, the algorithms, models, regressors, and/or classifiers of the present disclosure cannot be mentally performed. In some embodiments n is between 10,000 and 1 × 10⁷, between 100,000 and 5 x 10⁶, or between 500,000 and 1 × 10⁶. In some embodiments, the algorithms, models, regressors, and/or classifier of the present disclosure operate in a k-dimensional space, where k is a positive integer of 5 or greater (*e.g.,* 5, 6, 7, 8, 9, 10, *etc.*)*.* As such, the algorithms, models, regressors, and/or classifiers of the present disclosure cannot be mentally performed.

As used herein, the term "untrained model" refers to a model that has not been trained on a target dataset. In some embodiments, "training a model" (*e.g*., "training a neural network") refers to the process of training an untrained or partially trained model (*e.g*., "an untrained or partially trained neural network"). Moreover, it will be appreciated that the term "untrained model" does not exclude the possibility that transfer learning techniques are used in such training of the untrained or partially trained model. For instance, Fernandes et al., 2017, "Transfer Learning with Partial Observability Applied to Cervical Cancer Screening," Pattern Recognition and Image Analysis: 8th Iberian Conference Proceedings, 243-250, which is hereby incorporated by reference, provides non-limiting examples of such transfer learning. In instances where transfer learning is used, the untrained model described above is provided with additional data over and beyond that of the primary training dataset. Typically, this additional data is in the form of parameters (*e.g*., coefficients, weights, and/or hyperparameters) that were learned from another, auxiliary training dataset. Moreover, while a description of a single auxiliary training dataset has been disclosed, it will be appreciated that there is no limit on the number of auxiliary training datasets that can be used to complement the primary training dataset in training the untrained model in the present disclosure. For instance, in some embodiments, two or more auxiliary training datasets, three or more auxiliary training datasets, four or more auxiliary training datasets or five or more auxiliary training datasets are used to complement the primary training dataset through transfer learning, where each such auxiliary dataset is different than the primary training dataset. Any manner of transfer learning is used, in some such embodiments. For instance, consider the case where there is a first auxiliary training dataset and a second auxiliary training dataset in addition to the primary training dataset. In such a case, the parameters learned from the first auxiliary training dataset (by application of a first model to the first auxiliary training dataset) are applied to the second auxiliary training dataset using transfer learning techniques (*e.g*., a second model that is the same or different from the first model), which in turn results in a trained intermediate model whose parameters are then applied to the primary training dataset and this, in conjunction with the primary training dataset itself, is applied to the untrained model. Alternatively, in another example embodiment, a first set of parameters learned from the first auxiliary training dataset (by application of a first model to the first auxiliary training dataset) and a second set of parameters learned from the second auxiliary training dataset (by application of a second model that is the same or different from the first model to the second auxiliary training dataset) are each individually applied to a separate instance of the primary training dataset (*e.g*., by separate independent matrix multiplications) and both such applications of the parameters to separate instances of the primary training dataset in conjunction with the primary training dataset itself (or some reduced form of the primary training dataset such as principal components or regression coefficients learned from the primary training set) are then applied to the untrained model in order to train the untrained model.

### I. Exemplary System Embodiments

Now that an overview of some aspects of the present disclosure and some definitions used in the present disclosure have been provided, details of an exemplary system are described in conjunction with Figure 1.

Figure 1 illustrates a computer system 100 for screening for improved enhancer activity. In the examples of the present disclosure, activity for a sequence is a log of RNA counts to DNA counts for the given sequence in a sample. However, those of skill in the art will appreciate that other measures of activity are possible and all such measures are within the scope of the present disclosure. For instance, in some embodiments. The activity for a sequence is a ratio of RNA counts to DNA counts for the given sequence. In still other embodiments, the activity for a sequence is RNA counts for the given sequence. In still other embodiments, the activity for a sequence is log RNA counts for the given sequence. In such embodiments, each count of RNA or DNA is for a unique molecule present in the measured sample. For instance, in some embodiments unique molecular identifiers (UMIs) are used during sequencing to ensure that counts are for unique molecules in the biological sample.

In typical embodiments, computer system 100 comprises one or more computers. For purposes of illustration in Figure 1, the computer system 100 is represented as a single computer that includes all of the functionality of the disclosed computer system 100. However, the present disclosure is not so limited. The functionality of the computer system 100 may be spread across any number of networked computers and/or reside on each of several networked computers and/or virtual machines. One of skill in the art will appreciate that a wide array of different computer topologies is possible for the computer system 100 and all such topologies are within the scope of the present disclosure.

Turning to Figure 1 with the foregoing in mind, the computer system 100 comprises one or more processing units (CPUs) 52, a network or other communications interface 54, a user interface 56 (*e.g*., including an optional display 58 and optional input 60 (*e.g*. keyboard or other form of input device)), a memory 92 *(*e*.g.*, random access memory, persistent memory, or combination thereof), and one or more communication busses 94 for interconnecting the aforementioned components. To the extent that components of memory 92 are not persistent, data in memory 92 can be seamlessly shared with non-volatile memory (not shown) or portions of memory 92 that are non-volatile / persistent using known computing techniques such as caching. Memory 92 can include mass storage that is remotely located with respect to the central processing unit(s) 52. In other words, some data stored in memory 92 may in fact be hosted on computers that are external to computer system 100 but that can be electronically accessed by the computer system 100 over an Internet, intranet, or other form of network or electronic cable using network interface 54. In some embodiments, the computer system 100 makes use of models that are run from the memory associated with one or more graphical processing units in order to improve the speed and performance of the system. In some alternative embodiments, the computer system 100 makes use of models that are run from memory 92 rather than memory associated with a graphical processing unit.

The memory 92 of the computer system 100 stores:
- an optional operating system 102 that includes procedures for handling various basic system services;
- an analysis module 104 for screening for improved enhancer activity;
- a first training dataset 106 that comprises:
   ∘ a first plurality of training enhancer sequences (*e.g*., 108-1, 108-2, ..., 108-N; where N is a positive integer)
   ∘ for each respective training enhancer sequence 108 in the first plurality of training enhancer sequences, for each *in vivo* state in one or more *in vivo* states, a corresponding measurement (*e.g*., amount of transcriptional activity) of the respective training enhancer sequence 108 in the *in vivo* state (*e.g.*, 110-1-1, ..., 110-1-M, 110-2-1, ..., 110-2-M; 110-N-1, ..., 110-N-M, where M is a positive integer), and
   ∘ a measured activity (*e.g*., 112-1, ..., 112-M) for each respective training enhancer sequence 108 in the first plurality of training enhancer sequences where measurements 110 are available for more than one *in vivo* state;
- a model 114 comprising a plurality of parameters (*e.g*., 116-1, ..., 116-K, where K is a positive integer);
- for each respective training enhancer sequence 108 in the first plurality of training enhancer sequences, a corresponding predicted amount of activity of the respective training enhancer sequence in each of the one or more *in vivo* states *(e.g.,* 118-1, ..., 118-N); and
- a plurality of test enhancer sequences (*e.g*., 120-1, ..., 120-Q, where Q is a positive integer).

In some embodiments, one or more of the above identified data elements or modules of the computer system 100 are stored in one or more of the previously mentioned memory devices, and correspond to a set of instructions for performing a function described above. The above identified data, modules or programs (*e.g*., sets of instructions) need not be implemented as separate software programs, procedures or modules, and thus various subsets of these modules may be combined or otherwise re-arranged in various implementations. In some implementations, the memory 92 optionally stores a subset of the modules and data structures identified above. Furthermore, in some embodiments the memory 92 stores additional modules and data structures not described above.

### II. Methods for screening for improved enhancer activity

Referring to block 200 of Figure 2A a method of screening for improved enhancer activity is provided.

### Obtaining a training dataset.

Referring to block 202, a first training dataset 102 is obtained in electronic form. The first training dataset comprises a first plurality of training enhancer sequences and, for each respective training enhancer sequence 108 in the first plurality of training enhancer sequences, a corresponding measured amount of activity 110 of the respective training enhancer sequence in each of one or more *in vivo* states.

In some embodiments, each training enhancer sequence in the first plurality of training enhancer sequences is a tandem n-mer repeat sequence. In some embodiments, at least 10 percent, at least 20 percent, at least 30 percent, at least 40 percent, at least 50 percent at least 60 percent, at least 70 percent, at least 80 percent, or at least 90 percent of the training enhancer sequence in the first plurality of training enhancer sequences are tandem n-mer repeat sequences, while the remaining training enhancer sequences in the first plurality of training enhancer sequences are not tandem n-mer repeat sequences.

Figure 4 illustrates training enhancer sequences that are tandem n-mer repeat sequences. In Figure 4 the 5' (402) and 3' (404) strands of an enhancer are shown in the middle of the figure. In this illustration, the enhancer 5' (402) and 3' (404) strands depict that of a tandem n-mer repeat sequence. Moreover, strands 402 and 404 are examples of an "enhanceosome" 2004 of Figure 20 comprising a rich grammar of highly cooperative binding sites 2006 (*e.g*., transcription factor binding sites). Without being limited to any particular theory, enhanceosomes are thought to process the information of bound transcription factors within the overall enhancer sequence and give unitary outputs. The "enhanceosome'" model proposes that the DNA sequences of the individual binding sites within the enhancer operate as a scaffold to form a unified nucleoprotein complex. The enhanceosome features a high degree of cooperativity between enhancer-bound proteins, such that alterations in individual binding sites within the enhancer can have large effects on enhancer output. The function of the enhanceosome is thus more than the sum of individual factor contributions. Rather it emerges from a network of interactions. *See* Arnosti and Kulkarni, 2005, "Transcriptional Enhancers: Intelligent Enhanceosomes or Flexible Billboards?," Journal of Cellular Biochemistry 94, pp. 890-898, which is hereby incorporated by reference. This high degree of cooperativity between the binding sites 2006 introduces a nonlinearity between the overall sequence of the enhancer and the ability of the enhancer to bind transcription factors, and thus between the sequence of the enhancer and the expression of a gene that is operably connected to the transcription factor.

The tandem n-mer sampling, illustrated in Figure 4, of naturally occurring transcription factor binding sites, which presumably is best described as an "enhanceosome", produces training enhancer sequences 108 that are more simplistic and more adhere to the enhancer billboard model 2008 illustrated in Figure 20. The enhancer billboard model suggests that binding sites 2006 are flexibly disposed, because the proteins bound to the enhancer do not operate as a single unit, with a strictly defined overall architecture, but rather as an ensemble of separately acting factors or small groups of factors. This lower degree of cooperativity between the binding sites 2006 in the billboard model facilitates training a model to identify binding sites 2006 within an enhancer sequence and to predict the expression of a gene that is operably connected to such enhancers. Once a model has been trained to discover particular binding sites under the billboard model, in accordance with the present disclosure, the model can be further trained to predict the expression of a genes under the control of enhancers that follow the enhanceosome model.

Regardless of which model best describes the training enhancer sequences, the sampling of Figure 4 emphasizes the discovery and effect of individual binding sites within tandem repeat enhancer sequences. To illustrate construction of the first plurality of training enhancer sequences 108, consider training enhancer sequences 108 of Figure 4, where N is 10. Thus, training enhancer sequence 108-1 of Figure 4 will include residues 1-10 of sequence 404, where position numbering begins from the 5' end of sequence 404. Then, rather than taking any further sequence from sequence 404, the remainder of training enhancer sequence 108-1 of Figure 4 is a repeat of residues 1-10 from sequence 404 over and over, in tandem, until the desired length of training enhancer sequences 108-1 is reached, as illustrated in Figure 4. Thus, training enhancer sequence 108-2 of Figure 4 will include residues 2-11 of sequence 404, where position numbering begins from the 5' end of sequence 404. Then, rather than taking any further sequence from sequence 404, the remainder of training enhancer sequence 108-2 of Figure 4 is a repeat of residues 2-11 from sequence 404 over and over until the desired length of training enhancer sequences 108-2 is fully populated, as illustrated in Figure 4. The process for sampling sequence 404 to build training enhancer sequences can continue until all possible n-mers within sequence 404 have been sampled. For instance, enhancer sequence 108-8 of Figure 4 will include residues 8-17 of sequence 404, where position numbering begins from the 5' end of sequence 404. Then, rather than taking any further sequence from sequence 404, the remainder of training enhancer sequence 108-8 of Figure 4 is a tandem repeat of residues 8-17, where the initial residues and each repeat of residues 8-18 is denoted by element 406 in Figure 4, from sequence 404 over and over until the desired length of training enhancer sequences 108-8 is reached, as illustrated in Figure 4. In some embodiments, tandem n-mer training enhancer sequence comprises or consists of 2, 3, 4, 5, 6, 7, 8, 9, 10, 11, 12, 13, 14, 15, 16, 17, 18, 19, or 20 or more repeats (*e.g*., repeats 108). In this way, binding elements 2006 within the sequence 404 that are of length N are ultimately isolated and captured in one or more of the training enhancer sequences 108-8 that happened to exactly or almost exactly sample the position of such binding elements 2006 from the sequence 404. Such training enhancer sequences, with isolated and repeated binding elements 2006, is used to train a model in accordance with the present disclosure.

Referring to block 204, in some embodiments, the first plurality of training enhancer sequences comprises 10,000 or more training enhancer sequences, 20,000 or more training enhancer sequences, 30,000 or more training enhancer sequences, 40,000 or more training enhancer sequences, 50,000 or more training enhancer sequences, 60,000 or more training enhancer sequences, 70,000 or more training enhancer sequences, 80,000 or more training enhancer sequences, 90,000 or more training enhancer sequences, or 100,000 or more training enhancer sequences.

Referring to block 206, in some embodiments, each training enhancer sequence 108 in the first plurality of training enhancer sequences comprises 5 to 20 contiguous repeats of a different n-mer, where n is a positive integer between 4 and 20.

Referring to block 208, in some embodiments, each training enhancer sequence 108 in the first plurality of training enhancer sequences comprises 5 to 20 contiguous repeats of a different 10-mer. Example 1 provides justification for the use of a 10-mer for the tandem repeat training enhancer sequences. In some embodiments, each training enhancer sequence 108 in the first plurality of training enhancer sequences comprises 5 to 20 contiguous repeats of a different 7-mer, 8-mer, 9-mer, 10-mer, 11-mer, 12-mer, 13-mer, 14-mer, or 15-mer.

Referring to block 210, in some embodiments, each training enhancer sequence 108 in the first plurality of training enhancer sequences consists of between 15 residues and 200 residues. In some embodiments, each training enhancer sequence 108 in the first plurality of training enhancer sequences consists of between 30 residues and 300 residues. In some embodiments, each training enhancer sequence 108 in the first plurality of training enhancer sequences consists of between 40 residues and 500 residues. In some embodiments, each training enhancer sequence 108 in the first plurality of training enhancer sequences consists of between 50 residues and 500 residues, 70 residues and 500 residues, 90 residues and 500 residues, 110 residues and 500 residues, or 130 residues and 500 residues. In some embodiments, each training enhancer sequence 108 in the first plurality of training enhancer sequences consists of between 100 residues and 1000 residues, 120 residues and 1000 residues, 130 residues and 1000 residues, 140 residues and 1000 residues, or 150 residues and 1000 residues.

Referring to block 212, in some embodiments, each respective training enhancer sequence 108 in the first plurality of training enhancer sequences is operably connected to a common promoter and a common payload. Each respective *in vivo* state in the one or more *in vivo* states is an amount of expression of the common payload in a corresponding tissue. Figure 3 illustrates a training enhancer sequence 108 operably connected to a common promoter 310 and a common payload 312. The term "common" is used to mean that each respective training enhancer sequence 108 in the first plurality of training enhancer sequences is operably connected to a promoter 310 that has the same sequence and a payload 312 that has the same sequence. In some embodiments, the payload 312 encodes a marker gene such as the green fluorescent protein. As used here, the term "operably connected" means that the enhancer 108 is upstream of the promoter 310 and that the enhancer 108 and promoter 310 are positioned upstream of the 5' side of the payload 312 so that a transcription factor binds to the promoter 310 and translates the payload 312.

Referring to block 214, in some embodiments, the tissue represented by an *in vivo* state in the one or more *in vivo* states is brain tissue. In some embodiments, the tissue represented by an *in vivo* state in the one or more *in vivo* states is brain tissue. Referring to block 216, in some embodiments, the tissue represented by an *in vivo* state in the one or more *in vivo* states is blood, brain, colon, diaphragm, gastrocnemius muscle, heart, inguinal adipose, kidney, lung, lymph node, skin, small intestine, spinal cord, spleen, trachea, liver, stomach, large intestine, pancreas, gallbladder, bladder, eyes, thymus, or adrenal gland. Figures 18A and 18B illustrate one exemplary mechanism by which corresponding measured amounts of activity of respective training enhancer sequences in each of one or more *in vivo* states, where the one or more *in vivo* states are particular tissues (*e.g*., brain tissue, liver tissues, *etc*.), can be obtained in accordance with the present disclosure. In step 1802, an adeno-associated virus 9 (AAV9), gene delivery system is charged with a plurality of training enhancer sequences having the form illustrated in Figure 3 and is injected into mice. Each payload 312 is barcoded with a barcode that uniquely corresponds to a training enhancer sequence in the plurality of training enhancer sequences. In step 1804, 4 weeks after injection of the training enhancer sequences, the brain and liver are harvested from the mice. In accordance with step 1806 RNA is isolated from the brain and liver tissue using known molecular biology techniques including, as a nonlimiting example, the use of Trizol. This RNA includes the barcodes uses to uniquely identify each of the training enhancer sequences originally injected into the mice in step 1802. In accordance with step 1808 of Figure 18B, the isolated RNA is sequenced using, for example, a next generation sequencer and next generation sequencing software such as, for example Chromium (10X, Pleasanton, California). The barcoded sequence reads are processed to counts the number of barcodes attributable to respective training enhancer sequences in the plurality of training enhancer sequences. In this way, the *in vivo* transcriptional activity of each enhancer in the plurality of training enhancer sequences in both the brain tissue and the liver tissue is determined. In accordance with step 1810 of Figure 18B, the relative transcriptional activity of each training enhancer sequence in the brain tissue and the liver tissue allows for the determination of the specificity of each training enhancer sequence to brain tissue relative to liver tissue and vice versa. Such information can also be used to train models for improved transcriptional activity in brain tissue, or separately, improved transcriptional activity in liver tissue.

Referring to block 218, in some embodiments, each respective training enhancer sequence 108 in the first plurality of training enhancer sequences is operably connected to a common promoter and a common payload under a particular transcription factor. Each respective *in vivo* state in the one or more *in vivo* states is an amount of expression of the common payload in a corresponding cell line in a corresponding one or more different cell lines. Each such cell line in the one or more cell lines originates from a particular tissue in a corresponding one or more tissues.

Referring to block 220, in some embodiments, one such cell line is mouse primary neurons. HepG2, H4, or K562. In some embodiments, the transcription factor is FOSB::JUNB, FOSL2::JUNB, FOSL1::JUNB, FOS::JUN; FOSL1::JUN; BATF3, BNC2; ZBTB12; HSF4; HSF1; HSF2; REL; RELA; TEAD3; TEAD1; TP53; TP63; TP73; SMAD5; SMAD2; ZNF416; GRHL2; TFCP2; MLX; ARNT::HIF1A; or HIF1A.

Referring to block 222, in some embodiments, the one or more *in vivo* states consists of a first *in vivo* state and a second *in vivo* state, and the corresponding measured amount of activity (*e.g*., transcriptional activity) of the respective training enhancer sequence 108 in each of the one or more *in vivo* states comprises a log fold change in (i) an activity of the respective training enhancer sequence measured in the first *in vivo* state and (ii) an activity of the respective training enhancer sequence measured in the second *in vivo* state.

Referring to block 224, in some embodiments, the one or more *in vivo* states consists of a first *in vivo* state and a second *in vivo* state, and the corresponding measured amount of activity of the respective training enhancer sequence in each of the one or more *in vivo* states comprises a binary indication of measured selectivity between the first *in vivo* state and the second *in vivo* state. Thus, if more activity is observed in the first *in vivo* state the binary indication of measured selectivity has a first value (*e.g*., "1") and if more activity is observed in the second *in vivo* state, the binary indication of measured selectivity has a second value (*e.g*., "0").

Referring to block 226, in some embodiments, the corresponding measured amount of activity of the respective training enhancer sequence in each of the one or more *in vivo* states comprises a corresponding measured abundance of the respective training enhancer sequence in each of the *in vivo* states. In some such embodiments, the corresponding measured abundance of the respective training enhancer sequence is determined by measuring transcription of a barcoded payload associated with the respective training enhancer sequence in each of the one or more *in vivo* states in accordance with the construct illustrated in Figure 3.

Referring to block 228, in some embodiments, the corresponding measured abundance of the respective training enhancer sequence 108 in an *in vivo* state in the one or more *in vivo* states is a corresponding averaged or summed count of a number of barcoded instances of the respective training enhancer sequence found in a corresponding plurality of sequence reads of RNA harvested from each replica in a plurality of replicas of a plurality of cells, representative of the *in vivo* state exposed to the respective training enhancer sequence. In some embodiments, these barcoded instances of the respective training enhancer sequence are determined by measuring a transcription of a barcoded payload associated with the respective training enhancer sequence in the *in vivo* state in accordance with the construct illustrated in Figure 3.

Figure 6 illustrates. In the upper-left corner of Figure 6 is a partial sequence of a training enhancer sequence 108 consisting of the tandem 10-mer repeat sequence ATGCCAGGAA (Seq. ID NO: 6). As illustrated in panel 602 of Figure 6, the illustrated training enhancer sequence 108 is just one of 50,000 training enhancer sequences that each have their own unique tandem 10-mer repeat sequence. Each of these training enhancer sequences is set up in a construct illustrated in Figure 3 (*e.g*., with a core promoter 310 and a payload 312) and exposed to cells, in replicate form, representative of an *in vivo* state. In some embodiments, such exposure is by transfection. In some embodiments, the construct illustrated in Figure 3 is inserted into an adeno-associated virus (AAV), and AAV vector-mediated gene delivery is used to perform the exposure. In the example of Figure 6 there are two *in vivo* states. The first of these *in vivo* states consists of H4 cells (representative of brain tissue, and therefore the *in vivo* state "brain"). The second of these and *in vivo* states is HepG2 cells (representative of liver tissue, and therefore the *in vivo* state "liver"). Moreover, the payload 312 of each of the training enhancer sequences is barcoded. For instance, the payload 312 for the training enhancer sequence 108 is assigned a barcode. RNA is harvested from the cells, and sequence reads generated from this harvested RNA will include the barcodes of the respective source training enhancer sequences. Thus, it is possible to determine the abundance of the payload corresponding to each training enhancer sequence in each cell line from these barcodes and hence the transcriptional activity of the corresponding enhancer. In fact, in some embodiments, as illustrated in Figure 6 and in accordance with block 228, the plurality of training enhancer sequences 108 are delivered by AAV into a number of replicas of each of the one or more *in vivo* states and the barcodes individually assigned to the training enhancer sequences are used to obtain a count of the payload of each training enhancer sequence in each of the replicas for each of the one or more *in vivo.* For example, a first *in vivo* state may have 3 replicas of a first cell line and a second *in vivo* state may have 3 replicas of a second cell line. Then, in some embodiments, the individual counts for a given training enhancer sequence in each of the replicas for a particular *in vivo* state is averaged to get a count of the abundance of the given training enhancer sequence (*e.g*., the abundance of the payload of the given training enhancer sequence) in that particular *in vivo* state. For instance, if the counts for the given training enhancer sequence in replicas 1, 2, and 3 illustrated in Figure 6 is "120", "210", and "107" (for a particular *in vivo* state) respectively, then the count for the given training enhancer sequence (*e.g*., the abundance of the payload of the given training enhancer sequence) is taken to be the average of these numbers, or 146 in such embodiments. In alternative embodiments, the individual counts for a given training enhancer sequence in each of the replicas (for a particular *in vivo* state) is summed to get a count of the abundance of the given training enhancer sequence. For instance, again if the counts for the given training enhancer sequence in replicas 1, 2, and 3 (for a particular *in vivo* state) illustrated in Figure 6 is "120", "210", and "107" respectively, then the count for the given training enhancer sequence (*e.g*., the abundance of the payload of the given training enhancer sequence) is taken to be the sum of these numbers, or 437 in such embodiments.

Referring to block 230, in some embodiments, the corresponding averaged or summed count for the given training enhancer sequence is log normalized by an amount of the respective training enhancer sequence exposed to (*e.g*., by transfection, by adeno-associated virus (AAV) vector-mediated gene delivery, *etc.*) each replica of the plurality of cells (for a particular *in vivo* state). This is further illustrated in Figure 6. As discussed above in conjunction with Figure 6, counts for RNA resulting from exposure of barcoded training enhancer sequences have been described. To normalize for DNA, in some embodiments, the number of training enhancer sequences (which are in DNA form) exposed to a particular *in vivo* state in the one or more *in vivo* states, for each replica of the particular *in vivo* state, is traced and is used to normalize the measured counts of the payload for each training enhancer sequence from the sequence reads (which are derived from instances of the payload barcoded with each training enhancer sequence in RNA form). In some embodiments, the normalization is the log of the ratio of (i) the measured abundance of the training enhancer sequence (by virtue of the abundance of the payload with the barcode of the training enhancer sequence) in a particular *in vivo* state in the one or more *in vivo* states and (ii) the number of training enhancer sequences (which are in DNA form) exposed to the particular *in vivo* state (*e.g*., by transfection, by adeno-associated virus (AAV) vector-mediated gene delivery, *etc.*)*.*

Referring to block 232, in some embodiments, each respective training enhancer sequence 108 in the plurality of respective training enhancer sequences is uniquely assigned a corresponding plurality of barcodes comprising 5 or more, 10 or more, 20 or more, 50 or more, 75 or more, or 100 or more barcodes. Each replica of the plurality of cells representative of the *in vivo* state exposed to (*e.g.*, by transfection, by adeno-associated virus (AAV) vector-mediated gene delivery, *etc.*) the respective training enhancer sequence is, for each respective barcode uniquely assigned to the respective training enhancer sequence, one or more copies of the respective training enhancer sequence tagged with the respective barcode. Figure 6 illustrates. Rather than assigning a training enhancer sequence a single barcode, in some embodiments each training enhancer sequence is assigned several barcodes. Each construct in accordance with Figure 3 for a respective training enhancer sequence will use one of the several barcodes assigned to the respective training enhancer sequence. However, there will be several copies of constructs containing the respective training enhancer sequence for each barcode in the several barcodes assigned to the respective training enhancer sequence that are introduced into each replica of each *in vivo* state. For example, consider the case where 10 barcodes are assigned to a given training enhancer sequence. In some embodiments, a plurality of constructs of the given training enhancer sequence in accordance with Figure 3 having the first of the 10 barcodes is introduced into each replica of a given *in vivo* state, a plurality of constructs of the given training enhancer sequence in accordance with Figure 3 having the second of the 10 barcodes will be introduced into each replica of a given *in vivo* state, a plurality of constructs of the given training enhancer sequence in accordance with Figure 3 having the third of the 10 barcodes will be introduced into each replica of a given *in vivo* state and so forth.

Referring to block 234, in some embodiments, each barcode in the corresponding plurality of barcodes encodes a unique predetermined value selected from the set {1, ..., 1024}, {1, ..., 4096}, {1, ..., 16384}, {1, ..., 65536}, {1, ..., 262144}, {1, ..., 1048576}, {1, ..., 4194304}, {1, ..., 16777216}, {1, ..., 67108864}, or {1, ..., 1 × 10¹²}.

Referring to block 236, in some embodiments, the corresponding plurality of sequence reads of RNA harvested from each replica in the plurality of replicas of a plurality of cells comprises at least 1000, at least 2000, at least 3000, at least 4000, at least 5000, at least 6000, at least 7000, at least 8000, at least 9000, at least 10,000, at least 50,000, at least 100,000, at least 500,000, at least 1 million, at least 2 million, at least 3 million, at least 4 million, at least 5 million, at least 6 million, at least 7 million, at least 8 million, at least 9 million, or more sequence reads.

For instance, consider the case in which the barcode is represented by a set of five nucleotide positions. In this instance, each nucleotide position contributes four possibilities (A, T, C or G), giving rise, when all five positions are considered, to 4 x 4 x 4 x 4 x 4 = 1024 possibilities. As such, the five nucleotide positions form the basis of the set {1,..., 1024}. Thus, when the barcode sequence is a 5-mer, barcode encodes a unique predetermined value selected from the set {1,..., 1024}. Likewise, when the barcode sequence is represented by a set of six nucleotide positions, the six nucleotide positions collectively contribute 4 x 4 x 4 x 4 x 4 x 4 = 4096 possibilities. As such, the six nucleotide positions form the basis of the set {1,..., 4096}. In other words, when the barcode sequence is a 6-mer, the barcode encodes a unique predetermined value selected from the set {1,..., 4096}.

In some embodiments, the barcode is a contiguous set of oligonucleotides. In one such exemplary embodiment, the contiguous set of oligonucleotides is an n-mer, where n is an integer selected from the set {4, ..., 20}. In other words, in some embodiments, the barcode is a contiguous set of nucleotide positions (e.g., 4 contiguous nucleotide positions, 5 contiguous nucleotide positions, 6 contiguous nucleotide positions, 7 contiguous nucleotide positions, 8 contiguous nucleotide positions, 9 contiguous nucleotide positions, 10 contiguous nucleotide positions, 11 contiguous nucleotide positions, 12 contiguous nucleotide positions, 13 contiguous nucleotide positions, 14 contiguous nucleotide positions, 15 contiguous nucleotide positions, 16 contiguous nucleotide positions, 17 contiguous nucleotide positions, 18 contiguous nucleotide positions, 19 contiguous nucleotide positions, or 20 contiguous nucleotide positions).

Referring to block 238, in some embodiments, the corresponding plurality of replicas of the plurality of cells comprises 2, 3, 4, 5, 6, 7, 8, 9, or 10 replicas.

Referring to block 240, in some embodiments, the one or more *in vivo* states consists of a first *in vivo* state and a second *in vivo* state, and the corresponding measured amount of activity of the respective training enhancer sequence in each of the one or more of *in vivo* states comprises a scalar indication of measured selectivity between the first *in vivo* state and the second *in vivo* state. Thus, if 2.5 times more activity is observed in the first *in vivo* state than in the second *in vivo* state, the scalar indication has a value of 2.5. Thus, if 0.5 times less activity is observed in the first *in vivo* state than in the second *in vivo* state, the scalar indication has a value of 0.5.

### Training a model.

Referring to block 242, in some embodiments, a model 114 is trained. The model 114 comprises a plurality of parameters 116. The model is trained by a training procedure. In the training procedure a sequence of each respective training enhancer sequence 108 in the first plurality of training enhancer sequences is inputted into the model 114. The model 114 applies the plurality of parameters 116 to the sequence of each respective training enhancer sequence to generate, as output from the model, for each respective training enhancer sequence in the first plurality of training enhancer sequences, a corresponding predicted amount of activity of the respective training enhancer sequence 108 in a *in vivo* state in the plurality of *in vivo* states. In some embodiments, there is a different model for each in vivo state in the plurality of *in vivo* states. Further in the training procedure the plurality of parameters 116 are refined based on a differential between the corresponding measured amount of activity and the corresponding predicted amount of activity for each respective training enhancer sequence in the first plurality of training enhancer sequences of panel 602 of Figure 6 in the first *in vivo* state.

Figure 7 illustrates. In Figure 7, a training set drawn from the 50,000 10-mer tandem repeat training enhancer sequences 108 of panel 602 of Figure 6 was used to train a model. The model for panel 702 in Figure 7 was trained with the 50,000 10-mer tandem repeat training enhancer sequences 108 of panel 602. Panel 702 provides the model scores obtained for this model for H4 activity and for HepG2 activity. Here, the activity for a sequence is a log of RNA counts to DNA counts for the given sequence. Line 802 of Figure 8 illustrates the performance of the model 114 at predicting the H4 / HepG2 activity of each enhancer sequence in the training set drawn from the 50,000 10-mer tandem repeat training enhancer sequences 108 of panel 602 of Figure 6. In particular, the model 114 has an AUC of 0.962 for the H4/HepG2 activity of the training enhancer sequences.

Next, the trained model 114 was asked to predict the H4/HepG2 activity of each enhancer in a library of approximately 12,000 naturally occurring enhancers delivered to replicas of H4 cells and HepG2 cells using an adeno-associated virus (AAV) vector. The model 114 was not trained on the measurement of the payload expression of these deliveries. All that the model 114 received was the sequences of the naturally occurring enhancers. Figure 7 provides plots 706 and 708 of the model scores obtained for each of the naturally occurring enhancer sequences upon inputting their sequences into model 114. Line 804 of Figure 8 illustrates the performance of the model 114 at predicting the H4/HepG2 expression of each of the enhancer sequences in the naturally occurring enhancers. Model 114 has an AUC of 0.827 for the H4 HepG2 expression of the naturally occurring enhancers even though they were not used in model training.

Figure 15A shows a head to head comparison of the performance of (i) convolutional neural network model that has been trained on the 50,000 10-mer tandem repeat training enhancer sequences 108 of panel 602 of Figure 6 versus (ii) a convolutional neural network model that has been trained on the approximately 12,000 naturally occurring enhancers.

Bar 1502 of Figure 15A shows the performance (Spearman's rho, predicted versus truth) for the agreement of each of the tandem repeat training enhancer sequences between (i) predicted H4 activity of each of the tandem repeat training enhancer sequences from the trained CNN and (ii) their actual H4 activity in replicas of H4 cells. Bar 1504 of Figure 15A shows the performance (Spearman's rho, predicted versus truth) for the agreement of each of the approximately 12,000 naturally occurring enhancers between (i) CNN model predicted H4 activity of each of the approximately 12,000 naturally occurring enhancers and (ii) their actual H4 activity in replicas of H4 cells. Comparison of bar 1504 to bar 1502 shows the performance difference between the CNN model trained for H4 activity on the tandem repeat training enhancer sequences and the CNN model trained on the approximately 12,000 naturally occurring enhancers.

Bar 1506 of Figure 15A shows the performance (Spearman's rho, predicted versus truth) for the agreement of each of the tandem repeat training enhancer sequences between (i) predicted HepG2 activity of each of the tandem repeat training enhancer sequences from the trained CNN and (ii) their actual HepG2 activity in replicas of HepG2 cells. Bar 1508 of Figure 15A shows the performance (Spearman's rho, predicted versus truth) for the agreement of each of the approximately 12,000 naturally occurring enhancers between (i) CNN model predicted HepG2 activity of each of the approximately 12,000 naturally occurring enhancers and (ii) their actual HepG2 activity in replicas of HepG2 cells. Comparison of bar 1506 to bar 1508 shows the performance difference between the CNN model trained for HepG2 activity on the tandem repeat training enhancer sequences and the CNN model trained on the approximately 12,000 naturally occurring enhancers.

Figure 15A further reports the agreement between (i) the log fold change of predicted H2 to predicted HepG2 activity and (ii) actual log fold change of H2 activity to HepG2 activity in tandem repeat training enhancer sequences in replicas of cells (respectively H2 calls and HepG2 cells) for (i) the tandem repeat training enhancer sequences (bar 1510) and (ii) the approximately 12,000 naturally occurring enhancers (bar 1512). Bars 1510 and 1512 show that the CNN model trained on the tandem repeat training enhancer sequences shows better performance when analyzed by this log fold change.

Fig. 15B compares the performance of (i) CNN models trained using only naturally occurring enhancer sequences versus (ii) CNN models first trained using tandem repeat training enhancer sequences and then retrained using naturally occurring enhancer sequences in determining MPN versus HEPG2 specificity, MPN activity, and HepG2 activity. Fig. 15B shows that retrained CNNs don't always outperform CNNs only trained on CNS library at predicting CNS library, but are potentially more generalizable. Fig. 15B further shows that retained CNNs retain capability of predicting tandem repeat library (test split).

A model 114, in some embodiments, is associated with an objective function, which generates a value that describes the objective goal of the training process. For example, in some embodiments, the training intends to reduce the error rate of the model 114 in generating a prediction of the activity of an enhancer. In such a case, the objective function monitors the error rate of the machine learning model. Such an objective function, in some embodiments, is called a loss function. In some embodiments, other forms of objective functions are also used. In some embodiments, the loss function determines the difference between model output (predicted activity) and measured activity, and the gradient with respect to the input is calculated and backpropagated to update the model parameters. In various embodiments, the error rate is measured as cross-entropy loss, L1 loss (e.g., the sum of absolute differences between the predicted values and the actual value), L2 loss (e.g., the sum of squared distances).

Referring to block 244, in some embodiments, the model 114 is a support vector machine. Referring to block 246, in some such embodiments, the support vector machine comprises a gapped k-mer kernel. Non-limiting examples of support vector machines are provided in the definitions section above.

Referring to block 248, in some embodiments, the model 114 is a convolutional neural network (CNN). In some embodiments, the convolutional neural network includes different kinds of layers, such as convolutional layers, pooling layers, recurrent layers, fully connected layers, and custom layers. A convolutional layer convolves the input of the layer (*e.g*., an RNA sequence) with one or more weight kernels to generate different types of sequences that are filtered by the kernels to generate feature sequences. Each convolution result, in some embodiments, is associated with an activation function. A convolutional layer, in some embodiments, is followed by a pooling layer that selects the maximum value (max pooling) or average value (average pooling) from the portion of the input covered by the kernel size. The pooling layer reduces the spatial size of the extracted features. Optionally, in some embodiments, one or more convolutional layers and pooling layer is followed by an optional recurrent layer that includes one or more feedback loops. The feedback, in some embodiments, is used to account for spatial relationships of the features in an image or temporal relationships in sequences. In some embodiments, the above-described layers are followed by multiple fully connected layers that have nodes connected to each other. The fully connected layers are, in some embodiments, used for classification and regression. In various embodiments, a CNN includes one or more convolutional layer but does not include any pooling layer or recurrent layer. In various embodiments, a CNN includes one or more convolutional layer, one or more pooling layer, one or more recurrent layer, or any combination thereof. If a pooling layer is present, not all convolutional layers are always followed by a pooling layer. A recurrent layer, in some embodiments, is also positioned differently at other locations of the CNN. For each convolutional layer, the sizes of kernels (*e.g.*, 1x3, 1x5, 1x7, *etc.)* and the numbers of kernels allowed to be learned, in some embodiments, are different from other convolutional layers.

In some embodiments, a model includes certain layers, nodes, kernels and/or coefficients. Training of a model, such as a CNN, in some embodiments, in accordance with block 242 includes multiple iterations of forward propagation and backpropagation. In embodiment where the model is a CNN, each layer in the neural network, in some embodiments, includes one or more nodes that are fully or partially connected to other nodes in adjacent layers. In forward propagation, the neural network performs the computation in the forward direction based on outputs of a preceding layer. The operation of a node, in some embodiments, is defined by one or more functions. The functions that define the operation of a node, in some embodiments, include various computation operations such as convolution of data with one or more kernels, pooling, recurrent loop in RNN, various gates in LSTM, *etc.* The functions, in some embodiments, also include an activation function that adjusts the weight of the output of the node. Nodes in different layers, in some embodiments, are associated with different functions.

Each of the functions in the neural network, in some embodiments, is associated with different parameters (*e.g*., weights and kernel coefficients) that are adjustable during training. In addition, some of the nodes in a neural network, in some embodiments, are also associated with an activation function that decides the weight of the output of the node in forward propagation. Common activation functions include, but are not limited to, step functions, linear functions, sigmoid functions, hyperbolic tangent functions (tanh), and rectified linear unit functions (ReLU). After an input is provided into the neural network and passes through a neural network in the forward direction, in some implementations, the results are compared to the training labels or other values in the training set to determine the neural network's performance. In some embodiments, the process of prediction is repeated for other inputs in the training sets to compute the value of the objective function in a particular training round. In turn, the neural network performs backpropagation by using gradient descent such as stochastic gradient descent (SGD) to adjust the coefficients in various functions to improve the value of the objective function.

In some embodiments, multiple iterations of forward propagation and backpropagation are performed and the model is iteratively trained. In some embodiments, training is completed when the objective function has become sufficiently stable (e.g., the machine has converged) or after a predetermined number of rounds for a particular set of training enhancer sequences. In some embodiments, the trained model is used for performing various tasks as discussed in this disclosure.

Figure 22 illustrates one example convolutional neural network (CNN) 2201, an example of a model 114, used in some embodiments of the present disclosure. The order of the layers from input to output for CNN 2201 are from left to right as illustrated in Figure 22. CNN 2201 includes convolutional with ReLU activation layers 2202, 2206, and 2210, each respectively followed by maximum pooling layers 2204, 2208, and 2212, fulling connected layers 2214 and 2216, followed by the linear layer 2218.

Models 114 that are convolutional neural networks (CNNs) have unique advantages over other models because CNNs can model binding motifs within enhancer sequences independent of their positions within the enhancer sequences. Figures 12, 13, and 14 illustrate how such a convolutional neural network is good at modeling regulatory elements. For Figure 12, the 50,000 training enhancer sequences that each have their own unique tandem 10-mer repeat sequence described above in conjunction with panel 602 of Figure 6 was used to train a convolutional neural network in accordance with block 244. Each of these training enhancer sequences is set up in a construct illustrated in Figure 3 (*e.g*., with a core promoter 310 and a payload 312) and exposed to respective replicas of H4 cells and HepG2 cells. The activity of the reporter gene 312, in accordance with the construct illustrated in Figure 3, in such H4 cells and HepG2 cells, was then used to train the convolutional neural network (CNN) in accordance with block 244. Panel 1202 reports the agreement between (i) predicted H4 activity of each of the tandem repeat training enhancer sequences from the trained CNN (upon inputting the sequences of the tandem repeat training enhancer sequences into the CNN) and (ii) their actual H4 activity in replicas of H4 cells. Panel 1204 reports the agreement between (i) predicted HepG2 activity of each of the tandem repeat training enhancer sequences from the CNN (upon inputting the sequences of the tandem repeat training enhancer sequences into the CNN) and (ii) their actual HepG2 activity in replicas of HepG2 cells. Figure 13 reports the agreement between (i) the log fold change of predicted H2 to predicted HepG2 activity and (ii) actual log fold change of H2 activity to HepG2 activity of each of the tandem repeat training enhancer sequences in replicas of HepG2 cells. Figure 14 compares the performance (Spearman's rho, predicted versus truth) for the agreement between (i) predicted H4 activity of each of the tandem repeat training enhancer sequences from either the trained CNN (CNN, Figure 14 element 1402) or a trained XGBoost model (XGBoost, Figure 14 element 1404) and (ii) their actual H4 activity in replicas of H4 cells. Figure 14 further compares the performance (Spearman's rho, predicted versus truth) for the agreement between (i) predicted HepG2 activity of each of the tandem repeat training enhancer sequences from either the trained CNN (CNN, Figure 14 element 1406) or a trained XGBoost model (XGBoost, Figure 14 element 1408) and (ii) their actual H4 activity in replicas of HepG2 cells. Figure 14 further reports the agreement between (i) the log fold change of predicted H2 to predicted HepG2 activity and (ii) actual log fold change of H2 activity to HepG2 activity of each of the tandem repeat training enhancer sequences in replicas of HepG2 cells for the CNN model (element 1410) or the XGBoost model (element 1412).

In various embodiments, a wide variety of models 114 are applicable for performing the methods disclosed herein. Non-limiting examples include different forms of supervised learning models such as decision trees, support vector machines (SVMs), linear regression, logistic regression, Bayesian networks, and boosted gradient models. Deep learning models such as neural networks, including convolutional neural networks (CNN), recurrent neural networks (RNN), and attention-based models (such as Transformers), are also contemplated. In some embodiments ensembles of models are used.

In some embodiments, the model 114 is a convolutional neural network, a recurrent neural network, a multilayer perceptron, XGBoost (*e.g*., extreme Gradient Boosting), a transformer model, and/or a generative model.

As another example, in some embodiments, the model 114 makes use of a bagging architecture (*e.g*., random forest, extra tree algorithms) and/or a boosting architecture (*e.g*., gradient boosting, XGBoost, *etc.).* In some embodiments, the model 114 is an extreme gradient boost (XGBoost) model. Description of XGBoost models is found, for example, in Chen *et al.,* 2016, "XGBoost: A Scalable Tree Boosting System," arXiv:1603.02754v3 [cs.LG], the disclosure of which is hereby incorporated by reference, in its entirety, for all purposes, and specifically for its teaching of training and using XGBoost models.

In some embodiments boosting is used. In some embodiments, a boosting technique such as AdaBoost is used in conjunction with many other types of learning algorithms to improve the performance of the model 114. In this approach, the output of any of the models 114 disclosed herein, or their equivalents, is combined into a weighted sum that represents the final output of the boosted model. In some embodiments, the plurality of outputs from the models is combined using any measure of central tendency known in the art, including but not limited to a mean, median, mode, a weighted mean, weighted median, weighted mode, *etc.* In some embodiments, the plurality of outputs is combined using a voting method. In some embodiments, a respective model in the ensemble of models is weighted or unweighted.

Referring to block 250, in some embodiments, the plurality of parameters 116 comprises at least 100 parameters, 1000 parameters, at least 5000 parameters, at least 10,000 parameters, at least 100,000 parameters, at least 250,000 parameters, at least 500,000 parameters, or at least 1,000,000 parameters.

In some embodiments, the plurality of parameters 116 is at least 1000 parameters, at least 5000 parameters, at least 10,000 parameters, at least 100,000 parameters, at least 250,000 parameters, at least 500,000 parameters, or at least 1,000,000 parameters.

In some embodiments, the plurality of parameters 116 comprises at least 200, at least 500, at least 1000, at least 5000, at least 10,000, at least 100,000, at least 250,000, at least 500,000, at least 1,000,000, or at least 5,000,000 parameters. In some embodiments, the plurality of parameters comprises no more than 10,000,000, no more than 5,000,000, no more than 1,000,000, no more than 100,000, no more than 10,000, or no more than 1000 parameters. In some embodiments, the plurality of parameters consists of from 200 to 10,000, from 1000 to 100,000, from 5000 from 200,000, from 100,000 to 500,000, from 500,000 to 1,000,000, from 1,000,000 to 5,000,000, or from 5,000,000 to 10,000,000 parameters. In some embodiments, the plurality of parameters falls within another range starting no lower than 200 parameters and ending no higher than 10,000,000 parameters.

Referring to block 252, in some embodiments, the method further comprises obtaining a second training dataset, in electronic form. The second training dataset comprises a second plurality of training enhancer sequences. For each respective training enhancer sequence in the second plurality of training enhancer sequences, a corresponding measured amount of selectivity of the respective training enhancer sequence across the plurality of *in vivo* states. In practice, this involves the determination of a measured amount of activity in each *in vivo* state in the plurality of *in vivo* states. Each training enhancer sequence in the second plurality of training enhancer sequences is an endogenous enhancer. The procedure further comprises, after the inputting and refining described in block 242, inputting a sequence of each respective training enhancer sequence in the second plurality of training enhancer sequences into the model thereby obtaining as output from the model, for each respective training enhancer sequence in the second plurality of training enhancer sequences, a corresponding predicted activity of the respective training enhancer in at least a first *in vivo* state in the one or more *in vivo* states. The procedure refines the plurality of parameters based on a differential between the corresponding measured amount activity and the corresponding predicted activity in at least the first *in vivo* state for each respective training enhancer sequence in the second plurality of training enhancer sequences. Referring to block 254, in some embodiments, the second plurality of training enhancer sequences comprises 10,000 or more training enhancer sequences, 20,000 or more training enhancer sequences, 30,000 or more training enhancer sequences, 40,000 or more training enhancer sequences, 50,000 or more training enhancer sequences, 60,000 or more training enhancer sequences, 70,000 or more training enhancer sequences, 80,000 or more training enhancer sequences, 90,000 or more training enhancer sequences, or 100,000 or more training enhancer sequences.

Figure 16 illustrates the performance gain that is achieved by the transfer learning approach of blocks 252 and 254.

Bar 1602 of Figure 16 shows model performance (Spearman's rho, predicted versus truth) for a CNN model that has been pre-trained on the 50,000 tandem repeat training enhancer sequences of panel 602 of Figure 6 in accordance with block 242 of Figure 2E for H4 activity and then trained on the approximately 12,000 naturally occurring enhancers for H4 activity. For this training, the H4 activity in cells for each of the sequences in the tandem repeat 50,000 training enhancer sequences of panel 602 of Figure 6 and each of the approximately 12,000 naturally occurring enhancers is known and is used as a basis for the training. Bar 1602 is the performance (Spearman's rho, predicted versus truth) between (i) predicted H4 activity of each of the approximately 12,000 naturally occurring enhancers from the transfer learning trained CNN model and (ii) their actual H4 activity in replicas of H4 cells. Correspondingly, curve 1706 in the Area Under the Curve (ROC) plot 1702 of Figure 17 shows the performance of this model against the approximately 12,000 naturally occurring enhancers. Correspondingly, curve 1712 in the precision / recall plot 1704 of Figure 17 shows the performance of this model against the approximately 12,000 naturally occurring enhancers.

Bar 1604 of Figure 16 shows model performance (Spearman's rho, predicted versus truth) for a CNN model that has not been pre-trained on the 50,000 tandem repeat training enhancer sequences of panel 602 of Figure 6 for H4 activity. For bar 1604, the model has only been trained using the approximately 12,000 naturally occurring enhancers for H4 activity. Bar 1604 is the performance (Spearman's rho, predicted versus truth) between (i) predicted H4 activity of each of the approximately 12,000 naturally occurring enhancers from the trained CNN model and (ii) their actual H4 activity in replicas of H4 cells. Correspondingly, curve 1708 in the ROC plot 1702 of Figure 17 shows the performance of this model against the approximately 12,000 naturally occurring enhancers. Correspondingly, curve 1714 in the precision / recall plot 1704 of Figure 17 shows the performance of this model against the approximately 12,000 naturally occurring enhancers.

As can be seen by comparison of bars 1602 and 1604 of Figure 16, there is a performance improvement for predicting H4 activity by pre-training with the 50,000 training enhancer sequences of panel 602 of Figure 6 and then using transfer learning in which the model parameters for the CNN are retained from the training of the 50,000 training enhancer sequences of panel 602 of Figure 6 and used as a starting basis for continuing to train the model with the approximately 12,000 naturally occurring enhancers for H4 activity. This pre-training transfer learning performance improvement is also seen by comparing curves 1706 and 1708 in ROC plot 1702 of Figure 17, as well as by comparing curves 1712 and 1714 in the precision / recall plot 1704 of Figure 17.

By comparison to bars 1602 and 1604, bar 1606 shows that a degradation in model performance is observed when the model is trained on the 50,000 tandem repeat training enhancer sequences of panel 602 of Figure 6 for H4 activity and then model performance is ascertained using the approximately 12,000 naturally occurring enhancers for H4 activity. For bar 1606, the CNN model has only been trained using the 50,000 tandem repeat training enhancer sequences of panel 602 of Figure 6 for H4 activity. Bar 1606 is the performance (Spearman's rho, predicted versus truth) between (i) predicted H4 activity of each of the approximately 12,000 naturally occurring enhancers from this trained CNN model and (ii) their actual H4 activity in replicas of H4 cells. This degradation in performance is also seen by comparing curves 1706 and 1708 to curve 1710 in ROC plot 1702 of Figure 17, as well as by comparing curves 1712 and 1714 to curve 1716 in the precision / recall plot 1704 of Figure 17.

Bars 1608, 1610 and 1612 show that the transfer learning approach improves for selectivity of HepG2 activity as well.

Bar 1608 of Figure 16 shows model performance (Spearman's rho, predicted versus truth) for a CNN model that has been pre-trained on the 50,000 tandem repeat training enhancer sequences of panel 602 of Figure 6 in accordance with block 242 of Figure 2E for HepG2 activity and then trained on the approximately 12,000 naturally occurring enhancers for HepG2 activity. For this training, the HepG2 activity in cells for each of the sequences in the 50,000 training enhancer sequences of panel 602 of Figure 6 and each of the approximately 12,000 naturally occurring enhancers is known and is used as a basis for the training. Thus, bar 1608 is the performance (Spearman's rho, predicted versus truth) between (i) predicted HepG2 activity of each of the approximately 12,000 naturally occurring enhancers from the transfer learning trained CNN model and (ii) their actual HepG2 activity in replicas of HepG2 cells.

Bar 1610 of Figure 16 shows model performance (Spearman's rho, predicted versus truth) for a CNN model that has not been pre-trained on the 50,000 tandem repeat training enhancer sequences of panel 602 of Figure 6 for HepG2 activity. For bar 1610, the CNN model has only been trained using the approximately 12,000 naturally occurring enhancers for HepG2 activity. Therefore, bar 1610 is the performance (Spearman's rho, predicted versus truth) between (i) predicted HepG2 activity of each of the approximately 12,000 naturally occurring enhancers from the trained CNN model and (ii) their actual HepG2 activity in replicas of HepG2 cells.

As can be seen by comparison of bars 1608 and 1610 of Figure 16, there is a performance improvement for predicting HepG2 activity by pre-training with the 50,000 training enhancer sequences of panel 602 of Figure 6 and then using transfer learning in which the model parameters for the CNN are retained from the training of the 50,000 training enhancer sequences of panel 602 of Figure 6 and used as a starting basis for continuing to train the model with the approximately 12,000 naturally occurring enhancers for HepG2 activity.

Comparison to bars 1608 and 1610, bar 1612 of Figure 16 shows that a degradation in model performance is observed when the model is trained on the 50,000 tandem repeat training enhancer sequences of panel 602 of Figure 6 for HepG2 activity and then model performance is ascertained using the approximately 12,000 naturally occurring enhancers. For bar 1612, the CNN model has only been trained using the 50,000 tandem repeat training enhancer sequences of panel 602 of Figure 6 for HepG2 activity. Bar 1612 is the performance (Spearman's rho, predicted versus truth) between (i) predicted HepG2 activity of each of the approximately 12,000 naturally occurring enhancers from this trained CNN and (ii) their actual HepG2 activity in replicas of HepG2 cells.

The Spearman agreement in fold-changes reported in bars 1614, 1616, and 1618 are consistent with the previous observations that pre-training on the 50,000 training enhancer sequences of panel 602 of Figure 6 and then training on the approximately 12,000 naturally occurring enhancers in accordance with block 252 of Figure 2F leads to improved performance.

Bar 1614 of Figure 16 shows the agreement between (i) the log fold change of model predicted H2 to model predicted HepG2 activity and (ii) actual log fold change of H2 activity to HepG2 activity for enhancers in replicas of cells (respectively H2 calls and HepG2 cells) across the approximately 12,000 naturally occurring enhancers. The H2 and HepG2 models for bar 1614 are the transferred learned models used for bars 1602 and 1608 respectively.

Bar 1616 of Figure 16 shows the agreement between (i) the log fold change of model predicted H2 to model predicted HepG2 activity and (ii) actual log fold change of H2 activity to HepG2 activity for enhancers in replicas of cells (respectively H2 calls and HepG2 cells) across the approximately 12,000 naturally occurring enhancers. The H2 and HepG2 models for bar 1616 are the models used for bars 1604 and 1610, respectively, which were only trained on the approximately 12,000 naturally occurring enhancers.

Bar 1618 of Figure 16 shows the agreement between (i) the log fold change of model predicted H2 to model predicted HepG2 activity and (ii) actual log fold change of H2 activity to HepG2 activity for enhancers in replicas of cells (respectively H2 calls and HepG2 cells) across the approximately 12,000 naturally occurring enhancers. The H2 and HepG2 models for bar 1616 are the models used for bars 1606 and 1612, respectively, which were trained on the 50,000 training enhancer sequences of panel 602 of Figure 6.

Panel 1620 of Figure 16 includes a bar that corresponds to each of the bars discussed above in conjunction with panel 1600 of Figure 16 with the exception that the performance metric on the Y-axis is Pearson's r, rather than Spearman's rho. Panel 1620 agrees with panel 1600 in that improved performance (now Pearson's r in panel 1620 versus Spearman's rho of panel 1600) is realized by pre-training using the approximately 12,000 naturally occurring enhancers in accordance with block 252 of Figure 2F followed by transfer learning in which the model parameters are further trained using the approximately 12,000 naturally occurring enhancers.

Additional datasets can be used as a second training set beyond, or instead of, the approximately 12,000 naturally occurring enhancers described above in conjunction with Figures 16 and 17. These include 5183 neuron active enhancers, 2,343 candidate cis-regulatory elements within 100 kilobases of neuron marker genes, and 416 enhancers active in brain.

Figure 22 provides another illustration of transfer learning techniques in accordance with block 252. In accordance with Figure 22, an analysis model 106 obtains a first training dataset 106, in electronic form. The first training dataset 106 comprises a first plurality of training enhancer sequences 108 and, for each respective training enhancer sequence in the first plurality of training enhancer sequences, a corresponding measured amount of transcriptional activity of the respective training enhancer sequence for each of one or more *in vivo* states 112. The first training set used in Figure 22 is the 50,00 10-mer tandem repeat training enhancer sequence library 602 described above in conjunction with Figure 6. As such, each training enhancer sequence 108 in the first plurality of training enhancer sequences is a tandem 10-mer repeat sequence. Convolutional neural network (CNN) 2201, which is an example of a model 114 comprising a plurality of parameters 116, is trained by a procedure comprising (i) inputting a sequence of each respective training enhancer sequence 108 in the first plurality of training enhancer sequences into the CNN 2201. CNN 2201 applies the plurality of parameters, in accordance with the CNN 2201 model architecture illustrated in Figure 22, to the sequence of each respective training enhancer sequence 108 to generate as output from the CNN 2201, for each respective training enhancer sequence 108 in the first plurality of training enhancer sequences, a corresponding predicted amount of transcriptional activity for either mouse primary neuron (MPN) cells or HepG2 cells of the respective training enhancer sequence. The plurality of parameters 116 is refined based on a differential between the corresponding measured amount of transcriptional activity (in MPN cells or HepG2 cells) and the corresponding predicted amount of transcriptional activity (in MPN cells or HepG2 cells) for each respective training enhancer sequence in the first plurality of training enhancer sequences. As such, two separate CNN models 2201 are trained, one for MPN transcriptional activity and another for HepG2 activity. As illustrated in panel 2220 of Figure 20, a second training dataset is obtained in electronic form. The second training dataset comprises a second plurality of training enhancer sequences and, for each respective training enhancer sequence in the second plurality of training enhancer sequences, a corresponding measured amount of transcriptional activity in MPN cells and in HepG2 cells. Each training enhancer sequence in the second plurality of training enhancer sequences is an endogenous enhancer. As illustrated in Figure 22, there are about 10,000 enhancer sequences in the second plurality of training enhancer sequences. The procedure for training each of the CNNs 2201 further comprises a transfer learning approach in which each CNN 2201, already trained on the first training set 106 is now trained on the second training set by inputting a sequence of each respective training enhancer sequence in the second plurality of training enhancer sequences into CNN 2201 thereby obtaining as output from the CNN 2201, for each respective training enhancer sequence in the second plurality of training enhancer sequences, a corresponding predicted amount of transcriptional activity in MPN cells (for the CNN model being trained for transcriptional activity in MPN cells) or a corresponding predicted amount of transcriptional activity in HepG2 cells (for the CNN model being trained for transcriptional activity in HepG2 cells) of the respective training enhancer and refining the plurality of parameters 116 based on a differential between the corresponding measured amount of transcriptional activity and the corresponding predicted amount of transcriptional activity for each respective training enhancer sequence in the second plurality of training enhancer sequences. In this way each CNN 2201 is fine tuned to predict transcriptional activity. Moreover, log fold changes from the two models (HepG2 transcriptional activity trained model and MPN transcriptional activity trained model) can be calculated.

### Generate test enhancer sequences using the trained model

Referring to block 255 of Figure 2G, a plurality of test enhancer sequences (e.g., 120-1, ..., 120-Q, where Q is a positive integer) is generated using the trained model.

In some implementations, the trained model is used to score randomly and/or algorithmically generated novel nucleic acid sequences of test enhancer sequences. In some such embodiments, the model scores for these test enhancer sequences are used to rank and select new test enhancer sequences for experimental testing.

Referring to block 256, in some embodiments, the plurality of test enhancer sequences comprises 10 or more, 100 or more, 1000 or more, or 10,000 or more test enhancer sequences.

Referring to block 258, in some embodiments, the method further comprises applying a feature attribution method to a test nucleic acid sequence using the trained model 114 to obtain a predictive weight matrix that provides a predictive contribution of every residue position in the test nucleic acid sequence to the plurality of *in vivo* states. The generating of the plurality of test enhancer sequences using the trained model is in accordance with at least a portion of the predictive weight matrix.

Figure 5 illustrates applying a feature attribution method to a test nucleic acid sequence using the trained model 114 to obtain a predictive weight matrix that provides a predictive contribution of every residue position in the test nucleic acid sequence to the plurality of *in vivo* states. As shown in panel 502, for each position in the test nucleic acid sequence, the predictive contribution of the residue of test nucleic acid sequence occupying that residue position is provided. In the example of Figure 5, the predictive contribution is normalized to zero and is normalized so that is ranges from -0.01 (does not contribute to selectivity across the plurality of *in vivo* states) to +0.01 (contributes to selectivity across the plurality of *in vivo* states).

Referring to block 260, in some embodiments, the model is a convolutional neural network and the feature attribution method is an integrated gradients feature attribution method. One example an integrated gradients feature attribution method is GkmExplain. *See,* Shrikumar *et al.,* 2019, "GkmExplain: fast and accurate interpretation of nonlinear gapped k-mer SVMs to a test nucleic acid sequence using the trained model to obtain a predictive weight matrix," Bioinformatics 35, i173-i182, which is hereby incorporated by reference.

Referring to block 262, in some embodiments, a test enhancer sequence in the plurality of test enhancer sequences comprises a plurality of residue positions. Each respective residue position in the plurality of residue positions is populated with one of four naturally occurring nucleotides based on a corresponding probability for the respective residue position given by the predictive weight matrix. The probability at any given residue position across the four naturally occurring nucleotides (A, T, C, and G) sums to 100 percent probability in some embodiments.

Figure 5 illustrates. From the predictive weight matrix used to produce panel 502, a region of low activity 504 in the original test nucleic acid sequence is seen. Although only one region of low activity 504 is illustrated in Figure 5, in some embodiments there are 2, 3, 4, or 5 or more regions of low activity. Figure 5 further shows three proposals 506-1, 506-2, and 506-3 for replacing region 504 using the predictive weight matrix. Each respective residue position in the plurality of residue positions is populated with one of four naturally occurring nucleotides based on a corresponding probability for the respective residue position given by the predictive weight matrix. In each proposal, the original 5' A and the 3' terminal "CCG" are retained but each position between these termini is replaced on a probabilistic basis in accordance with the predictive weight matrix. For instance, consider the position 2 in sequences 120-1, 120-2, and 120-3. If the predictive weight matrix assigned the probability that this position is a "C" to be 0.63, the probability that this position is an "A" to be 0.05, the probability that this position is a "T" to be 0.8, and the probability that this position is a "G" to be 0.24, the position corresponding to position 2 in a test enhancer sequence will be assigned "C" with a probability of 0.63, "A" with a probability of 0.05, "T" with a probability of 0.8, and "G" with a probability of 0.24. The remaining positions (*e*.*g*., 2, 3, 4, *etc*.) in each test enhancer sequence that correspond to the region 504 are similarly populated based on the corresponding probabilities given for "A", "T", "G", and "C" given for each of these positions. Panel 506 shows the full length of test enhancer sequence 120-1, after swapping out the region of low activity of panel 502 with a sequence generated using the predictive weight matrix. As can be seen in panel 506, the region of low activity found in panel 502 has now been replaced with a sequence that potentially will have higher activity.

In some embodiments, the position weight matrix derived from the trained model 114 through feature attribution is used to identify one or more regions of low activity, such as region 504 of Figure 5, and then known binding sites (*e*.*g*., transcription binding motif sequences) are substituted into these one or more regions to generate test enhancer sequences from a test nucleic acid sequence. One source for such binding sites is JASPAR, a regularly maintained open-access database storing manually curated transcription factors (TF) binding profiles as position frequency matrices (PFMs), available on the Internet at jaspar.genereg.net. *See* also, Stormo, 2013, "Modeling the specificity of protein-DNA interactions," Quant Biol. 1(2), pp. 115-130; and Wasserman and Sandelin, 2004, "Applied bioinformatics for the identification of regulatory elements," Nat Rev Genet 5(4), pp. 276-287, each of which is hereby incorporated by reference. Such an approach is call "Motif replacement" and is used to generate the test enhancer sequences summarized in panel 2108 of Figure 21 as well as panel 2406 of Figure 24. In some embodiments, rather than replacing regions of low activity 502 with motifs as was done for panel 2108 of Figure 21 as well as panel 2406 of Figure 24, such regions are replaced by one of the k-mers that is the basis for a top scoring tandem n-mer tandem training enhancer sequence 108 in the first training set 106. For instance, consider an n-mer tandem training enhancer sequence 108 that has the highest activity among all the n-mer tandem training enhancer sequence 108 in the first training set 106 in an *in vivo* state of interest. The n-mer from this top scoring n-mer tandem training enhancer sequence 108 can be used to replace regions of low activity 504 in the test nucleic acid sequence to generate test enhancer sequences. Such an approach is call "k-mer replacement" and is used to generate the test enhancer sequences summarized in panel 2106 of Figure 21 as well as panel 2408 of Figure 24.

In some embodiments the position weight matrices available for known transcription binding motif sequences is used to generate, tens, hundreds, or thousands of sequences for the one or more regions of low activity, such as region 502 of Figure 5, identified through the trained model 114. Thus, in such embodiments, the position weight matrix derived for a proposed (*e*.*g*., naturally occurring) enhancer sequence (test nucleic acid sequence of block 258) is determined using the trained model 114, one or more regions of low activity are identified using the position weight matrix determined using the trained model 114, and then the position weight matrix of a known transcription binding motif is used to determine subsequences to replace the one or more regions of low activity in the naturally occurring enhancer sequence. Thus, in such embodiments, the position weight matrix used in step 2) in such embodiments is that of the binding site being inserted into the one or more regions of low activity.

Figure 9 provides a further illustration of block 262. Cluster 902 in Figure 9 illustrates the model score upon sequentially inputting each of the enhancer sequences in an approximately 12,000 naturally occurring enhancer library after training the model in accordance with block 242 on a 50,000 member 10-mer tandem repeat training enhancer library. From cluster 902, approximately 200 naturally occurring enhancer sequences were identified based on their high activity in H4. Cluster 904 illustrates the model score upon sequentially inputting each of these selected enhancer sequences into the trained model. Thus, cluster 904 is a subset of cluster 902. A feature attribution method was applied to each of the enhancer sequences of cluster 904 using the trained model to obtain a corresponding position weight matrix to identify regions of each enhancer sequence in cluster 904 that should be replaced. These regions of low activity were replaced with sequences derived from known binding sites. Thus, the naturally occurring enhancer sequences of cluster 904 were used to produce corresponding engineered enhancer sequences (test enhancer sequences) which, in turn, were tested in replicas of H4 and HepG2 cells using the Figure 3 construct and adeno-associated virus vector-mediated gene delivery into H4 and HepG2. Cluster 906 shows the model scores for the engineered enhancer sequences (test enhancer sequences). There is a 1 to 1 correspondence between cluster 904 and cluster 906. In other words, for each respective naturally occurring enhancer sequence in cluster 904, there is a corresponding enhancer sequence in cluster 906 that is derived from the respective naturally occurring enhancer sequence in accordance with the methods described above in conjunction with Figure 5. As can be seen in Figure 9, the engineering results in an overall higher model score (activity for H4) for the enhancer sequences of cluster 906 than for the enhancer sequences of cluster 904, indicating that the substitution of regions in each enhancer sequence in accordance with the corresponding position weight matrix leads to improved H4 activity.

In some embodiments, between 5 and 25 residues, between 10 and 30 residues, or between 15 and 50 residues of the test nucleic acid sequence of block 258 that have regions of low activity across the plurality of *in vivo* states are replaced with sequences that are either known binding sites or are generated from the position frequency matrices of such binding sites.

Referring to block 264, in some embodiments, the trained model is used to generate the plurality of test enhancer sequences using an input optimization algorithm. Figure 23 illustrates input optimization, for the case of the CNN 2201 optimized for HepG2 transcriptional activity using the approach illustrated in Figure 22. For Figure 23, the CNN 2201 trained for HepG2 transcriptional activity is asked to solve for test enhancer sequences 108 that the model predicts will have a certain target HepG2 transcriptional activity (a high value). These designs are then validated in *in vivo* transcriptional experiment by placing them in the construct illustrated in Figure 3 and testing them for transcriptional activity in HepG2 cells. Figure 23 thus compares the HepG2 transcriptional activity of the original enhancer sequences (panel 2302) to the test enhancer sequences 114 that have been generated by the CNN 2202 through input operation (panel 2304). Figure 23 illustrates that the input optimization can generate highly active enhancers in HepG2.

In embodiments that make use of input optimization, the model comprises an input layer configured to accept a test sequence. The model is configured to output, a calculated activity of the sequence responsive to inputting the test sequence. A desired activity level for the test enhancer sequence (in a particular *in vivo* state) is set and a difference between this desired activity level and the calculated activity of the sequence is backpropagated through the model with the parameters fixed and the test enhancer sequence is modified to resolve the difference between the desired activity level and the calculated activity of the sequence, thereby generating candidate sequences. Such an approach is referred to in statistics as Response surface methodology. In such approaches, the backpropagation involves using a gradient ascent/descent optimization function on the input test sequence rather than the parameter trained model to find the optimal input (enhancer sequences with desired activity. Such an approach is call "Input optimization" and is used to generate the test enhancer sequences summarized in panel 2104 of Figure 21.

Referring to block 266, in some embodiments, the trained model is used to generate the plurality of test enhancer sequences using a bit diffusion algorithm. In some such implementations, a generative model is first trained by randomly adding noise to a test nucleic acid sequence thereby generating a seed (forward diffusion process). The model then performs a reverse diffusion process to remove the noise from the test nucleic acid sequence conditioned on an optimal activity in the desired *in vivo* state. To generate novel enhancer sequences, the reverse diffusion process is run on randomly generated test enhancer sequences 108. The performance of the test enhancer sequences 108 is then experimentally tested, and such performance measures and/or generated polymer sequences can be fed back into the generative model for iterative training until a target performance is achieved. *See* Killoran *et al.,* 2017, "Generating and designing DNA with deep generative models," arXiv:1712.06148v1, which is hereby incorporated by reference. Such an approach is call "Bit diffusion" and is used to generate the test enhancer sequences summarized in panel 2102 of Figure 21. See also Example 2.

### Determine the activity of the test enhancer sequences.

Referring to block 267 of Figure 2H, the activity of each respective test enhancer sequence in the plurality of test enhancer sequences is determined in an *in vivo* assay thereby screening for improved enhancer selectivity.

Referring to block 268, in some embodiments, the determining the activity of each respective test enhancer sequence in the plurality of test enhancer sequences in an *in vivo* assay comprises exposing the respective test enhancer sequence operably connected to the common promoter and the common payload to each respective *in vivo* state in the one or more *in vivo* states and measuring an abundance of the common payload in each respective *in vivo* state in the one or more *in vivo* states.

Figure 21 illustrates results in accordance with block 268. In Figure 21, the activity of test enhancer sequences generated using a trained model 114 in mouse primary neuron cells (MPN, a proxy for brain tissue) and HepG2 cells (a proxy for liver) is displayed. In Figure 21, each dot represents a different test enhancer sequence and is color coded to represent whether the test enhancer sequence was designed to be active in HepG2 cells (pink - meaning that it is intended to cause a payload to highly express in HepG2 cells); for MPN cells (orange - meaning that it is intended to cause a payload to express highly in MPN cells); or blue, meaning that what is being displayed is the log fold change in expression in MPN versus Hepg2 cells for a given test enhancer sequence.

Panel 2102 shows the activity of test enhancer sequences 120 generated using a trained convolutional neural network model 114 using bit diffusion algorithm in accordance with block 266.

Panel 2104 shows the activity of test enhancer sequences 120 generated using a trained convolutional neural network model 114 using an input optimization method in accordance with block 264.

Panel 2106 shows the activity of test enhancer sequences 120 generated using a trained convolutional neural network model 114 in which the feature attribution method gkmExplain (Shrikumar et al., 2019, "GkmExplain: fast and accurate interpretation of nonlinear gapped k-mer SVMsto a test nucleic acid sequence using the trained model to obtain a predictive weight matrix," Bioinformatics 35, i173-i182, which is hereby incorporated by reference), was used to generate a predictive weight matrix that provides a predictive contribution of every residue position in the test nucleic acid sequence to the activity for mouse primary neurons or to HepG2 cells. Then, these predictive weight matrices were used to replace k-mers in enhancer sequences that the predictive weight matrix indicated had low scoring activity (for MPN or conversely for HepG2) with k-mers the predictive weight matrix indicated had a greater contribution to activity in MPN or HepG2.

Panel 2108 shows the activity of test enhancer sequences 120 generated using a trained convolutional neural network model 114 in which the feature attribution method gkmExplain (Shrikumar et al., 2019, "GkmExplain: fast and accurate interpretation of nonlinear gapped k-mer SVMs, "Bioinformatics 35, i173-i182, which is hereby incorporated by reference), was used to generate a predictive weight matrix that provided a predictive contribution of every residue position in the test nucleic acid sequence to the activity in mouse primary neuron cells or in HepG2 cells. Then, these predictive weight matrices were used to replace k-mers within enhancer sequences that the predictive weight matrix indicated had low scoring activity (for MPN or conversely for HepG2) with k-mers the predictive weight matrix indicated had a greater contribution to activity.

### Panel 2110 is a negative control.

Panel 2112 is the activity of the original test enhancer sequences prior to optimization.

### Panel 2114 is a positive control.

Panel 2116 illustrates an embodiment in which regions of test nucleic acid sequences tested in accordance with block 258 that have suitable activity are combined together to produce composite test enhancer sequences from these regions.

Figure 24 provides further comparison of the results from original hit validation (panel 2402), subjecting these original hits to k-mer replacement (panel 2404), subjecting these original hits to motif replacement (panel 2406), and combining positive regions (panel 2408). What is illustrated in panels 2204, 2406, and 2408 is the transcriptional activity in HepgG2 cells of the test enhancer sequences respectively produced through the k-mer replacement, motif replacement, and positive region combined methods of the present disclosure relative to the original enhancer sequence prior to such optimization.

### Additional Embodiments

Another aspect of the present disclosure provides a computer system comprising one or more processors and a non-transitory computer-readable medium including computer-executable instructions that, when executed by the one or more processors, cause the processors to perform any of the methods and/or embodiments disclosed herein.

Yet another aspect of the present disclosure provides a non-transitory computer-readable storage medium having stored thereon program code instructions that, when executed by a processor, cause the processor to perform any of the methods and/or embodiments disclosed herein.

### EXAMPLES

Although inventions have been particularly shown and described with reference to a preferred embodiment and various alternate embodiments, it will be understood by persons skilled in the relevant art that various changes in form and details can be made therein without departing from the spirit and scope of the invention.

### Example 1 - Validation of use of a 10-mer repeat in constructing training enhancer sequences.

Referring to the left portion of Figure 10, what is shown is the position weight matrix for forward and reverse motifs of a HNF4A transcription factor binding site. *See,* for example, Fang et al., Nucleic Acids Research 40(12), pp. 5343-5356, which is hereby incorporated by reference. A model was trained in accordance with block 242 against a 50,000 member tandem 10-mer repeat training enhancer sequence library constructed in accordance with Figure 4. The model was then used to generate the position weight matrices depicted in Figure 10. Those sequences in the 50,000 member tandem 10-mer repeat training enhancer sequence library that shared at least 80 percent sequence identity to either the forward or reference position weight matrix for the HNF4A transcription factor binding site were then selected. Figure 11 compares the log fold change (logFC) between HepG2 versus H4 expression of the 50,000 member tandem 10-mer repeat training enhancer sequence library (left bar) and those sequences in the 50,000 member tandem 10-mer repeat training enhancer sequence library that share at least 80 percent sequence identity to either the forward or reference position weight matrix for the HNF4A transcription factor binding site (right bar). The HNF4a motif matched sequences are more specific to HepG2 expression (versus H4 expression) than the 50,000-member tandem 10-mer repeat training enhancer sequence library as a whole (background). This result indicates that the tandem 10-mer encoding of Figure 4 can identify sequences that confer activity for specific *in vivo* states (*e*.*g*., biological regulatory sequences that regulate gene expression in the brain, biological regulatory sequences that regulate gene expression in lung tissue) that are present in the complex naturally occurring enhancers. In other words, this result indicates that the tandem k-mer library can identify biological plausible sequences such as HNF4a transcription factor binding as being active. HNF4a transcription factor is a known regulatory protein specific for regulating gene expression in the liver (and HepG2) cells. As noted in Figure 11, the p-value of the observed change in average logFC between the background and HNF4a match is 1e-16.

### Example 2 - Diffusion model for conditional generation of enhancer sequences.

A model was trained to generate enhancer sequences conditioned on mouse primary neuron (MPN) activity, liver cancer cell (HepG2) activity, and/or a metric comprising log fold change of MPN vs HepG2 activity. The bit diffusion model included a U-Net model comprising an architecture as illustrated in FIGS. 23A-B of U.S. Provisional Patent Application No. 63/585,119, filed September 25, 2023, which is hereby incorporated by reference.

The model was trained on the unbiased tandem repeat library of panel 602 of Figure 6 and then retrained on a tissue-specific CNS library. Model training was performed as described in blocks 242 and 252 of Figure 2. In particular, models 114 were first trained using the tandem repeat library. Updated parameters (*e*.*g*., weights) obtained from the tandem repeat library training were then used as the initialization point to retain the model using the CNS library. Training inputs to the model were the enhancer sequences from the training libraries and the training was conditioned using measured activity for (i) mouse primary neuron (MPN) activity, (ii) liver cancer cell (HepG2) activity, or (iii) a metric comprising log fold change of MPN versus HepG2 activity.

A total of 25,000 enhancer sequences predicted to have mouse primary neuron (MPN) activity were generated from the above-described models. A total of 25,000 enhancer sequences predicted to have HepG2 activity were generated form the above-described models A total of 25,000 enhancer sequences were generated that were predicted to have appreciable log fold MPN versus HepG2 activity using the above-described models. After subsampling the generated enhancer sequences, on an unweighted random basis, a corresponding 2000 enhancer sequences predictive to have MPN activity, 200 enhancer sequences predicted to have HepG2 activity, and 500 enhancer sequences predicted to have appreciable log fold change in MPN versus HepG2 activity remained. The generated sequences could be further synthesized for experimental validation.

A proof-of-concept assay was performed to assess the ability of the above described models to generate enhancer sequences conditioned on a particular target property for MPN activity. Performance of the generated enhancer sequences generated by the above-described models was estimated using a trained CNN model that produced, as output, predicted labels according to each of three performance criteria (MPN activity, HepG2 activity, and/or log fold change of MPN vs HepG2 activity) upon receiving a generated enhancer sequence as input. The output of the bit diffusion model was furtbased on a comparison of the predicted labels (y-axis) with the "true" labels used for conditioning (x-axis). Figure 19 illustrates the results of this evaluation, in which good concordance is observed for MPN activity (r = 0.83) and MPN vs. HepG2 log fold change in activity (r = 0.83), but poor concordance is observed for HepG2 activity where conditioning values were of small dynamic range. These results illustrate the bit diffusion model's ability to generate enhancer sequences that are active for a target property (MPN activity) but not active for non-target properties (HepG2 activity). In general, activity (*e*.*g*., MPN and HepG2 activity) for enhancer sequences generated by the bit diffusion model were positively correlated with conditioning values. However, the enhancer sequences generated by the bit diffusion model did not outperform the top candidates identified through screening in the initial proof-of-concept study.

### Example 3 - Performance of a model trained on n-mer tandem repeats.

In this example, the effect of using a model trained to score enhancer sequences conditioned on epithelial cell line (ARPE-19) activity using a 10-mer tandem repeat training enhancer sequence library was investigated.

To determine this effect, first the base line selectivity between HepG2 (liver) and ARPE-19 (epithelial) cells was determined. For the column marked "endogenous" in Figure 25, a random selection of the approximately 12,000 naturally occurring enhancers described above (*e*.*g*., in conjunction with Figure 6) was made. The activity of each of these randomly selected endogenous enhancers in ARPE-19 cells and in HepG2 cells was evaluated by measuring RNA counts and DNA counts in cells transfected with the endogenous enhancer and determining the log ratio of these two counts. Then, ratio of the activity of each of the selected endogenous enhancers in ARPE19 versus HepG2 cells was plotted in the column entitled "endogenous" in Figure 25. This established a baseline selectivity between ARPE19 and HepG2 cells for endogenous enhancers.

The column marked "Random sample" provides another basis for background enhancer selectivity between ARPE19 and HepG2 cells. The enhancers for this column were randomly selected enhancers from the 50,000 10-mer tandem repeat training enhancer sequence library described above in conjunction with Figure 6. The activity of each of these randomly selected tandem repeat training enhancer sequences in ARPE-19 cells and in HepG2 cells was determined by measuring RNA counts and DNA counts in cells transfected with the tandem repeat training enhancer sequence and determining the log ratio of these two counts. Next, the ratio of the activity of each of the selected tandem repeat training enhancer sequences in ARPE19 versus HepG2 cells was plotted in the column entitled "random sample" in Figure 25. This established a baseline selectivity between ARPE19 and HepG2 cells for tandem repeat training enhancer sequences.

Next, a model was trained on the unbiased tandem repeat library of panel 602 of Figure 6. Model training was performed as described in block 242 of Figure 2. In particular, the model 114 was trained using the tandem repeat library using measured activity of the tandem repeats in ARPE19 cells.

The tandem repeat library trained model was then used to identify positive regions (regions that contribute to activity) in endogenous enhancers using the feature attribution techniques (*e*.*g*., as disclosed above in conjunction with Figure 5 and block 258 of Figure 2G). Endogenous regions in endogenous enhancers that had significant contribution to activity, as predicted by the tandem repeat library trained model using attribution, were randomly combined to generate a set of enhancer sequences. For instance, an enhancer in this set of enhancer sequences could include regions from 2, 3, 4, 5, or 6 or more endogenous enhancers that attribution techniques, using the tandem repeat library trained model, indicated contributed to activity in ARPE19 cells. Then, the activity of each of the enhancers in this set of enhancer sequences in ARPE19 cells and in HepG2 cells was determined by measuring RNA counts and DNA counts in cells transfected with the tandem repeat training enhancer sequence and determining the log ratio of these two counts. Next, the ratio of the activity of each of the enhancer sequences in this set of enhancer sequences in ARPE19 versus HepG2 cells was plotted in the column entitled "gkm positive regions combined" in Figure 25. It is seen that this set of enhancers had greater selectivity for ARPE19 cells versus the endogenous enhancers as well as the random sample enhancers, indicating that the tandem repeat library trained model, trained solely on the tandem repeat library, was capable of predicting enhancers that had selectivity between ARPE19 and HepgG2 cells. Figure 26 is a plot of the predicted ARPE19 activity of each of these enhancers (Y-axis) from the tandem repeat library trained model versus the measured ARPE19 versus HepG2 log fold change in activity for these enhancers. The plot shows there was a positive correlation between model predicted ARPE19 activity and measured ARPE19 versus HepG2 log fold change in activity. Figure 27 is a plot of the measured ARPE19 activity and the measured HepG2 activity of five of these enhancers (enhancers 5, 6, 7, 8 and 9 in Figure 27) compared to four scrambled negative control enhancers (enhancers 1, 2, 3, and 4 in Figure 27). Figure 27 shows that the five machine learning optimized enhancers (enhancers that were assigned high ARPE19 activity scores by the tandem repeat library trained model) have activity in ARPE19 that was significantly above background.

The tandem repeat library trained model was also used to rank the approximately 12,000 naturally occurring enhancers described above (*e*.*g*., in conjunction with Figure 6). Top ranked enhancers determined by the tandem repeat library trained model were tested for activity in ARPE-19 cells and in HepG2 cells by measuring RNA counts and DNA counts in cells transfected with each of the top ranked endogenous enhancers. Next, the ratio of the activity of each of the top ranked enhancers was plotted in the column entitled "top scoring endogenous by arpe tandem repeat prediction" in Figure 25. It is seen that this set of enhancers had greater selectivity for ARPE19 cells versus the endogenous enhancers and the random sample enhancers, indicating that the tandem repeat library trained model, trained solely on the tandem repeat library with ARPE19 activity data, was capable of selecting endogenous enhancers that have selectivity between ARPE19 and HepgG2 cells.

### Equivalents and Incorporation by Reference

All references cited herein are incorporated by reference to the same extent as if each individual publication, database entry (*e*.*g*., Genbank sequences or GeneID entries), patent application, or patent, was specifically and individually indicated to be incorporated by reference in its entirety, for all purposes. This statement of incorporation by reference is intended by Applicants, pursuant to 37 C.F.R. §1.57(b)(1), to relate to each and every individual publication, database entry (*e*.*g*., Genbank sequences or GenelD entries), patent application, or patent, each of which is clearly identified in compliance with 37 C.F.R. §1.57(b)(2), even if such citation is not immediately adjacent to a dedicated statement of incorporation by reference. The inclusion of dedicated statements of incorporation by reference, if any, within the specification does not in any way weaken this general statement of incorporation by reference. Citation of the references herein is not intended as an admission that the reference is pertinent prior art, nor does it constitute any admission as to the contents or date of these publications or documents.

### Exemplary Embodiments

Embodiment 1. A method of screening for improved enhancer activity comprising: obtaining a first training dataset, in electronic form, wherein the first training dataset comprises a first plurality of training enhancer sequences and, for each respective training enhancer sequence in the first plurality of training enhancer sequences, a corresponding measured amount of activity of the respective training enhancer sequence in each of one or more *in vivo* states, wherein each training enhancer sequence in the first plurality of training enhancer sequences is a tandem n-mer repeat sequence; training a model comprising a plurality of parameters by a procedure comprising: (i) inputting a sequence of each respective training enhancer sequence in the first plurality of training enhancer sequences into the model, wherein the model applies the plurality of parameters to the sequence of each respective training enhancer sequence to generate as output from the model, for each respective training enhancer sequence in the first plurality of training enhancer sequences, a corresponding predicted amount of activity of the respective training enhancer sequence for at least one a first *in vivo* state of the one or more *in vivo* states, and (ii) refining the plurality of parameters based on a differential between the corresponding measured amount of activity and the corresponding predicted amount of activity in the first *in vivo* state for each respective training enhancer sequence in the first plurality of training enhancer sequences; generating a plurality of test enhancer sequences using the trained model; and determining an activity of each respective test enhancer sequence in the plurality of test enhancer sequences in an *in vivo* assay thereby screening for improved enhancer activity.

Embodiment 2. The method of embodiment 1, wherein the model is a support vector machine.

Embodiment 3. The method of embodiment 2, wherein the support vector machine comprises a gapped k-mer kernel.

Embodiment 4. The method of embodiment 1, wherein the model is a convolutional neural network.

Embodiment 5. The method of any one of embodiments 1-4, wherein the method further comprises applying a feature attribution method to a test nucleic acid sequence using the trained model to obtain a predictive weight matrix that provides a predictive contribution of every residue position in the test nucleic acid sequence to an activity in the first *in vivo* state, and the generating the plurality of test enhancer sequences using the trained model is in accordance with at least a portion of the predictive weight matrix.

Embodiment 6. The method of embodiment 5, wherein the model is a convolutional neural network and the feature attribution method is an integrated gradients feature attribution method.

Embodiment 7. The method of embodiment 5, wherein a test enhancer sequence in the plurality of test enhancer sequences comprises a plurality of residue positions, and each respective residue position in the plurality of residue positions is populated with one of four naturally occurring nucleotides based on a corresponding probability for the respective residue position given by the predictive weight matrix.

Embodiment 8. The method of embodiment 1, wherein the trained model is used to generate the plurality of test enhancer sequences using an input optimization algorithm.

Embodiment 9. The method of embodiment 1, wherein the trained model is used to generate the plurality of test enhancer sequences using a bit diffusion algorithm.

Embodiment 10. The method of any one of embodiments 1-9, wherein the plurality of parameters is at least 100 parameters, 1000 parameters, at least 5000 parameters, at least 10,000 parameters, at least 100,000 parameters, at least 250,000 parameters, at least 500,000 parameters, or at least 1,000,000 parameters.

Embodiment 11. The method of any one of embodiment 1-10, wherein each training enhancer sequence in the first plurality of training enhancer sequences comprises 5 to 20 contiguous repeats of a different n-mer, wherein n is a positive integer between 4 and 20.

Embodiment 12. The method of any one of embodiments 1-10, wherein each training enhancer sequence in the first plurality of training enhancer sequences comprises 5 to 20 contiguous repeats of a different 10-mer.

Embodiment 13. The method of any one of embodiments 1-12, wherein each training enhancer sequence in the first plurality of training enhancer sequences consists of between 15 residues and 200 residues.

Embodiment 14. The method of any one of embodiments 1-13, wherein each respective training enhancer sequence in the first plurality of training enhancer sequences is operably connected to a common promoter and a common payload; and the corresponding measured amount of activity in the first *in vivo* state is a transcriptional activity in the first *in vivo* state in a tissue.

Embodiment 15. The method of embodiment 14, wherein the tissue is brain tissue or liver tissue.

Embodiment 16. The method of embodiment 14, wherein the tissue is blood, brain, colon, diaphragm, gastrocnemius muscle, heart, inguinal adipose, kidney, lung, lymph node, skin, small intestine, spinal cord, spleen, trachea, liver, stomach, large intestine, pancreas, gallbladder, bladder, eyes, thymus, or adrenal gland.

Embodiment 17. The method of any one of embodiments 1-13, wherein
each respective training enhancer sequence in the first plurality of training enhancer sequences is operably connected to a common promoter and a common payload under a common transcription factor; and each respective *in vivo* state in the one or more *in vivo* states is an amount of expression of the common payload in a corresponding cell line originating from a particular tissue.

Embodiment 18. The method of embodiment 17, wherein the cell line is HepG2, H4, or K562.

Embodiment 19. The method of embodiment 17, wherein the common transcription factor is FOSB::JUNB, FOSL2::JUNB, FOSL1::JUNB, FOS::JUN; FOSL1::JUN; BATF3, BNC2; ZBTB12; HSF4; HSF1; HSF2; REL; RELA; TEAD3; TEAD1; TP53; TP63; TP73; SMAD5; SMAD2; ZNF416; GRHL2; TFCP2; MLX; ARNT::HIF1A; or HIF1A.

Embodiment 20. The method of any one of embodiments 1-19, wherein the corresponding measured amount of activity of the respective training enhancer sequence in the one or more *in vivo* states comprises a corresponding measured abundance of a payload associated with the respective training enhancer sequence in each of the one or more *in vivo* states.

Embodiment 21. The method of embodiment 20, wherein the corresponding measured abundance of the respective training enhancer sequence in an *in vivo* state in the one or more *in vivo* states is a corresponding averaged or summed count of a number of barcoded instances of a payload associated with the respective training enhancer sequence found in a corresponding plurality of sequence reads of RNA harvested from each replica in a plurality of replicas of a plurality of cells, representative of the *in vivo* state exposed to the respective training enhancer sequence.

Embodiment 22. The method of embodiment 21, wherein the corresponding averaged or summed count is log normalized by an amount of the respective training enhancer sequence used for exposing each replica of the plurality of cells.

Embodiment 23. The method embodiment 21 or embodiment 22, wherein each respective training enhancer sequence in the plurality of respective training enhancer sequences is uniquely assigned a corresponding plurality of barcodes comprising 5 or more, 10 or more, 20 or more, 50 or more, 75 or more, or 100 or more barcodes, and each replica of the plurality of cells representative of the *in vivo* state exposed to the respective training enhancer sequence is, for each respective barcode uniquely assigned to the respective training enhancer sequence, one or more copies of the respective training enhancer sequence tagged with the respective barcode.

Embodiment 24. The method of embodiment 23, wherein each barcode in the corresponding plurality of barcodes encodes a unique predetermined value selected from the set {1, ..., 1024}, {1, ..., 4096}, {1, ..., 16384}, {1, ..., 65536}, {1, ..., 262144}, {1, ..., 1048576}, {1, ..., 4194304}, {1, ..., 16777216}, {1, ..., 67108864}, or {1, ..., 1 × 10¹²}.

Embodiment 25. The method of any one of embodiment 21-24, wherein the corresponding plurality of sequence reads of RNA harvested from each replica in the plurality of replicas of a plurality of cells comprises at least 1000, at least 2000, at least 3000, at least 4000, at least 5000, at least 6000, at least 7000, at least 8000, at least 9000, at least 10,000, at least 50,000, at least 100,000, at least 500,000, at least 1 million, at least 2 million, at least 3 million, at least 4 million, at least 5 million, at least 6 million, at least 7 million, at least 8 million, at least 9 million, or more sequence reads.

Embodiment 26. The method of any one of embodiments 21-25, wherein the corresponding plurality of replicas of the plurality of cells comprises 2, 3, 4, 5, 6, 7, 8, 9, or 10 replicas.

Embodiment 27. The method of any one of embodiment 1-26, wherein the first plurality of training enhancer sequences comprises 10,000 or more training enhancer sequences, 20,000 or more training enhancer sequences, 30,000 or more training enhancer sequences, 40,000 or more training enhancer sequences, 50,000 or more training enhancer sequences, 60,000 or more training enhancer sequences, 70,000 or more training enhancer sequences, 80,000 or more training enhancer sequences, 90,000 or more training enhancer sequences, or 100,000 or more training enhancer sequences.

Embodiment 28. The method of any one of embodiments 1-27, wherein the one or more *in vivo* states consists of a first *in vivo* state and a second *in vivo* state, and the corresponding measured amount of activity of the respective training enhancer sequence in each of the one or more *in vivo* states comprises a log fold change in (i) an abundance of the respective training enhancer sequence measured in the first *in vivo* state and (ii) an abundance of the respective training enhancer sequence measured in the second *in vivo* state.

Embodiment 29. The method of any one of embodiments 1-27, wherein the one or more *in vivo* states consists of a first *in vivo* state and a second *in vivo* state, and the corresponding measured amount of activity of the respective training enhancer sequence in each of the one or more *in vivo* states comprises a binary indication of measured activity between the first *in vivo* state and the second *in vivo* state.

Embodiment 30. The method of any one of embodiments 1-27, wherein the one or more *in vivo* states consists of a first *in vivo* state and a second *in vivo* state, and the corresponding measured amount of activity of the respective training enhancer sequence across the one or more *in vivo* states comprises a scalar indication of measured activity between the first *in vivo* state and the second *in vivo* state.

Embodiment 31. The method of any one of embodiments 1-30, wherein the method further comprises: obtaining a second training dataset, in electronic form, wherein the second training dataset comprises a second plurality of training enhancer sequences and, for each respective training enhancer sequence in the second plurality of training enhancer sequences, a corresponding measured amount of activity of the respective training enhancer sequence in each of the one or more *in vivo* states, wherein each training enhancer sequence in the second plurality of training enhancer sequences is an endogenous enhancer; and the procedure further comprises, after the (i) inputting and (ii) refining, (iii) inputting a sequence of each respective training enhancer sequence in the second plurality of training enhancer sequences into the model thereby obtaining as output from the model, for each respective training enhancer sequence in the second plurality of training enhancer sequences, a corresponding predicted amount of activity of the respective training enhancer in at least the first *in vivo* state, and (iv) refining the plurality of parameters based on a differential between the corresponding measured amount of activity and the corresponding predicted amount of activity in at least the first *in vivo* state for each respective training enhancer sequence in the second plurality of training enhancer sequences.

Embodiment 32. The method of embodiment 31, wherein the second plurality of training enhancer sequences comprises 10,000 or more training enhancer sequences, 20,000 or more training enhancer sequences, 30,000 or more training enhancer sequences, 40,000 or more training enhancer sequences, 50,000 or more training enhancer sequences, 60,000 or more training enhancer sequences, 70,000 or more training enhancer sequences, 80,000 or more training enhancer sequences, 90,000 or more training enhancer sequences, or 100,000 or more training enhancer sequences.

Embodiment 33. The method of any one of embodiments 1-32, wherein the plurality of test enhancer sequences comprises 10 or more, 100 or more, 1000 or more, or 10,000 or more test enhancer sequences.

Embodiment 34. The method of any one of embodiments 1-33, wherein the determining the activity of each respective test enhancer sequence in the plurality of test enhancer sequences in an *in vivo* assay comprises: exposing the respective test enhancer sequence operably connected to the common promoter and the common payload to each respective *in vivo* state in the one or more *in vivo* states; and measuring an abundance of the common payload in each respective *in vivo* state in the one or more *in vivo* states.

Embodiment 35. A computer system comprising: one or more processors; and
a non-transitory computer-readable medium including computer-executable instructions that, when executed by the one or more processors, cause the processors to perform the method according to any one of embodiments 1-34.

Embodiment 36. A non-transitory computer-readable storage medium having stored thereon program code instructions that, when executed by a processor, cause the processor to perform the method according to any one of embodiments 1-34.

### Additional Consideration

The foregoing description of the embodiments has been presented for the purpose of illustration; it is not intended to be exhaustive or to limit the patent rights to the precise forms disclosed. Persons skilled in the relevant art will appreciate that many modifications and variations are possible in light of the above disclosure.

Any feature mentioned in one claim category, *e*.*g*., method, can be claimed in another claim category, *e.g*., computer program product, system, storage medium, as well. The dependencies or references back in the attached claims are chosen for formal reasons only. However, any subject matter resulting from a deliberate reference back to any previous claims (in particular multiple dependencies) can be claimed as well, so that any combination of claims and the features thereof is disclosed and can be claimed regardless of the dependencies chosen in the attached claims. The subject matter, in some embodiments, includes not only the combinations of features as set out in the disclosed embodiments but also any other combination of features from different embodiments. Various features mentioned in the different embodiments can be combined with explicit mentioning of such combination or arrangement in an example embodiment or without any explicit mentioning. Furthermore, any of the embodiments and features described or depicted herein, in some embodiments, are claimed in a separate claim and/or in any combination with any embodiment or feature described or depicted herein or with any of the features.

Some portions of this description describe the embodiments in terms of algorithms and symbolic representations of operations on information. These operations and algorithmic descriptions, while described functionally, computationally, or logically, are understood to be implemented by computer programs or equivalent electrical circuits, microcode, or the like. Furthermore, it has also proven convenient at times, to refer to these arrangements of operations as engines, without loss of generality. The described operations and their associated engines are, in some embodiments, embodied in software, firmware, hardware, or any combinations thereof.

Any of the steps, operations, or processes described herein, in some embodiments, are performed or implemented with one or more hardware or software engines, alone or in combination with other devices. In one embodiment, a software engine is implemented with a computer program product comprising a computer-readable medium containing computer program code, which can be executed by a computer processor for performing any or all of the steps, operations, or processes described. The term "steps" does not mandate or imply a particular order. For example, while this disclosure describes, in some embodiments, a process that includes multiple steps sequentially with arrows present in a flowchart, the steps in the process do not need to be performed by the specific order claimed or described in the disclosure. In some implementations, some steps are performed before others even though the other steps are claimed or described first in this disclosure. Likewise, any use of (i), (ii), (iii), *etc.,* or (a), (b), (c), *etc.* in the specification or in the claims, unless specified, is used to better enumerate items or steps and also does not mandate a particular order.

## Claims

1. A method of modeling enhancer activity comprising:
obtaining a training dataset, in electronic form, wherein the training dataset comprises a plurality of training enhancers and, for each respective training enhancer in the plurality of training enhancers, a corresponding measured amount of activity of the respective training enhancer in each of one or more states, wherein each training enhancer in the plurality of training enhancers is a tandem repeat;
training a model comprising a plurality of parameters by a procedure comprising:
(i) inputting each respective training enhancer in the plurality of training enhancers into the model, wherein the model applies the plurality of parameters to the respective training enhancer to generate as output from the model, for each respective training enhancer in the plurality of training enhancers, a corresponding predicted amount of activity of the respective training enhancer for at least one a first state of the one or more states, and
(ii) refining the plurality of parameters based on a differential between the corresponding measured amount of activity and the corresponding predicted amount of activity in the first state for each respective training enhancer in the plurality of training enhancers;
generating a plurality of test enhancers using the trained model; and
determining an activity of each respective test enhancer in the plurality of test enhancers sequences thereby modeling enhancer activity.

2. A computer system comprising:
one or more processors; and
a non-transitory computer-readable medium including computer-executable instructions that, when executed by the one or more processors, cause the processors to perform the method according to claim 1.

3. A non-transitory computer-readable storage medium having stored thereon program code instructions that, when executed by a processor, cause the processor to perform the method according to claim 1.
